(19) Europäisches Patentamt
European Patent Office
Office européen des brevets

(11) Veröffentlichungsnummer: **0 010 661**
**B1**

(12) # EUROPÄISCHE PATENTSCHRIFT

(45) Veröffentlichungstag der Patentschrift: **12.09.84**

(21) Anmeldenummer: **79103908.4**

(22) Anmeldetag: **11.10.79**

(51) Int. Cl.³: **C 07 D 471/04,** A 61 K 31/445, C 07 C 93/12, C 07 C 97/07, C 07 D 217/04, C 07 D 209/08 // C07C125/065, C07C93/14 (C07D471/04, 221/00, 209/00)

(54) **Isochinolinderivate, Verfahren zu ihrer Herstellung, Zwischenprodukte, Arzneimittel enthaltend solche Isochinolinderivate und ihre pharmazeutische Verwendung.**

(30) Priorität: **13.10.78 US 950947**
**10.09.79 US 73813**

(43) Veröffentlichungstag der Anmeldung: **14.05.80 Patentblatt 80/10**

(45) Bekanntmachung des Hinweises auf die Patenterteilung: **12.09.84 Patentblatt 84/37**

(84) Benannte Vertragsstaaten: **AT BE CH DE FR GB IT LU NL SE**

(56) Entgegenhaltungen:
**CHEMICAL ABSTRACTS, Band 86, Nr. 25, 20. Juni 1977, Zusammenfassung Nr. 189902k, Seite 611, Columbus, Ohio, US**

(73) Patentinhaber: **F. HOFFMANN-LA ROCHE & CO. Aktiengesellschaft, CH-4002 Basel (CH)**

(72) Erfinder: **Berger, Leo, 7 The Parkway, Montclair, N.J. (US)**
Erfinder: **Olson, Gary Lee, 431 Everson Place, Westfield N.J. (US)**

(74) Vertreter: **Lederer, Franz, Dr. et al, Patentanwälte Dr. Franz Lederer Reiner F. Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung bezieht sich auf Octahydro-1H-pyrrolo[2,3-g]-isochinoline der allgemeinen Formel

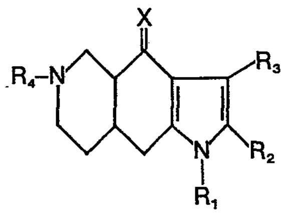

A

worin $R_1$ Wasserstoff, $(C_1–C_7)$-Alkyl, $(C_1–C_7)$-Alkanoyl oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Benzoyl oder Phenyl-$(C_1–C_7)$-alkyl, $R_2$ und $R_3$ unabhängig voneinander Wasserstoff, $(C_1–C_7)$-Alkyl, $(C_3–C_6)$-Cycloalkyl, $(C_2–C_7)$-Alkenyl, $(C_1–C_7)$-Alkanoyl oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Benzoyl, Phenyl oder Phenyl-$(C_1–C_7)$-alkyl und $R_4$ Wasserstoff,
$(C_1–C_7)$-Alkyl, Hydroxy-$(C_1–C_7)$-alkyl,
Phenyl-hydroxy-$(C_1–C_7)$-alkyl,
Halogenphenyl-hydroxy-$(C_1–C_7)$-alkyl,
$(C_1–C_7)$-Alkylphenyl-hydroxy-$(C_1–C_7)$-alkyl,
$(C_1–C_7)$-Alkoxyphenyl-hydroxy-$(C_1–C_7)$-alkyl,
$(C_1–C_7)$-Alkoxy-$(C_1–C_7)$-alkyl,
$(C_1–C_7)$-Alkoxy-hydroxy-$(C_1–C_7)$-alkyl,
$(C_1–C_7)$-Alkanoyloxy-$(C_1–C_7)$-alkyl,
$(C_1–C_7)$-Alkoxycarbonyl-$(C_1–C_7)$-alkyl,
$(C_2–C_7)$-Alkenyl,
$(C_3–C_6)$-Cycloalkyl-$(C_1–C_7)$-alkyl,
$(C_2–C_7)$-Alkinyl, Thienyl-$(C_1–C_7)$-alkyl,
Furyl-$(C_1–C_7)$-alkyl,
$(C_1–C_7)$-Alkanoyl-$(C_1–C_7)$-alkyl,
$(C_3–C_6)$-Cycloalkoxy-$(C_1–C_7)$-alkyl,
$(C_3–C_6)$-Cycloalkyl-hydroxy-$(C_1–C_7)$-alkyl,
$(C_2–C_7)$-Alkenyloxy-$(C_1–C_7)$-alkyl,
N-$(C_1–C_7)$-Alkyl-pyrrolidinyl-$(C_1–C_7)$-alkyl,
Trifluoralkyl mit 2 bis 6 Kohlenstoffatomen oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Phenoxy-hydroxy-$(C_1–C_7)$alkyl, Benzoyloxy-$(C_1–C_7)$-alkyl, Benzoyl-$(C_1–C_7)$-alkyl, Phenyl-$(C_1–C_7)$-alkyl, Phenylcarboxamido-$(C_1–C_7)$-alkyl, Phenyl-$(C_2–C_7)$-alkenyl, Phenoxy-$(C_1–C_7)$-alkyl
oder Phenyl-N-imidazolonyl-$(C_1–C_7)$-alkyl oder

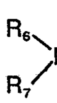 N-$(C_1–C_7)$-Alkyl sind, worin $R_6$ und $R_7$ unabhängig voneinander Wasserstoff oder $(C_1–C_7)$-Alkyl oder zusammen mit dem Stickstoffatom ein 5- oder 6gliedriger gesättigter heterocyclischer Ring, der als Ringglied noch ein Sauerstoffatom oder ein gegebenenfalls durch $(C_1–C_7)$-Alkyl oder Hydroxy-$(C_1–C_7)$-alkyl substituiertes Stickstoffatom enthalten kann, sind und X O oder S ist, ferner auf optische und geometrische Isomere dieser Verbindungen und auf pharmazeutisch annehmbare Säureadditionssalze hiervon.

In Chemical Abstracts 86, 189902 k (1977) werden Verbindungen beschrieben, welche ein ähnliches Grundgerüst besitzen und dämpfende Wirkungen auf das zentrale Nervensystem entfalten. Die anmeldegemässen Verbindungen unterscheiden sich durch die Anwesenheit einer Oxo- oder Thionogruppe, den nichtaromatischen Charakter des mittleren Rings und ein andersartiges Substitutionsmuster in signifikanter Weise von den vorbekannten Verbindungen.

Der Ausdruck «$(C_1–C_7)$-Alkyl», wie er hier verwendet wird, bezeichnet einen geradkettigen oder verzweigtkettigen, gesättigten Kohlenwasserstoff mit 1 bis 7 Kohlenstoffatomen, z.B. Methyl, Äthyl, Propyl, Isopropyl, Butyl, t-Butyl, Neopentyl, Pentyl, Heptyl u.dgl. Der Betriff «$(C_1–C_7)$-Alkoxy» bezeichnet eine Alkyläthergruppe, in der die Alkylgruppe wie zuvor beschrieben ist, z.B. Methoxy, Äthoxy, Propoxy, Pentoxy u.dgl. Der Begriff «$(C_2–C_7)$-Alkenyl» bezeichnet eine geradkettige oder verzweigtkettige, ungesättigte Kohlenwasserstoffgruppe mit 2 bis 7 Kohlenstoffatomen, z.B. Vinyl, Allyl u.dgl. Der Begriff «$(C_2–C_7)$-Alkinyl» bezeichnet eine geradkettige oder verzweigtkettige, ungesättigte Kohlenwasserstoffgruppe mit 2 bis 7 Kohlenstoffatomen, z.B. Äthinyl, Propargyl, Methylbutinyl u.dgl. Der Begriff «Halogen» oder «Halogeno» bezeichnet die Halogene Brom, Chlor, Fluor und Jod. Der Begriff «Trifluoralkyl mit 2 bis 6 Kohlenstoffatomen» bedeutet vorzugsweise 2,2,2-Trifluoräthyl u.dgl. Der Begriff «Aryl» bedeutet Phenyl oder einen oder mehrere Substituenten der Gruppe Halogen, Trifluormethyl, Niederalkyl, Niederalkoxy, Nitro, Amino, Niederalkylamino und Diniederalkylamino tragendes Phenyl. Der Begriff «Aralkyl» bedeutet bevorzugt Benzyl u.dgl. Der Begriff «Aryloxy» bedeutet eine Aryläthergruppe, in der die Arylgruppe wie oben beschrieben ist, z.B. Phenoxy u.dgl. Die Begriffe «$(C_1–C_7)$-Alkanoyl» und «$(C_1–C_7)$-Alkanoyloxy» leiten sich von aliphatischen Carbonsäuren mit 1 bis 7 Kohlenstoffatomen ab, z.B. Formyl, Acetyl, Propionyl, Formyloxy, Acetoxy, Propionyloxy u.dgl. Der 5gliedrige oder 6gliedrige heterocyclische Ring leitet sich von einer gesättigten heterocyclischen Verbindung mit einem oder zwei Stickstoffatomen oder einem Stickstoffatom und einem Sauerstoffatom ab, wobei das zweite Stickstoffatom durch $(C_1–C_7)$-Alkyl oder Hydroxy-$(C_1–C_7)$-alkyl substituiert sein kann, z.B. Morpholino, N-Methyl-piperazino, Piperazino, Pyrrolidino u.dgl.

Bevorzugte Verbindungen der Formel A sind solche, bei denen $R_1$ Wasserstoff, $R_2$ und $R_3$ $(C_1–C_7)$-Alkyl, $R_4$ $(C_1–C_7)$-alkyl, Hydroxy-$(C_1–C_7)$-alkyl, Phenyl-hydroxy-$(C_1–C_7)$-alkyl, Halogenphenylhydroxy-$(C_1–C_7)$-alkyl, $(C_1–C_7)$-Alkoxy-$(C_1–C_7)$-alkyl oder jeweils gegebenenfalls am Phenylring

durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Phenoxy-($C_1$–$C_7$)-alkyl, Benzoyl-($C_1$–$C_7$)-alkyl oder Phenyl-($C_1$–$C_7$)-alkyl sind und X O ist.

Die am stärksten bevorzugten erfindungsgemässen Verbindungen der Formel A, in der X O ist, sind folgende:

3-Äthyl-2,6-dimethyl-4,4a,5,6,7,8,8a,9-octahydro-4a,8a-trans-1H-pyrrolo[2,3-g]isochinolin-4-on;

(–)-3-Äthyl-2,6-dimethyl-4,4a,5,6,7,8,8a,9-octahydro-4a,8a-trans-1H-pyrrolo[2,3-g]isochinolin-4-on;

(–)-3-Äthyl-2,6-dimethyl-4,4a,5,6,7,8,8a,9-octahydro-4a,8a-trans-1H-pyrrolo[2,3-g]isochinolin-4-on-Hydrochlorid, 0,25 Mol-Hydrat;

2-Methyl-3-äthyl-6-(2-hydroxy-2-phenyläthyl)-4,4a,5,6,7,8,8a,9-octahydro-4a,8a-trans-1H-pyrrolo[2,3-g]isochinolin-4-on;

2,3,6-Trimethyl-4,4a,5,6,7,8,8a,9-octahydro-4a,8a-trans-1H-pyrrolo[2,3-g]-isochinolin-4-on;

2,3,6-Trimethyl-4,4a,5,6,7,8,8a,9-octahydro-4a,8a-trans-1H-pyrrolo[2,3-g]-isochinolin-4-on-Hydrochlorid;

2-Methyl-3-äthyl-6-benzyl-4,4a,5,6,7,8,8a,9-octahydro-4a,8a,trans-1H-pyrrolo[2,3-g]isochinolin-4-on;

2-Methyl-3-äthyl-6-benzzyl-4,4a,5,6,7,8,8a,9-octahydro-4a,8a-trans-1H-pyrrolo[2,3-g]isochinolin-4-on-Hydrochlorid;

2-Methyl-3-äthyl-6-(2-phenyläthyl)-4,4a,5,6,7,8,8a,9-octahydro-4a,8a-trans-1H-pyrrolo[2,3-g]isochinolin-4-on;

2-Methyl-3-äthyl-6-(2-äthoxyäthyl)-4,4a,5,6,7,-8,8a,9-octahydro-4a,8a-trans-1H-pyrrolo[2,3-g]isochinolin-4-on;

2-Methyl-3-äthyl-6-(2-äthoxyäthyl)-4,4a,5,6,7,8,-8a,9-octahydro-4a,8a-trans-1H-pyrrolo[2,3-g]isochinolin-4-on-Hydrochlorid;

2-Methyl-3-äthyl-6-[4-(4-fluorphenyl)-4-oxobutyl]-4,4a,5,6,7,8,8a,9-octahydro-4a,8a-trans-1H-pyrrolo[2,3-g]isochinolin-4-on;

2-Methyl-3-äthyl-6-[3-(4-fluorphenyl)-3-oxopropyl]-4,4a,5,6,7,8,8a,9-octahydro-4a,8a-trans-1H-pyrrolo[2,3-g]isochinolin-4-on;

2-Methyl-3-äthyl-6-(3-phenoxypropyl)-4,4a,5,6,7,-8,8a,9-octahydro-4a,8a-trans-1H-pyrrolo[2,3-g]isochinolin-4-on;

2-Methyl-3-äthyl-6-(2-hydroxy-3-methylbutyl)-4,4a,5,6,7,8,8a,9-octahydro-4a,8a-trans-1H-pyrrolo[2,3-g]isochinolin-4-on;

2-Methyl-3-äthyl-6-(2-hydroxy-3,3-dimethylbutyl)-4,4a,5,6,7,8,8a,9-octahydro-4a,8a-trans-1H-pyrrolo[2,3-g]isochinolin-4-on und

2,6-Dimethyl-3-äthyl-4,4a,5,6,7,8,8a,9-octahydro-4a,8a-trans-1H-pyrrolo[2,3-g]-isochinolin-4-on-Hydrochlorid-Dihydrat.

Beispielhaft für die Verbindungen der Formel A, wobei X O ist, sind:

2-Methyl-3-äthyl-4,4a,5,6,7,8,8a,9-octahydro-1H-pyrrolo[2,3-g]isochinolin-4-on;

3-Äthyl-2,6-dimethyl-4,4a,5,6,7,8,8a,9-octahydro-1H-pyrrolo[2,3-g]isochinolin-4-on;

3-Äthyl-1,2,6-trimethyl-4,4a,5,6,7,8,8a,9-octahydro-1H-pyrrolo[2,3-g]isochinolin-4-on;

1-Benzoyl-2,6-dimethyl-3-äthyl-4,4a,5,6,7,8,8a,9-octahydro-1H-pyrrolo[2,3-g]isochinolin-4-on;

3-Äthyl-2-methyl-6-(2-propinyl)-4,4a,5,6,7,8,8a,9-octahydro-1H-pyrrolo[2,3-g]isochinolin-4-on;

(+)-3-Äthyl-2,6-dimethyl-4,4a,5,6,7,8,8a,9-octahydro-4a,8a-trans-1H-pyrrolo[2,3-g]isochinolin-4-on;

(+)-3-Äthyl-2,6-dimethyl-4,4a,5,6,7,8,8a,9-octahydro-4a,8a-trans-1H-pyrrolo[2,3-g]isochinolin-4-on-Hydrochlorid, 0,25 Mol-Hydrat;

3,6-Dimethyl-2-(2-propyl)-4,4a,5,6,7,8,8a,9-octahydro-1H-pyrrolo[2,3-g]isochinolin-4-on-Hydrochlorid;

3,6-Dimethyl-2-(2-propyl)-4,4a,5,6,7,8,8a,9-octahydro-1H-pyrrolo[2,3-g]isochinolin-4-on;

2,6-Dimethyl-3-butyl-4,4a,5,6,7,8,8a,9-octahydro-1H-pyrrolo[2,3-g]isochinolin-4-on;

2-Methyl-3-äthyl-6-[2-hydroxy-2-(4-chlorphenyl)-äthyl]-4,4a,5,6,7,8,8a,9-octahydro-1H-pyrrolo-[2,3-g]isochinolin-4-on;

2-Methyl-3-äthyl-6-(2-hydroxyäthyl)-4,4a,5,6,7,8,-8a,9-octahydro-1H-pyrrolo[2,3-g]isochinolin-4-on;

2-Methyl-3-äthyl-6-(2-propenyl)-4,4a,5,6,7,8,8a,9-octahydro-1H-pyrrolo[2,3-g]isochinolin-4-on;

2-Methyl-3-äthyl-6-(2-propenyl)-4,4a,5,6,7,8,8a,9-octahydro-1H-pyrrolo[2,3-g]isochinolin-4-on-Hydrochlorid, 0,5 Mol-Hydrat;

2-Methyl-äthyl-6-(cyclopropylmethyl)-4,4a,5,6,7,-8,8a,9-octahydro-1H-pyrrolo[2,3-g]isochinolin-4-on;

2-Methyl-3-äthyl-6-(cyclopropylmethyl)-4,4a,5,6,7,8,8a,9-octahydro-1H-pyrrolo[2,3-g]isochinolin-4-on-Hydrochlorid, 0,2 Mol-Hydrat;

2,6-Dimethyl-3-äthyl-1-(2,2-dimethyl-1-oxopropyl)-4,4a,5,6,7,8,8a,9-octahydro-1H-pyrrolo[2,3-g]isochinolin-4-on;

2,6-Dimethyl-3-cyclopropyl-4,4a,5,6,7,8,8a,9-octahydro-1H-pyrrolo[2,3-g]isochinolin-4-on;

3-Äthyl-2-methyl-6-(2-dimethylaminoäthyl)-4,4a,5,6,7,8,8a,9-octahydro-1H-pyrrolo[2,3-g]isochinolin-4-on;

1-Benzyl-2,6-dimethyl-3-äthyl-4,4a,5,6,7,8,8a,9-octahydro-1H-pyrrolo[2,3-g]isochinolin-4-on;

3-Äthyl-2-methyl-4,4a,5,6,7,8,8a,9-octahydro-1H-pyrrolo[2,3-g]isochinolin-4-oxo-6-essigsäureäthylester;

2-Benzyl-3,6-dimethyl-4,4a,5,6,7,8,8a,9-octahydro-1H-pyrrolo[2,3-g]isochinolin-4-on;

3,6-Dimethyl-2-(2-propenyl)-4,4a,5,6,7,8,8a,9-octahydro-1H-pyrrolo[2,3-g]isochinolin-4-on;

2,6-Dimethyl-3-(2-propyl)-4,4a,5,6,7,8,8a,9-octahydro-1H-pyrrolo[2,3-g]isochinolin-4-on;

3-Äthyl-2-methyl-6-[2-hydroxy-3-(4-chlorphenoxy)propyl]-4,4a,5,6,7,8,8a,9-octahydro-1H-pyrrolo[2,3-g]isochinolin-4-on;

3,6-Diäthyl-2-methyl-4,4a,5,6,7,8,8a,9-octahydro-1H-pyrrolo[2,3-g]isochinolin-4-on;

2-Methyl-3-äthyl-6-propyl-4,4a,5,6,7,8,8a,9-octahydro-1H-pyrrolo[2,3-g]isochinolin-4-on;

2-Methyl-3-äthyl-6-butyl-4,4a,5,6,7,8,8a,9-octahydro-1H-pyrrolo[2,3-g]isochinolin-4-on;

2-Methyl-3-äthyl-6-pentyl-4,4a,5,6,7,8,8a,9-octahydro-1H-pyrrolo[2,3-g]isochinolin-4-on;

3,6-Dimethyl-4,4a,5,6,7,8,8a,9-octahydro-1H-pyrrolo[2,3-g]isochinolin-4-on;

6-Methyl-4,4a,5,6,7,8,8a,9-octahydro-1H-pyrrolo-[2,3-g]isochinolin-4-on;

6-Benzyl-4,4a,5,6,7,8,8a,9-octahydro-1H-pyrrolo-[2,3-g]isochinolin-4-on;

2,6-Dimethyl-3-propyl-4,4a,5,6,7,8,8a,9-octahydro-1H-pyrrolo[2,3-g]isochinolin-4-on;

3-Äthyl-2-methyl-6-[2-hydroxy-3-(4-t-butylphenoxy)-propyl)-4,4a,5,6,7,8,8a,9-octahydro-1H-pyrrolo[2,3-g]isochinolin-4-on;

3-Äthyl-2-methyl-6-[2-hydroxy-2-(4-chlor-3-trifluormethylphenyl)äthyl]-4,4a,5,6,7,8,8a,9-octahydro-1H-pyrrolo[2,3-g]isochinolin-4-on;

3-Äthyl-2-methyl-6-(2,-hydroxy-2-adamantyläthyl)-4,4a,5,6,7,8,8a,9-octahydro-1H-pyrrolo[2,3-g]isochinolin-4-on;

3-Äthyl-2-methyl-6-[2-(4-morpholino)äthyl]-4,4a,5,6,7,8,8a,9-octahydro-1H-pyrrolo[2,3-g]isochinolin-4-on;

3-Äthyl-2-methyl-6-[2-oxo-2-(4-fluorphenyl)äthyl]-4,4a,5,6,7,8,8a,9-octahydro-1H-pyrrolo[2,3-g]isochinolin-4-on;

3-Äthyl-2-methyl-6-(2-methylpropyl)-4,4a,5,6,7,8,-8a,9-octahydro-1H-pyrrolo[2,3-g]isochinolin-4-on;

3-Äthyl-2-methyl-6-cyclobutylmethyl-4,4a,5,6,7,8,-8a,9-octahydro-1H-pyrrolo[2,3-g]isochinolin-4-on;

3-Äthyl-2-methyl-6-hexyl-4,4a,5,6,7,8,8a,9-octahydro-1H-pyrrolo[2,3-g]isochinolin-4-on;

3-Äthyl-2-methyl-6-heptyl-4,4a,5,6,7,8,8a,9-octahydro-1H-pyrrolo[2,3-g]isochinolin-4-on;

3-Äthyl-2-methyl-6-[2-(1-pyrrolidinyl)äthyl]-4,4a,-5,6,7,8,8a,9-octahydro-1H-pyrrolo[2,3-g]isochinolin-4-on;

3-Äthyl-2-methyl-6-(4-methoxy-2-phenyläthyl)-4,-4a,5,6,7,8,8a,9-octahydro-1H-pyrrolo[2,3-g]isochinolin-4-on;

2,6-Dimethyl-3-äthyl-1-(1-oxobutyl)-4,4a,5,6,7,8,-8a,9-octahydro-1H-pyrrolo[2,3-g]isochinolin-4-on;

3-Äthyl-2-methyl-6-(4-chlor-2-phenyläthyl)-4,4a,5,6,7,8,8a,9-octahydro-1H-pyrrolo[2,3-g]isochinolin-4-on;

2,6-Dimethyl-3-propyl-4,4a,5,6,7,8,8a,9-octahydro-4a,8a-trans-1H-pyrrolo[2,3-g]isochinolin-4-on;

2,3-Dimethyl-6-(2-phenyläthyl)-4,4a,5,6,7,8,8a,9-octahydro-4a,8a-trans-1H-pyrrolo[2,3-g]isochinolin-4-on;

2,3-Dimethyl-6-[4-(4-fluorphenyl)-4-oxobutyl]-4,4a,5,6,7,8,8a,9-octahydro-4a,8a-trans-1H-pyrrolo[2,3-g]isochinolin-4-on;

3-Äthyl-2-methyl-6-(2,2,2,-trifluoräthyl)-4,4a,5,6,-7,8,8a,9-octahydro-4a,8a-trans-1H-pyrrolo[2,3-g]isochinolin-4-on;

2,6-Dimethyl-3-äthyl-4,4a,5,6,7,8,8a,9-octahydro-4a,8a-cis-1H-pyrrolo[2,3-g]isochinolin-4-on;

2-Acetyl-3,6-dimethyl-4,4a,5,6,7,8,8a,9-octahydro-4a,8a-trans-1H-pyrrolo[2,3-g]isochinolin-4-on;

3-Äthyl-2-methyl-6-[2-(2-thienyl)äthyl]-4,4a,5,6,7,8,8a,9-octahydro-4a,8a-trans-1H-pyrrolo[2,3-g]isochinolin-4-on;

3-Äthyl-2-methyl-6-[2-(2-furyl)äthyl]-4,4a,5,6,7,8,-8a,9-octahydro-4a,8a-trans-1H-pyrrolo[2,3-g]isochinolin-4-on und

3-Äthyl-2-methyl-6-[2-(1,3-dihydro-2(2H)-benzimidazolonyl)-äthyl]-4,4a,5,6,7,8,8a,9-octahydro-4a,8a,trans-1H-pyrrolo[2,3-g]isochinolin-4-on.

Die am meisten bevorzugten Verbindungen der Formel A gemäss der Erfindung, worin X S ist, sind:

2-Methyl-3-äthyl-6-(2-phenyläthyl)-4,4a,5,6,7,8,-8a,9-octahydro-4a,8a-trans-1H-pyrrolo[2,3-g]isochinolin-4-thion;

2,6-Dimethyl-3-äthyl-4,4a,5,6,7,8,8a,9-octahydro-4a,8a-trans-1H-pyrrolo[2,3-g]isochinolin-4-thion;

2-Methyl-3-äthyl-6-[4-(4-fluorphenyl)-4-oxobutyl]-4,4a,5,6,7,8,8a,9-octahydro-4a,8a-trans-1H-pyrrolo[2,3-g]isochinolin-4-thion, und

2-Methyl-3-äthyl-6-(2-hydroxy-3,3-dimethylbutyl)4,4a,5,6,7,8,8a,9-octahydro-4a,8a-trans-1H-pyrrolo[2,3-g]isochinolin-4-thion.

Beispiele für Verbindungen der Formel A, worin X S ist, sind:

2-Methyl-3-äthyl-6-(2-hydroxy-2-phenyläthyl)-4,4a,5,6,7,8,8a,9-octahydro-4a,8a-trans-1H-pyrrolo[2,3-g]isochinolin-4-thion;

2,3,6-Trimethyl-4,4a,5,6,7,8,8a,9-octahydro-4a,8a-trans-1H-pyrrolo[2,3-g]isochinolin-4-thion;

2-Methyl-3-äthyl-6-benzyl-4,4a,5,6,7,8,8a,9-octahydro-4a,8a-trans-1H-pyrrolo[2,3-g]isochinolin-4-thion;

2-Methyl-3-äthyl-6-(2-äthoxyäthyl)-4,4a,5,6,7,8,8a,9-octahydro-4a,8a-trans-1H-pyrrolo[2,3-g]isochinolin-4-thion;

2-Methyl-3-äthyl-6-[3-(4-fluorphenyl)-3-oxopropyl]-4,4a,5,6,7,8,8a,9-octahydro-4a,8a-trans-1H-pyrrolo[2,3-g]isochinolin-4-thion;

2-Methyl-3-äthyl-6-(3-phenoxypropyl)-4,4a,5,6,7,8,8a,9-octahydro-4a,8a-trans-1H-pyrrolo[2,3-g]isochinolin-4-thion;

2-Methyl-3-äthyl-6-(2-hydroxy-3-methylbutyl)-4,4a,5,6,7,8,8a,9-octahydro-4a,8a-trans-1H-pyrrolo[2,3-g]isochinolin-4-thion und

2-Methyl-3-äthyl-4,4a,5,6,7,8,8a,9-octahydro-1H-pyrrolo[2,3-g]isochinolin-4-thion.

Die erfindungsgemässen Verbindungen können als 4a,8a-trans- oder 4a,8a-cis-Isomere oder deren Gemische auftreten; die 4a,8a-trans-Isomeren sind bevorzugt.

Die Verbindungen der obigen Formel A, ihre optischen und geometrischen Isomeren und ihre pharmazeutisch annehmbaren Säureadditionssalze können erfindungsgemäss so hergestellt werden, dass man

a) zur Herstellung einer Verbindung der allgemeinen Formel

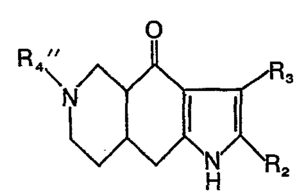

Ia,

worin

$R_4''$ ($C_1$–$C_7$)-Alkyl, ($C_1$–$C_7$)-Alkoxy-($C_1$–$C_7$-alkyl

oder ($C_3$–$C_6$)-Cycloalkyl-($C_1$–$C_7$)-alkyl und $R_2$ und $R_3$ wie zuvor beschrieben sind, eine Verbindung der allgemeinen Formel

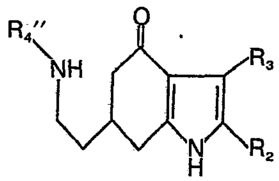

IX,

worin $R_2$, $R_3$ und $R_4''$ wie zuvor beschrieben sind, mit Formaldehyd behandelt, oder

b) zur Herstellung einer Verbindung der obigen Formel Ia eine Verbindung der allgemeinen Formel

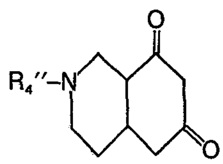

XII,

worin $R_4''$ wie zuvor beschrieben ist, mit einer Verbindung der allgemeinen Formel

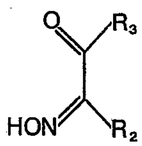

VII

in Gegenwart eines Reduktionsmittels, oder mit einer Verbindung der allgemeinen Formel

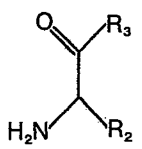

VIII,

worin $R_2$ und $R_3$ wie zuvor beschrieben sind, oder einer Vorstufe von dieser, behandelt, oder

c) zur Herstellung einer Verbindung der allgemeinen Formel

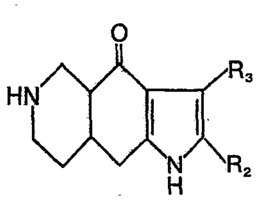

Ib

worin $R_2$ und $R_3$ wie zuvor beschrieben sind, eine Verbindung der obigen Formel Ia, worin $R_4''$ Methyl ist, N-demethyliert, oder

d) zur Herstellung einer Verbindung der allgemeinen Formel

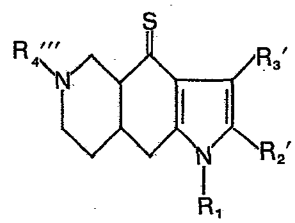

IIc'',

worin $R_2'$ und $R_3'$ unabhängig voneinander Wasserstoff, ($C_1$–$C_7$)-Alkyl, ($C_3$–$C_6$)-Cycloalkyl, ($C_2$–$C_7$)-Alkenyl oder jeweils gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Phenyl oder Phenyl-($C_1$–$C_7$)-alkyl, $R_4'''$ Wasserstoff, ($C_1$–$C_7$)-Alkyl, ($C_1$–$C_7$)-Alkoxy-($C_1$–$C_7$)-alkyl, ($C_2$–$C_7$)-Alkenyl, ($C_3$–$C_6$)-Cycloalkyl-($C_1$–$C_7$)-alkyl, ($C_2$–$C_7$)-Alkinyl, Thienyl-($C_1$–$C_7$)-alkyl, Furyl-($C_1$–$C_7$)-alkyl, ($C_2$–$C_7$)-Alkenyloxy-($C_1$–$C_7$)-alkyl oder jeweils gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Phenyl-($C_1$–$C_7$-alkyl, Phenyl-($C_2$–$C_7$)-alkenyl oder Phenoxy-($C_1$–$C_7$)-alkyl oder Trifluoralkyl mit 2 bis 6 Kohlenstoffatomen und $R_1$ wie zuvor beschrieben sind, eine Verbindung der allgemeinen Formel

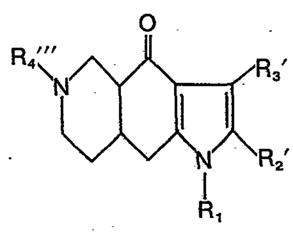

Ic'',

worin $R_1$, $R_2'$, $R_3'$ und $R_4'''$ wie zuvor beschrieben sind, mit Phosphorpentasulfid behandelt, oder

e) zur Herstellung einer Verbindung der allgemeinen Formel

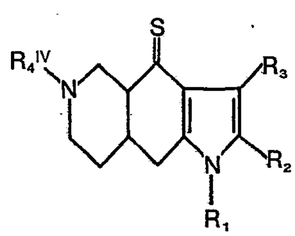

IIc''',

worin
$R_4^{IV}$ Hydroxy-($C_1$–$C_7$)-alkyl,
Phenyl-hydroxy-($C_1$–$C_7$)-alkyl
Halogenphenyl-hydroxy-($C_1$–$C_7$)-alkyl
($C_1$–$C_7$)-Alkyl-phenyl-hydroxy-($C_1$–$C_7$)alkyl,

$(C_1–C_7)$-Alkoxyphenyl-hydroxy-$(C_1–C_7)$-alkyl, $(C_1–C_7)$-Alkoxy-hydroxy-$(C_1–C_7)$-alkyl, $(C_1–C_7)$-Alkanoyloxy-$(C_1–C_7)$-alkyl, $(C_1–C_7)$-Alkoxycarbonyl-$(C_1–C_7)$-alkyl, $(C_1–C_7)$-Alkanoyl-$(C_1–C_7)$-alkyl, $(C_3–C_6)$-Cycloalkoxy-$(C_1–C_7)$-alkyl, $(C_3–C_6)$-Cycloalkyl-hydroxy-$(C_1–C_7)$-alkyl, N-$(C_1–C_7)$-Alkyl-pyrrolidinyl-$(C_1–C_7)$-alkyl oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Phenyl-N-imidazolonyl-$(C_1–C_7)$-alkyl, Phenoxy-hydroxy-$(C_1–C_7)$-alkyl, Benzoyloxy-$(C_1–C_7)$-alkyl, Benzoyl-$(C_1–C_7)$-alkyl oder Phenylcarboxamido-$(C_1–C_7)$-alkyl oder

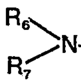N-$(C_1–C_7)$-Alkyl bedeutet und $R_1$, $R_2$, $R_3$, $R_6$ und $R_7$ wie zuvor beschrieben sind, die Schutzgruppe oder -gruppen in einer Verbindung der allgemeinen Formel

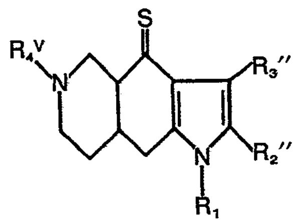

IIf,

worin $R_2''$ und $R_3''$ die gleiche Bedeutung wie $R_2$ und $R_3$ haben, ausgenommen wenn $R_2$ und $R_3$ $(C_1–C_7)$-Alkanoyl oder gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Benzoyl bedeuten, sind $R_2''$ und $R_3''$ in geschützter Form, $R_4^V$ die gleiche Bedeutung wie $R_4^{IV}$ hat, aber in geschützter Form, und $R_1$ wie zuvor beschrieben ist, abspaltet, oder

f) zur Herstellung einer Verbindung der allgemeinen Formel

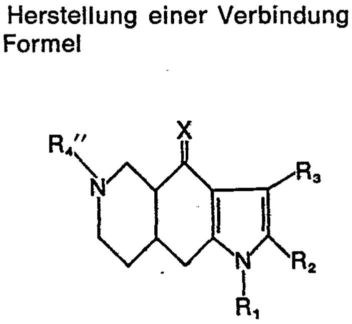

Ac,

worin $R_1'$ $(C_1–C_7)$-Alkyl, $(C_1–C_7)$-Alkanoyl oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Benzoyl oder Phenyl-$(C_1–C_7)$-alkyl und $R_2$, $R_3$, $R_4''$ und X wie zuvor beschrieben sind, eine Verbindung der allgemeinen Formel

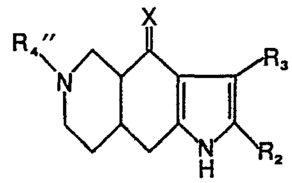

Aa,

worin $R_2$, $R_3$, $R_4''$ und X wie zuvor beschrieben sind, am Pyrrol-Stickstoffatom substituiert, oder

g) zur Herstellung einer Verbindung der allgemeinen Formel

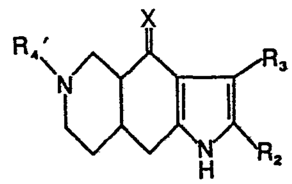

Ad,

worin

$R_4'$ $(C_1–C_7)$-Alkyl, Hydroxy-$(C_1–C_7)$-alkyl, Phenyl-hydroxy-$(C_1–C_7)$-alkyl, Halogenphenyl-hydroxy-$(C_1–C_7)$-alkyl, $(C_1–C_7)$-Alkylphenyl-hydroxy-$(C_1–C_7)$-alkyl, $(C_1–C_7)$-Alkoxyphenyl-hydroxy-$(C_1–C_7)$-alkyl, $(C_1–C_7)$-Alkoxy-$(C_1–C_7)$-alkyl, $(C_1–C_7)$-Alkoxy-hydroxy-$(C_1–C_7)$-alkyl, $(C_1–C_7)$-Alkanoyloxy-$(C_1–C_7)$-alkyl, $(C_1–C_7)$-Alkoxycarbonyl-$(C_1–C_7)$-alkyl, $(C_2–C_7)$-Alkenyl, $(C_3–C_6)$-Cycloalkyl-$(C_1–C_7)$-alkyl, $(C_2–C_7)$-Alkinyl, Thienyl-$(C_1–C_7)$-alkyl, Furyl-$(C_1–C_7)$-alkyl, $(C_1–C_7)$-Alkanoyl-$(C_1–C_7)$-alkyl, $(C_3–C_6)$-Cycloalkoxy-$(C_1–C_7)$-alkyl, $(C_3–C_6)$-Cycloalkyl-hydroxy-$(C_1–C_7)$-alkyl, $(C_2–C_7)$-Alkenyloxy-$(C_1–C_7)$-alkyl, N-$(C_1–C_7)$-Alkyl-pyrrolidinyl-$(C_1–C_7)$-alkyl, Trifluoralkyl mit 2 bis 6 Kohlenstoffatomen oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Phenoxy-hydroxy-$(C_1–C_7)$-alkyl, Benzoyloxy-$(C_1–C_7)$-alkyl, Benzoyl-$(C_1–C_7)$-alkyl, Phenyl-$(C_1–C_7)$-alkyl, Phenylcarboxamido-$(C_1–C_7)$-alkyl, Phenyl-$(C_2–C_7)$-alkenyl, Phenoxy-$(C_1–C_7)$-alkyl oder Phenyl-N-imidazolonyl-$(C_1–C_7)$-alkyl oder 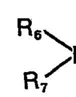N-$(C_1–C_7)$-Alkyl bedeutet und $R_2$, $R_3$, $R_6$, $R_7$ und X wie zuvor beschrieben sind,

eine Verbindung der allgemeinen Formel

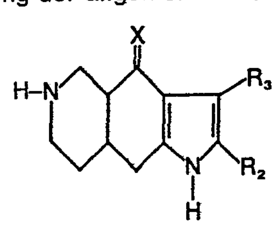

Ab,

worin $R_2$, $R_3$ und X wie zuvor beschrieben sind, am Isochinolin-Stickstoffatom substituiert, oder

h) zur Herstellung einer Verbindung der allgemeinen Formel

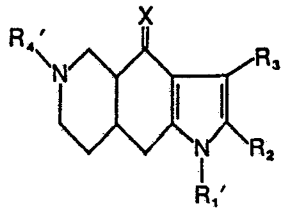

Af,

worin $R_1'$, $R_2$, $R_3$, $R_4'$ und X wie zuvor beschrieben sind, eine Verbindung der folgenden Formel Ae am Isochinolin-Stickstoffatom substituiert, oder

i) zur Herstellung einer Verbindung der allgemeinen Formel

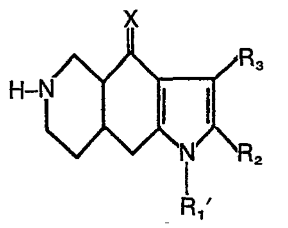

Ae,

worin $R_1'$, $R_2$, $R_3$ und X wie zuvor beschrieben sind, die Schutzgruppe in einer Verbindung der allgemeinen Formel

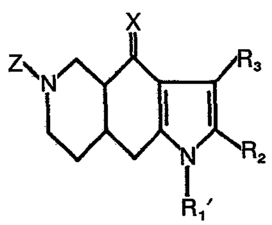

Ah,

worin Z eine Schutzgruppe ist und $R_1'$, $R_2$, $R_3$ und X wie zuvor beschrieben sind, abspaltet, oder

j) zur Herstellung einer Verbindung der allgemeinen Formel

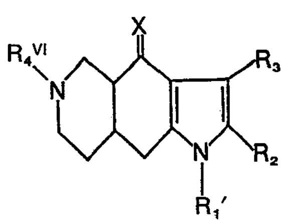

Af',

worin $R_4^{VI}$ Wasserstoff,
$(C_1–C_7)$-Alkyl, $(C_1–C_7)$-Alkoxy-$(C_1–C_7)$-alkyl,
$(C_1–C_7)$-Alkanoyloxy-$(C_1–C_7)$-alkyl,
$(C_1–C_7)$-Alkoxycarbonyl-$(C_1–C_7)$-alkyl,
$(C_2–C_7)$-Alkenyl,
$(C_3–C_6)$-Cycloalkyl-$(C_1–C_7)$-alkyl,
$(C_2–C_7)$-Alkinyl, Thienyl-$(C_1–C_7)$-alkyl,
Furyl-$(C_1–C_7)$-alkyl,
$(C_1–C_7)$-Alkanoyl-$(C_1–C_7)$-alkyl,
$(C_3–C_6)$-Cycloalkyloxy-$(C_1–C_7)$-alkyl,
$(C_2–C_7)$-Alkenyloxy-$(C_1–C_7$-alkyl,
N-$(C_1–C_7)$-Alkyl-pyrrolidinyl-$(C_1–C_7)$-alkyl,
Trifluoralkyl mit 2 bis 6 Kohlenstoffatomen oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di$(C_1–C_7)$-alkylamino substituiertes Phenyl-N-imidazolonyl-$(C_1–C_7)$-alkyl, Benzoyloxy-$(C_1–C_7)$-alkyl, Phenyl-$(C_1–C_7)$-alkyl, Phenyl-carboxamido-$(C_1–C_7)$-alkyl, Benzoyl-$(C_1–C_7)$-alkyl, Phenyl-$(C_2–C_7)$-alkenyl oder Phenoxy-$(C_1–C_7)$-alkyl bedeutet und $R_1'$, $R_2$, $R_3$ und X wie zuvor beschrieben sind, eine Verbindung der allgemeinen Formel

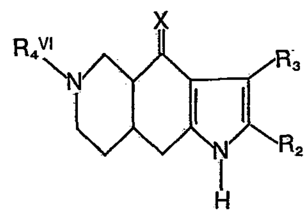

Ad',

worin $R_2$, $R_3$, $R_4^{VI}$ und X wie zuvor beschrieben sind, am Pyrrol-Stickstoffatom substituiert, oder

k) zur Herstellung einer Verbindung der allgemeinen Formel

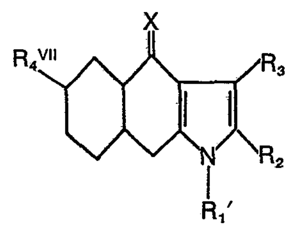

Af''

worin
$R_4^{VII}$ Hydroxy-$(C_1–C_7)$-alkyl,
Phenyl-hydroxy-$(C_1–C_7)$-alkyl,
Halogenphenyl-hydroxy-$(C_1–C_7)$-alkyl,
$(C_1–C_7)$-Alkylphenyl-hydroxy-$(C_1–C_7)$-alkyl,
$(C_1–C_7)$-Alkoxyphenyl-hydroxy-$(C_1–C_7)$-alkyl,
$(C_1–C_7)$-Alkoxy-hydroxy-$(C_1–C_7)$-alkyl,
$(C_3–C_6)$-Cycloalkyl-hydroxy-$(C_1–C_7)$-alkyl, 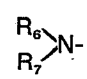
$(C_1–C_7)$-Alkyl oder gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Phenoxy-hydroxy-$(C_1–C_7)$-alkyl bedeutet und $R_1'$, $R_2$, $R_3$, $R_6$, $R_7$ und X wie zuvor beschrieben sind, die Schutzgruppe in einer Verbindung der allgemeinen Formel

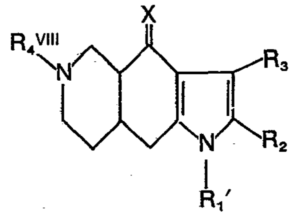

Af'''

worin $R_4^{VIII}$ die gleiche Bedeutung wie $R_4^{VII}$ hat, jedoch in geschützter Form, und $R_1'$, $R_2$, $R_3$ und X wie zuvor beschrieben sind, abspaltet, oder

l) das erhaltene Gemisch der cis- und trans-Isomeren zu einem Endverhältnis, das überwiegend das trans-Isomere aufweist, isomerisiert, oder

m) das trans-Isomere aus dem erhaltenen Gemisch abtrennt, oder

n) ein erhaltenes racemisches Gemisch in die optischen Antipoden aufspaltet, und

o) wenn gewünscht, eine erhaltene Verbindung oder ein pharmazeutisch nicht annehmbares Säureadditionssalz in ein pharmazeutisch annehmbares Säureadditionssalz überführt.

Insbesondere können die Verbindungen der obigen Formel A, ihre optischen und geometrischen Isomeren und ihre pharmazeutisch annehmbaren Säureadditionssalze sowie verschiedene Zwischenstufen wie nachfolgend im einzelnen veranschaulicht hergestellt werden.

So werden z.B. die Verbindungen der Formel A, worin X O ist, durch die Formel

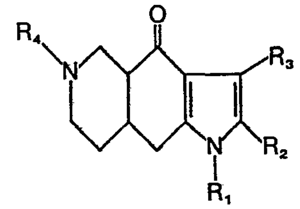

I

charakterisiert, worin $R_1$, $R_2$, $R_3$ und $R_4$ wie zuvor beschrieben sind, und können hergestellt werden, wie in den Schemata I bis IV aufgeführt und weiter beschrieben.

Formelschema I

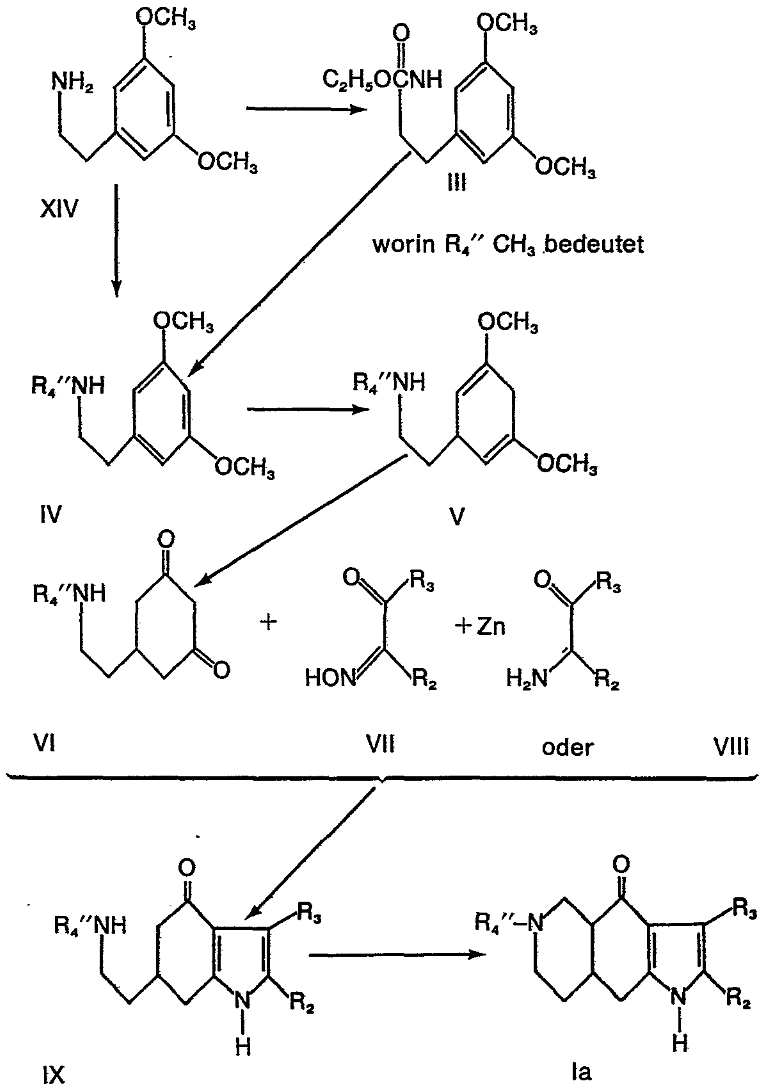


XIV III IV V VI VII oder VIII IX Ia

worin $R_2$, $R_3$ und $R_4''$ wie zuvor beschrieben sind.

Nach dem Formelschema I werden Verbindungen der Formel IV, worin $R_4''$ Methyl ist, durch Umsetzen des primären Amins der Formel XIV mit Chlorameisensäureäthylester zum Urethan der Formel III hergestellt, das mit Lithiumaluminiumhydrid zu dem N-Methylamin der Formel IV reduziert wird. Gemäss einem breiteren Aspekt können die Verbindungen der Formel IV durch reduktive Alkylierung der Verbindung der Formel XIV unter Einsatz des entsprechenden Aldehyds, z.B. Acetaldehyd u.dgl., und Natriumcyanoborhydrid unter bekannten Bedingungen (vgl. z.B. R.F. Borch, J. Am. Chem. Soc., 93, 2897 (1971) hergestellt werden. Die Birch-Reduktion des Amins der Formel IV mit Lithium in t-Butanol enthaltendem

Ammoniak liefert das Dihydroamin der Formel V. Andere Abwandlungen der Birch-Reduktion können auch angewandt werden. So kann das Amin der Formel IV mit einem Alkalimetall, wie Natrium, Lithium, Kalium oder Cäsium, in Ammoniak oder einem Amin, wie Methylamin oder Äthylamin in Gegenwart eines niederen Alkanols, wie Äthanol, Butanol oder t-Butanol, umgesetzt werden. Die Umsetzung erfolgt im allgemeinen beim Siedepunkt des Lösungsmittels oder darunter, z.B. von −78 bis 15 °C. Wird Ammoniak verwendet, erfolgt die Reaktion bei Rückfluss. Gegebenenfalls können Cosolventien, wie Diäthyläther oder Tetrahydrofuran, zugesetzt werden.

Die Hydrolyse des Dihydroamins der Formel V erfolgt leicht nach üblichen Methoden der Hydrolyse von Enoläthern, z.B. mit wässriger Säure. Beispielhaft für verwendbare Säuren sind Salzsäure, Bromwasserstoffsäure, Ameisensäure, Essigsäure, p-Toluolsulfonsäure und Perchlorsäure. Sie können in wässrigen Lösungen oder gemischten Lösungsmitteln verwendet werden. Mindestens zwei Äquivalente Wasser pro Mol Dihydroamin und mehr als 1 Äquivalent Säure sind nötig. Tetrahydrofuran, Benzol, Diäthyläther, Aceton, Toluol, Dioxan oder Acetonitril sind Beispiele für verwendbare Lösungsmittel. Beispielsweise führt die Hydrolyse des Dihydroamins der Formel V, worin $R_4''$ Methyl ist, in 2n Salzsäure bei Raumtemperatur oder darüber in wässriger Essigsäure zwischen 40° und Rückfluss zu dem Diketon der Formel VI, worin $R_4''$ Methyl ist.

Das Diketon der Formel VI wird in einer Knorr-Kondensation zu dem Dihydroindolon-äthylamin der Formel IX kondensiert. Die Knorr-Kondensation ist ein wohlbekanntes Verfahren zur Herstellung von Pyrrolen, und das Verfahren kann bei jeder der bekannten Abwandlungen angewandt werden (zu beispielsweisen Bedingungen vgl. J.M. Patterson, Synthesis, 281, 1976 und die dort genannten Literaturstellen). So wird angenommen, dass z.B. die Reaktion eines Isonitrosoketons der Formel VII in Gegenwart eines Reduktionsmittels, z.B. mit Zink in wässriger Essigsäure oder Salzsäure, über die Aminocarbonylverbindung der Formel VIII verläuft, die dann mit dem Diketon der Formel VI zu dem Dihydroindolon-äthylamin der Formel IX kondensiert. Anderseits kann die Kondensation mit einer Aminocarbonylverbindung der Formel VIII oder einer Vorstufe, wie einem Aminoketon-Hydrochlorid oder einem Acetalderivat eines Aminoketons oder Aminoaldehyds, erfolgen. Die Verwendung einer Vorstufe des Aminoketons oder Aminoaldehyds wird bevorzugt, da solche Substanzen leicht zur Selbstkondensation neigen. Sie können am besten in situ verwendet werden, wo die Aminocarbonyl-Komponente in Gegenwart des Diketons der Formel VI freigesetzt wird. Die Aminocarbonyl-Komponente reagiert sofort zu dem Dihydroindolon-äthylamin der Formel IX. Das Diketon der Formel VI muss nicht notwendigerweise vor der Durchführung der Knorr-Kondensation isoliert werden, da die angewandten Reaktionsbedingungen zur Hydrolyse des Dihydroamins der Formel V zu dem Diketon der Formel VI ausreichen. Die Knorr-Kondensation wird am besten bei einem pH von etwa 2 bis 6 durchgeführt. Bei einem pH wesentlich über 6 tritt erheblicher Ausbeutenverlust ein, und zwar aufgrund der Bildung von Selbstkondensationsprodukten der Aminocarbonylverbindung der Formel VIII.

Vorzugsweise wird ein Isonitrosoketon der Formel VII und Zinkstaub in wässriger Essigsäure mit einem Diketon der Formel VI kondensiert, worin $R_4''$ Methyl ist, um das Produkt, das Dihydroindolon-äthylamin der Formel IX zu ergeben, worin $R_4''$ Methyl ist.

Die Knorr-Kondensation erfolgt bevorzugt bei einem Temperaturbereich von etwa Raumtemperatur bis Rückfluss. Die Isonitrosoketone der Formel VII sind bekannte Verbindungen [siehe z.B. Ferris, J. Org. Chem., 24, 1726 (1959)] oder können leicht durch Nitrosierung der entsprechenden Ketone, z.B. mit einem Alkylnitrit, oder im Falle stark saurer β-Diketone oder β-Ketoester mit Natriumnitrit, hergestellt werden.

Beispiele für bei der Knorr-Kondensation verwendbare Isonitrosoketone sind folgende:
2-Isonitroso-3-pentanon;
2,3-Butandion-monoxim;
2-Isonitroso-4-methyl-3-pentanon;
2-Isonitroso-3-hexanon;
2-Isonitroso-3-heptanon;
3-Isonitroso-4-methyl-2-pentanon;
2-Isonitroso-1-cyclopropyl-1-propanon;
3-Isonitroso-5-hexen-2-on;
Cyclopropyl-2-isonitroso-1-propanon und
3-Isonitroso-4-phenyl-2-butanon.

Beispiele für bei der Knorr-Kondensation verwendbare Aminocarbonyl-Vorstufen sind folgende:
Aminoacetaldehyd-dimethylacetal und
2-Amino-3-pentanon-Hydrochlorid.

Das Amin der Formel IX wird in die Verbindung der Formel Ia über eine intramolekulare Mannich-Reaktion überführt. Die Mannich-Reaktion wird gewöhnlich von einem Keton und einem Dialkylaminsalz, z.B. Dimethylamin-Hydrochlorid und Formaldehyd (z.B. als wässrige Lösung, als Paraformaldehyd oder als Trioxan) in einem alkoholischen Lösungsmittel, wie Äthanol, ausgehend, bei Rückfluss durchgeführt. Bei der hier beschriebenen Ausführungsform wird ein Säureadditionssalz des Dihydroindolon-äthylamins der Formel IX mit Formaldehyd, zugesetzt in Form von Paraformaldehyd, Trioxan oder als wässriger Formaldehyd in einem Lösungsmittel, umgesetzt. Beispielsweise kann ein hochsiedendes hydroxylisches Lösungsmittel, wie Amylalkohol, Octanol, Äthylenglykol oder Diäthylenglykol-monoäthyläther, ein hochsiedendes polares aprotisches Lösungsmittel, wie Dimethylformamid, N-Methylpyrrolidinon oder Diäthylenglykoldimethyläther, ein tiefsiedendes polares Lösungsmittel, wie Äthanol, Butanol oder 2-Propanol unter Druck, oder ein tiefsiedendes aprotisches Lösungsmittel unter Druck, wie Dioxan oder Tetrahydrofuran, bei einer Temperatur im Bereich von etwa 135 bis etwa 200° verwendet werden, um die Pyrrolo-[2,3-g]isochinoline der

Formel Ia zu ergeben. Die Reaktion führt insbesondere bei Temperaturen unter 150° zu einem Gemisch von cis- und trans-Isomeren, d.h. z.B.

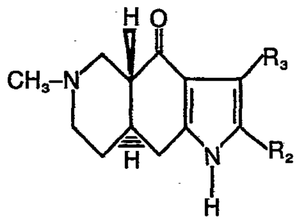

I'a' trans

Längeres Erwärmen des Reaktionsgemisches oder getrenntes Erwärmen des Isomerengemisches der Hydrochloride der Formeln I'a und I''a, z.B. in Äthylenglykol 2 h unter Rückfluss, kann zur Gleichgewichtseinstellung der cis- und trans-Isomeren zu einem Endverhältnis angewandt werden, das überwiegend das trans-Isomere enthält, wenn $R_4''$ Methyl ist, zu Verbindungen der Formeln

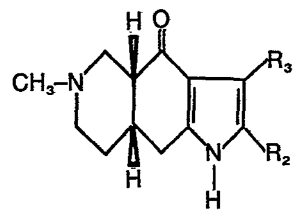

I''a' cis

das leicht durch Kristallisation oder durch chromatographische Trennung isoliert werden kann.

Bevorzugt ist die Reaktion des Hydrochlorids des Dihydroindolon-äthylamins der Formel IX, worin $R_4''$ Methyl ist, mit Paraformaldehyd in Octanol bei 180° für 2 h, wobei das Produkt fast ausschliesslich als das trans-Isomere I'a isoliert wird.

Formelschema II

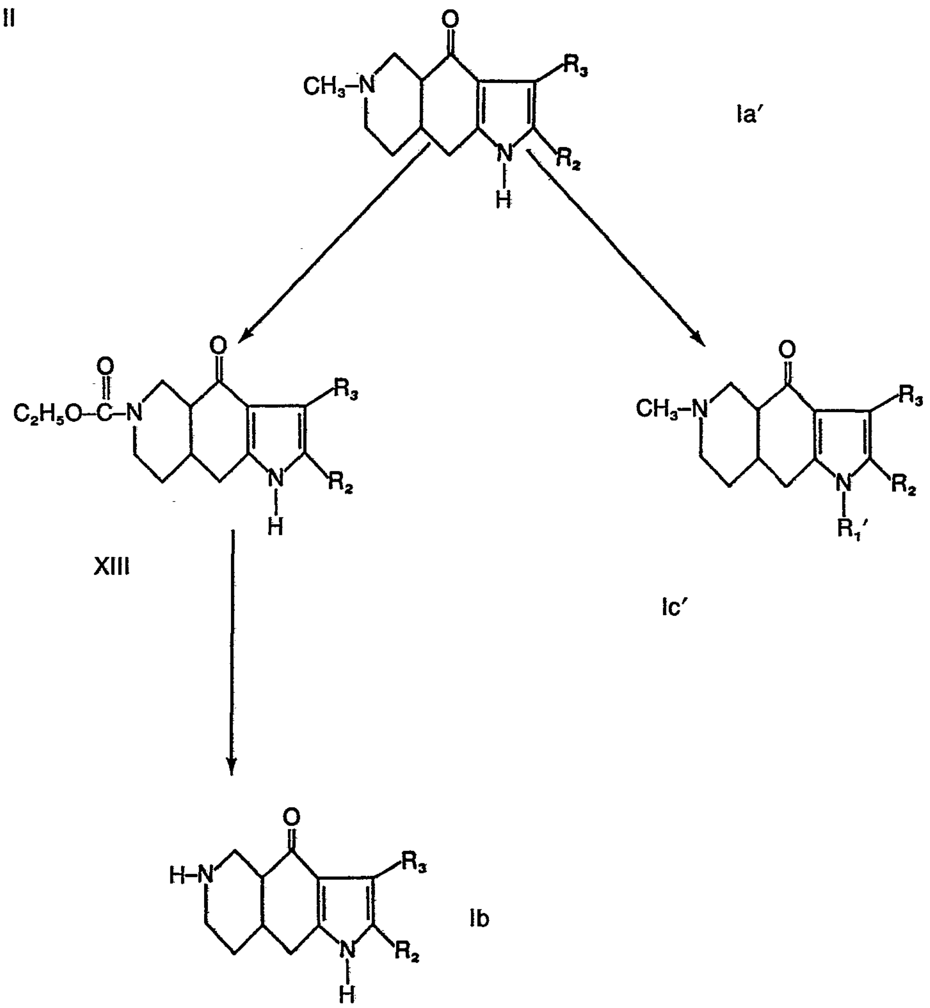

Ia'

XIII

Ic'

Ib

worin $R_1$, $R_2$ und $R_3$ wie zuvor beschrieben sind.

Nach dem Formelschema II werden Verbindungen der Formel Ic' durch Alkylieren oder Acylieren des Pyrrol-Stickstoffs einer Verbindung der Formel Ia' und anderer N-6-Alkylderivate durch Bildung des Pyrrol-Anions mit starker Base, z.B. Natriumamid, Kaliumhydrid, Natriummethylsulfinylcarbanion oder Butyllithium, oder mit einem

Alkalimetall und anschliessendes Abschrecken mit einem Alkyl- oder Acylhalogenid in einem Lösungsmittel, wie Tetrahydrofuran, Dioxan, Äthyläther, Dimethylformamid oder Dimethylsulfoxid, hergestellt. Beispielsweise liefert die Behandlung einer Verbindung der Formel Ia′, worin $R_2$ Methyl und $R_3$ Äthyl ist, mit Natrium in flüssigem Ammoniak und anschliessendes Abschrecken mit Methyljodid das 1-Methylderivat, d.h. eine Verbindung der Formel Ic′, worin $R_2$ Methyl, $R_3$ Äthyl und $R_1'$ Methyl ist. Ähnlich liefert die Reaktion einer Verbindung der Formel Ia′, worin $R_2$ Methyl und $R_3$ Äthyl ist, mit Butyllithium in Tetrahydrofuran bei −30° und anschliessendes Abschrecken mit Benzoylchlorid das 1-Benzoylderivat, d.h. eine Verbindung der Formel Ic′, worin $R_1'$ Benzoyl, $R_2$ Methyl und $R_3$ Äthyl ist.

Die N-Entmethylierung der Verbindung der Formel Ia′ kann nach für die N-Entalkylierung üblichen Standardverfahren erfolgen, wie die von Braun-Methode [H.A. Hageman, Org. Reactions, 7, 198 (1953)] oder über Säuren- oder Basenhydrolyse eines Urethan-Derivats, wie von K.C. Rice [J. Org. Chem. 40, 1850 (1975) aufgeführt. Eine Arbeitsweise zur Entalkylierung der Verbindung der Formel Ia′ verläuft über das Urethan der Formel XIII und Säurehydrolyse zum sec.-Amin der Formel Ib. Beispielsweise wird eine Verbindung der Formel Ia′, worin $R_2$ Methyl und $R_3$ Äthyl ist, in Diäthylketon mit überschüssigem Chlorameisensäureäthylester und Kaliumbicarbonat 3 h rückflussgekocht, um eine Verbindung der Formel XIII zu liefern, worin $R_2$ Methyl und $R_3$ Äthyl ist. Hydrolyse der vorstehenden Verbindung mit konzentrierter Salzsäure in Essigsäure unter 24stündigem Rückfluss liefert eine Verbindung der Formel Ib, worin $R_2$ Methyl und $R_3$ Äthyl ist. Die gleiche Verbindung wird durch Hydrolyse des Urethans der Formel XIII mit Natriumhydroxid in rückflusskochendem Äthanol erhalten.

Formelschema III

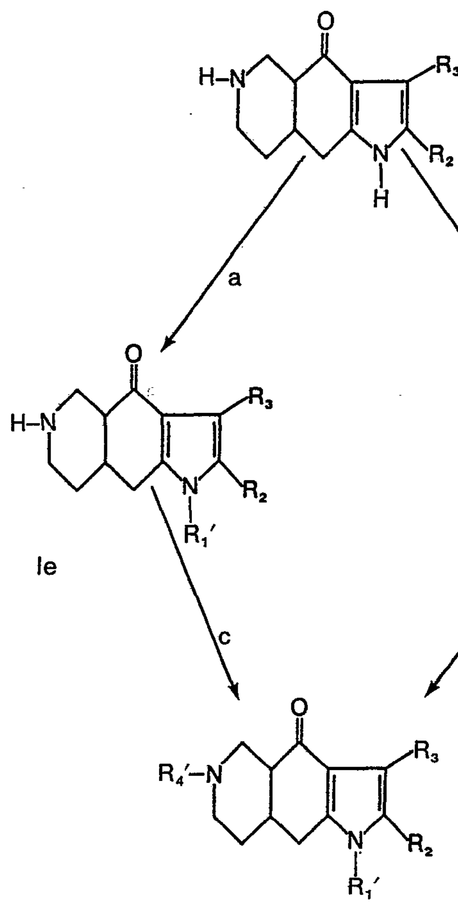


Ib

Ie

Id

Ic

worin $R_1'$, $R_2$, $R_3$ und $R_4'$ wie zuvor beschrieben sind.

Nach dem Formelschema III werden die Verbindungen der Formeln Ic, Id und Ie aus dem sec.-Amin der Formel Ib, dem Ausgangsmaterial für die Herstellung zahlreicher von der Formel I umfasster Derivate, durch Substitution am basischen Amin-Stickstoff (N-6) und/oder am Pyrrol-Stickstoff (N-1) hergestellt. Beispielsweise liefert die Behandlung einer Verbindung der Formel Ib mit einem Alkylhalogenid, wie Äthylbromid, einem Alkenylhalogenid, wie Allylbromid, einem Cycloalkylalkylhalogenid, wie Chlormethylcyclopropan, oder einem Aralkylhalogenid, wie Benzylbromid, in Gegenwart einer Base, z.B. Kaliumcarbonat, in Aceton, 2-Propanon oder Dimethylformamid, die entsprechend substituierte Verbindung der Formel Id, d.h. worin $R_4'$ ($C_1$–$C_7$)-Alkyl, ($C_2$–$C_7$)-Alkenyl, ($C_3$–$C_6$)-Cycloalkyl-($C_1$–$C_7$)-alkyl bzw. gegebenenfalls am Phenylring durch Halogen, Trifluor-

metyhl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Phenyl-($C_1$–$C_7$)-alkyl ist. Mit reaktiven Halogeniden kann die Reaktion bei Raumtemperatur ablaufen; mit weniger reaktiven Halogeniden werden Rückflusstemperaturen angewandt, und in manchen Fällen kann die Reaktionsgeschwindigkeit durch Zugabe eines Jodids, wie Lithiumjodid, zum Reaktionsgemisch gesteigert werden.

Die Umsetzung einer Verbindung der Formel Ib mit Epoxyalkanen liefert die hydroxyalkylsubstituierte Verbindung der Formel Id. Die Behandlung mit einem substituierten Epoxyalkan liefert die 2-substituierten 2-Hydroxyalkyl-Analoga einer Verbindung der Formel Id, z.B. ergibt die Umsetzung einer Verbindung der Formel Ib mit Styroloxid eine Verbindung der Formel Id, worin $R_4'$ 2-Phenyl-2-hydroxyäthyl ist. Die Reaktion erfolgt gewöhnlich in Gegenwart eines alkoholischen Lösungsmittels, wie Methanol, bei etwa Raumtemperatur bis zur Rückflusstemperatur des Gemisches. Die Expoxyalkane sind entweder im Handel erhältlich oder werden durch Epoxydation der entsprechenden Olefine oder durch Methylenierung eines Ketons mit einem Sulfoniummethylid oder Sulfoxoniummethylid, z.B. Dimethylsulfoniummethylid, hergestellt. So liefert z.B. die Behandlung von Benzaldehyd mit Dimethylsulfoniummethylid Styroloxid.

Die Verbindung der Formel Ib kann in die Verbindung der Formel Ie nach einem Verfahren überführt werden, bei dem nacheinander der basische Amin-Stickstoff (N-6) der Verbindung der Formel Ib mit einer hydrolysierbaren oder hydrogenolysierbaren Schutzgruppe geschützt, am Pyrrolstickstoff (N-1) substituiert und die Schutzgruppe abgespalten wird. Typische Schutzgruppen sind Acetyl, t-Butoxycarbonyl, Benzolsulfonyl oder Benzyl. Die Umsetzung der geschützten Zwischenstufe erfolgt praktisch wie für die Überführung der Verbindung der Formel Ia in die Verbindung der Formel Ic′ beschrieben. Die substituierte Zwischenstufe wird durch Basen- oder Säurehydrolyse oder Hydrogenolyse, je nach Eignung für die Schutzgruppe, von der Schutzgruppe befreit. Beispielsweise wird die Verbindung der Formel Ib, worin $R_2$ Methyl und $R_3$ Äthyl ist, mit Ameisen/Essigsäureanhydrid behandelt, um das 6-Formyl-Derivat zu ergeben. Die Behandlung des Derivats mit Natriumhydrid in Dimethylsulfoxid und anschliessende Behandlung mit Methyljodid und Säurehydrolyse liefert die Verbindung der Formel Ie, worin $R_1'$ Methyl, $R_2$ Methyl und $R_3$ Äthyl ist.

Die Behandlung einer Verbindung der Formel Ib mit einem Halogenalkylamin in Gegenwart einer Base, z.B. Kaliumcarbonat, oder eines Aziridins liefert aminsubstituierte Analoga einer Verbindung der Formel Id. Die Reaktionsbedingungen sind wie für die Herstellung der Alkylderivate beschrieben.

In manchen Fällen, wo nämlich $R_4'$ in der Verbindung der Formel Id keine funktionellen Gruppen enthält, die eine Alkylierung oder Acylierung erfahren können, können die im Formelschema III aufgeführten Arbeitsweisen direkt zur Herstellung N-1-substituierter Analoga der Formel Ic angewandt werden. Alkylierungen können bei Verbindungen eintreten, worin

$R_4'$ Hydroxy-($C_1$–$C_7$)-alkyl, Phenyl-hydroxy-($C_1$–$C_7$)-alkyl, Halogenphenyl-hydroxy-($C_1$–$C_7$)-alkyl, ($C_1$–$C_7$)-Alkylphenyl-hydroxy-($C_1$–$C_7$)-alkyl, ($C_1$–$C_7$)-Alkoxyphenyl-hydroxy-($C_1$–$C_7$)-alkyl, ($C_1$–$C_7$)-Alkoxy-hydroxy-($C_1$–$C_7$)-alkyl, ($C_3$–$C_6$)-Cycloalkyl-hydroxy-($C_1$–$C_7$)-alkyl, 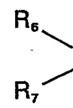N-($C_1$–$C_7$)-Alkyl oder gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Phenoxy-hydroxy-($C_1$–$C_7$)-alkyl bedeutet.

Die funktionellen Gruppen darin, z.B. Hydroxyl oder sec.-Amino, müssen mit einer basenstabilen Schutzgruppe, wie Tetrahydropyranyl, geschützt werden. Nach der N-1-Alkylierung wird die Schutzgruppe durch saure Hydrolyse abgespalten.

Die Verbindung der Formel Ie wird in die Verbindung der Formel Ic unter Anwendung praktisch der gleichen Arbeitsweise, wie sie zur Überführung der Verbindung der Formel Ib in die Verbindung der Formel Id angewandt wurde, überführt.

Bei den in den Formelschemata I, II und III beschriebenen Reaktionen können sowohl die trans-Isomeren der Formel

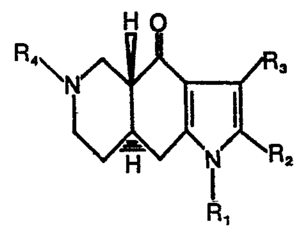

I′

worin $R_1$, $R_2$, $R_3$ und $R_4$, wie zuvor beschrieben sind, als auch cis-Isomere der Formel

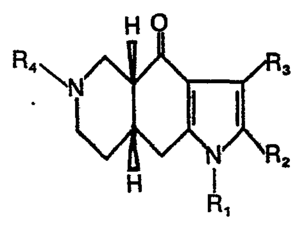

I″

worin $R_1$, $R_2$, $R_3$ und $R_4$ wie zuvor beschrieben sind, der Verbindungen der Formel I entstehen, wobei das trans-Isomere überwiegt. Das reine trans-Isomere kann durch Chromatographie oder Kristallisation abgetrennt werden. Ausserdem kann das Gemisch isomerisiert werden, wie für die Isomerisierung der trans- und cis-Isomeren der Oxoverbindung der Formel I′a′ und I″a′ beschrieben.

Formelschema IV

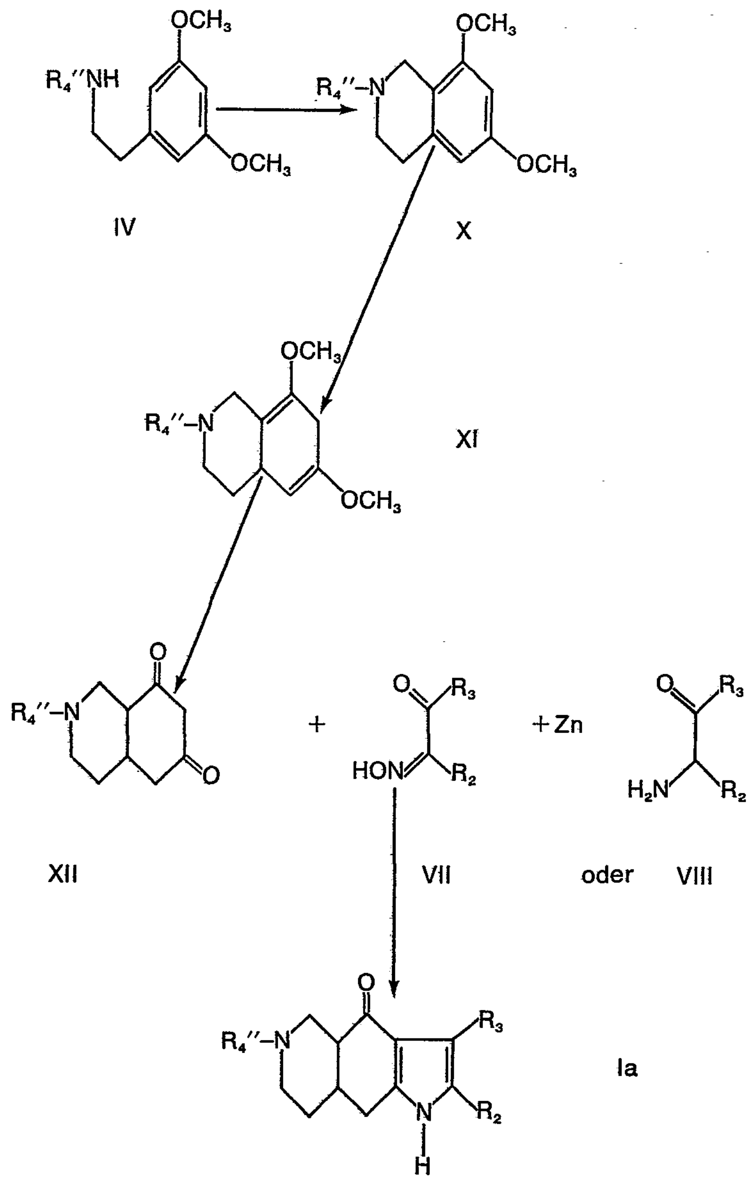


IV X XI XII VII VIII Ia

worin $R_2$, $R_3$ und $R_4''$ wie zuvor beschrieben sind.

Eine andere Synthese für die Verbindungen der Formel Ia ist im Formelschema IV beschrieben, wobei der Isochinolinring vor der Bildung des Pyrrolrings gebildet wird. Nach dem Formelschema IV wird das (3,5-Dimethoxyphenyl)-äthylamin der Formel IV mit wässrigem Formaldehyd rückflussgekocht, um das Tetrahydroisochinolin der Formel X zu liefern. Die Birch-Reduktion des Tetrahydroisochinolins der Formel X mit Lithium in t-Butanol enthaltendem flüssigem Ammoniak unter praktisch den gleichen Bedingungen, wie für die Birch-Reduktion der Verbindung der Formel IV beschrieben, liefert das Hexahydroisochinolin der Formel XI. Hydrolyse des rohen Hexahydroisochinolins der Formel XI unter praktisch gleichen Bedingungen, wie für die Hydrolyse des Dihydroamins der Formel V beschrieben, liefert das Diketon der Formel XII. Die Verbindung der Formel XII wird in einer Knorr-Kondensation, wie bei der Herstellung des Dihydroindolon-äthylamins der Formel IX beschrieben, mit dem Isonitrosoketon der Formel VII oder mit der Aminocarbonylverbindung der Formel VIII zu dem Pyrroloisochinolin der Formel Ia umgesetzt. Bevorzugt ist die Reaktionsfolge nach dem Formelschema IV, ausgehend von dem Amin der Formel IV, worin $R_4''$ Methyl ist, was zum entsprechenden N-Methylpyrroloisochinolin der Formel Ia' führt, und zwar als Gemisch des trans-Isomeren I'a' und des cis-Isomeren der Formel I''a'.

Um hauptsächlich das trans-Isomere der Formel I'a' zu erhalten, können die gleichen Arbeitsweisen für die Isomerisierung des Gemisches der Pyrroloisochinoline der Formeln I'a' und I''a', wie zuvor beschrieben, angewandt werden.

Die Verbindungen der Formel A, worin X S ist, sind durch die Formel

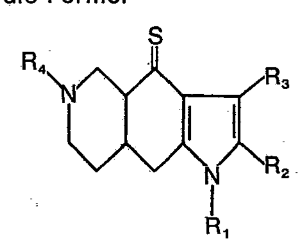

II

worin $R_1$, $R_2$, $R_3$ und $R_4$, wie zuvor beschrieben sind, charakterisiert und können, wie nachfolgend ausgeführt, hergestellt werden.

Thione der Formel II werden im allgemeinen durch Umsetzen von Phosphorpentasulfid in Ketoverbindungen der Formel I hergestellt. Wenn die Verbindung der Formel I keine funktionellen Gruppen hat, die mit Phosphorpentasulfid reagieren können, ausser der 4-Oxo-Gruppe, kann die Verbindung der Formel I in die Verbindung der Formel II direkt durch Erwärmen mit Phosphorpentasulfid in einem inerten organischen Lösungsmittel überführt werden. Wenn anderseits die Verbindung der Formel I funktionelle Gruppen enthält, die mit Phosphorpentasulfid zu reagieren vermögen, z.B., wenn $R_4$ eine andere Bedeutung als Wasserstoff, $(C_1–C_7)$-Alkyl, $(C_1–C_7)$-Alkoxy-$(C_1–C_7)$-alkyl, $(C_2–C_7)$-Alkenyl, $(C_3–C_6)$-Cycloalkyl-$(C_1–C_7)$-alkyl, $(C_2–C_7)$-Alkinyl, Thienyl-$(C_1–C_7)$-alkyl, Furyl-$(C_1–C_7)$-alkyl, $(C_2–C_7)$-Alkenyloxy-$(C_1–C_7)$-alkyl oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Phenyl-$(C_1–C_7)$-alkyl, Phenyl-$(C_2–C_7)$-alkenyl oder Phenoxy-$(C_1–C_7)$-alkyl oder Trifluoralkyl mit 2 bis 6 Kohlenstoffatomen hat, oder wenn $R_2$ oder $R_3$ $(C_1–C_7)$-Alkanoyl oder gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino subsituiertes Benzoyl bedeutet, müssen diese Gruppen vor der Umsetzung geschützt und danach von der Schutzgruppe wieder befreit werden. Ketogruppen z.B. können als Ketal, wie als Äthylenketal, und Alkoholgruppen als Ätherderivat, wie als Benzyläther, geschützt werden. Anderseits können Thione der Formel II nach geeigneten, in den Formelschemata V und VI beschriebenen Arbeitsweisen hergestellt werden.

Formelschema V

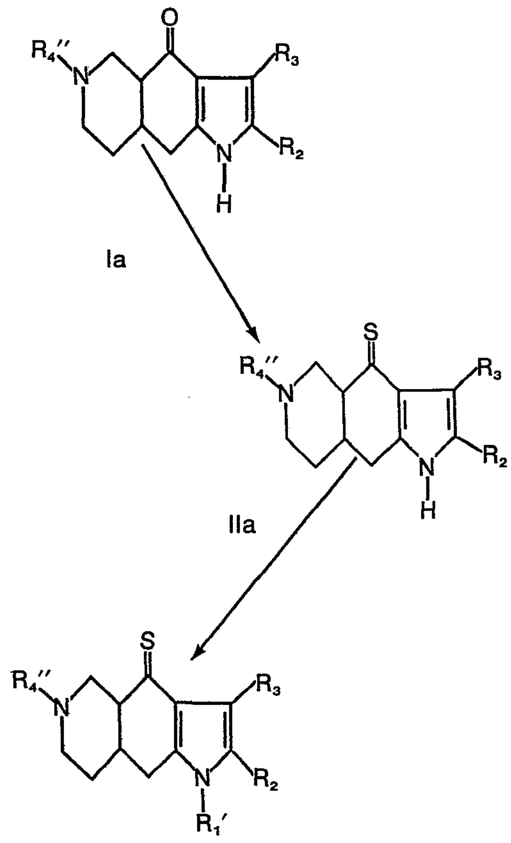

Ia

IIa

IIc′

worin $R_1'$, $R_2$, $R_3$ und $R_4''$ wie zuvor beschrieben sind.

Im Formelschema V wird die Verbindung der Formel Ia in die Verbindung der Formel IIa durch Erwärmen in einem inerten organischen Lösungsmittel mit Phosphorpentasulfid überführt. Bevorzugte Lösungsmittel sind Tetrahydrofuran, Benzol und Dioxan, und die Umsetzung erfolgt im allgemeinen bei Rückflusstemperatur. Die Verbindung der Formel IIa kann dann zur Verbindung der Formel IIc′ in der gleichen Weise wie im Formelschema II für die Umwandlung der Verbindung der Formel Ia′ in die Verbindung der Formel Ic′ alkyliert oder acyliert werden.

Weitere Verbindungen der Formel II können hergestellt werden, wie im Formelschema VI beschrieben:

Formelschema VI

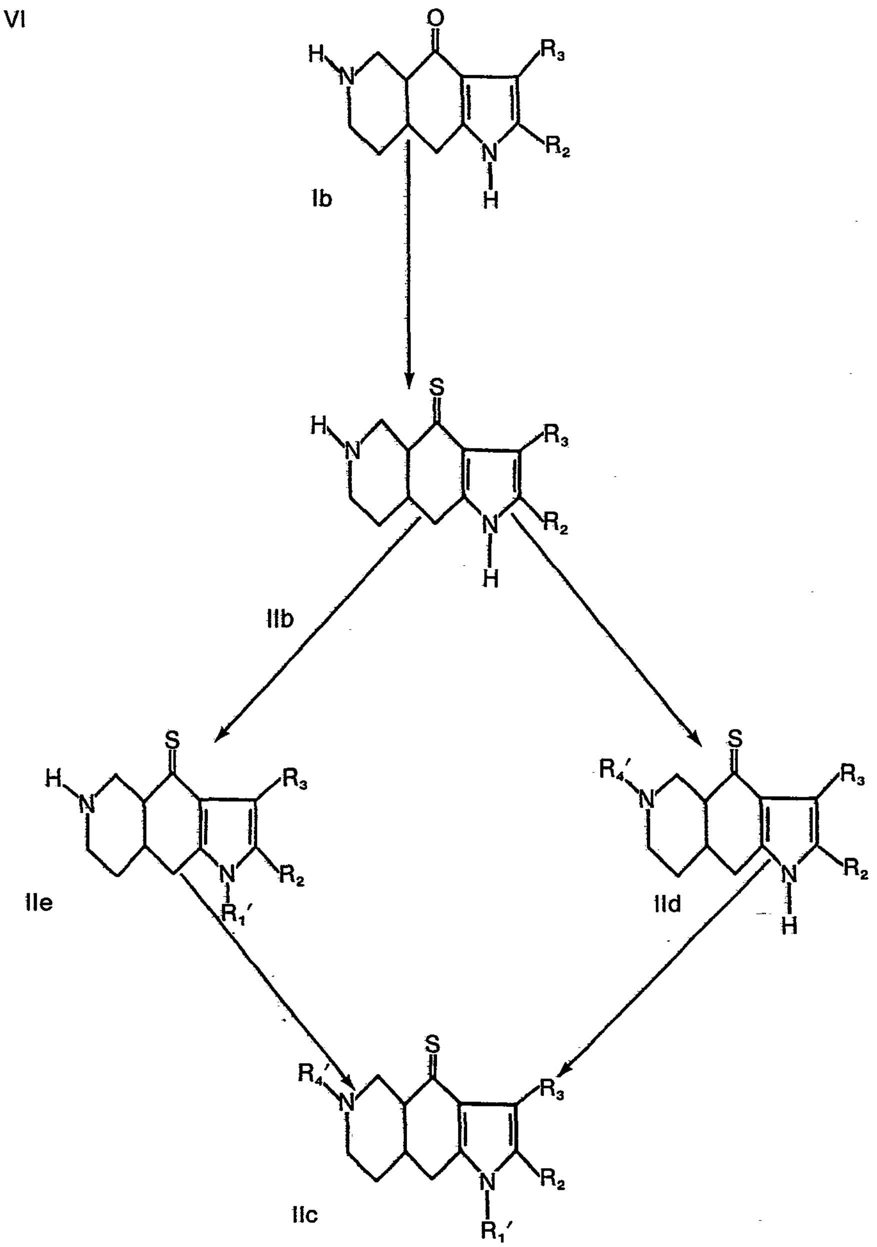

Ib

IIb

IIe

IId

IIc

worin $R_2$, $R_3$, $R_1'$ und $R_4'$ wie zuvor beschrieben sind.

Im Formelschema VI wird die Verbindung der Formel Ib mit Phosphorpentasulfid, wie oben beschrieben, zu dem Thion der Formel IIb umgesetzt. Anschliessende Umwandlungen der Verbindung der Formel IIb in die Verbindungen der Formeln IIc, IId und IIe erfolgen ähnlich wie im Formelschema III für die analoge Oxoverbindung Ib bei der Überführung in die Verbindungen der Formeln Ic, Id und Ie beschrieben.

Bei diesen Reaktionen können sowohl die trans-Isomeren der Formel

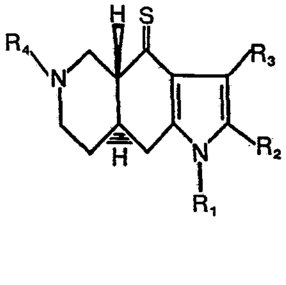

II',

worin $R_1$, $R_2$, $R_3$ und $R_4$ wie zuvor beschrieben sind, als auch cis-Isomere der Formel

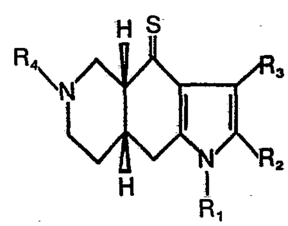

II'',

worin $R_1$, $R_2$, $R_3$ und $R_4$ wie zuvor beschrieben sind, der Verbindungen der Formel II entstehen, wobei das trans-Isomere überwiegt. Das reine trans-Isomere kann durch Chromatographie oder Kristallisation abgetrennt werden. Ausserdem kann das Gemisch isomerisiert werden, wie für die Isomerisierung der trans- und cis-Isomeren der Oxoverbindung der Formel I'a' und I''a' beschrieben.

Die Verbindungen der Formel A bilden Säureadditionssalze mit anorganischen oder organischen Säuren. So bilden sie pharmazeutisch annehmbare Säureadditionssalze sowohl mit pharmazeutisch annehmbaren organischen als auch anorganischen Säuren, z.B. mit Halogenwasserstoffsäure, wie Chlorwasserstoffsäure, Bromwasserstoffsäure, Jodwasserstoffsäure, andere Mineralsäuresalze, wie mit Schwefelsäure, Salpetersäure, Phosphorsäure od.dgl., mit Alkyl- und Monoarylsulfonsäuren, wie Äthansulfonsäure, Toluolsulfonsäure, Benzolsulfonsäure od.dgl., anderen organischen Säuren, wie Essigsäure, Weinsäure, Maleinsäure, Zitronensäure, Benzoesäure, Salicylsäure, Ascorbinsäure u.dgl. Pharmazeutisch nicht annehmbare Säureadditionssalze von Verbindungen der Formel A können in pharmazeutisch annehmbare Säureadditionssalze über herkömmliche Austauschreaktionen überführt werden, wodurch das pharmazeutisch nicht annehmbare Anion durch ein pharmazeutisch annehmbares Anion ersetzt wird, oder anderseits durch Neutralisieren des pharmazeutisch nicht annehmbaren Säureadditionssalzes und anschliessendes Umsetzen der so erhaltenen freien Base mit einem Reagens, das ein pharmazeutisch annehmbares Säureadditionssalz liefert. Die Säureadditionssalze können auch Hydrate bilden.

Die Verbindungen der Formel A und ihre pharmazeutisch annehmbaren Säureadditionssalze zeigen neuroleptische Aktivität. Ihnen fehlt jedoch auffallenderweise hypotensive Aktivität, und sie zeigen nur schwache kataleptische Aktivität. Folglich sind die Verbindungen der Formel A als antipsychotische Mittel brauchbar, z.B. bei der Behandlung von Schizophrenie. Die Aktivität der Verbindungen der Formel A, die sie als antipsychotische Mittel brauchbar macht, kann nach bekannten Arbeitsweisen an Warmblütern demonstriert werden.

Nach einer Arbeitsweise werden z.B. trainierte Ratten in Versuchskammern gebracht, die mit einem Reaktionshebel, einem Stahldrahtboden zum Austeilen eines elektrischen Schocks und einem Lautsprecher für akustische Stimulation ausgestattet sind. Jeder Versuch besteht in einem Warnton von 15 s (bedingter Stimulus), der für weitere 15 s seine Fortsetzung findet und dabei von einem elektrischen Schock begleitet wird (unbedingter Stimulus; 1,0 mA, 350 V Wechselspannung). Die Ratten können einen Versuch an jeder Stelle durch Niederdrücken des Reaktionshebels beenden. Eine Reaktion während des ersten 15 s-Warntons beendet den Versuch, bevor ein Schock ausgeteilt wird, und wird als Vermeidungsreaktion angesehen, während eine Reaktion, die während der Schockausteilung erfolgt, eine Fluchtreaktion ist. Versuche werden alle 2 min während einer einstündigen Testdauer vorgenommen (30 Versuche pro Testdauer).

Trainierte Ratten zeigen eine verlässliche Grundlinie des Vermeidungsverhaltens (0 bis 3 ausbleibende Vermeidungsreaktionen pro Testdauer). Mindestens 3 bis 4 Ratten erhalten zu geeigneten Vorbehandlungszeiten Verbindungen in jeder Dosierungsmenge über einen Dosierungsbereich verabreicht. Während Testzeiten zur Kontrolle erhalten Ratten Trägermittel alleine. Jede Woche wechselt eine Kontroll- und eine Versuchsdauer; jedes Tier dient als eigene Kontrolle.

Jede Versuchsdauer ist in drei aufeinanderfolgende 20 min-Abschnitte (10 Versuche) unterteilt. Die Anzahl der Reaktionen wird über alle Individuen bei gegebener Dosis innerhalb jedes Abschnitts summiert.

Die Anzahl der Versuche, bei denen die Ratten keine Vermeidungsreaktion (Vermeidungs-Blokkierung, VB) oder keine Fluchtreaktion (Fluchtblockierung, FB) zeigten, wird für den Abschnitt bestimmt, der bei jeder Dosis den maximalen Effekt entfaltete. Diese Zahl ist ausgedrückt als Prozentsatz der innerhalb des Abschnitts insgesamt vorgenommenen Versuche. Die für 50% Vermeidungsblockierung (VB 50) berechnete Dosis wird aus der Dosis/Wirkung-Regressionslinie erhalten, aufgestellt nach der Methode der kleinsten Quadrate. Die geringste Dosis, die 20% Blockierung der Fluchtreaktion (FB 20) hervorrief, wird aus einem Dosis/Wirkungs-Diagramm abgelesen. Um diese Werte zu erhalten, wird die Wirkung in % gegen den log der Dosis aufgetragen.

Antipsychotische Mittel können von anderen Wirkstoffarten, die das Verhalten von Ratten bei dieser Arbeitsweise beeinträchtigen, durch die grössere Trennung zwischen Dosen, die Vermeidungsreaktion blockieren, und Dosen, die Fluchtreaktion blockieren, unterschieden werden. Die klinische Leistungsstärke antipsychotischer Wirkstoffe mit bekannten therapeutischen Anwendungen und Eigenschaften hängt wesentlich und stark mit ihrer Leistungsstärke bei dieser Prozedur zusammen. Folglich können die Verbindungen der Formel A therapeutisch in Dosierungen eingesetzt werden, die mit ihrer Leistungsstärke bei Testverfahren in Übereinstimmung stehen.

Wenn

3-Äthyl-2,6-dimethyl-4,4a,5,6,7,8,8a,9-octahydro-4a,8a-trans-1H-pyrrolo-[2,3-g]isochinolin-4-on-Hydrochlorid,

das eine $LD_{50}$ von z.B. 350 mg/kg p.o. bei Mäusen zeigte, als Testsubstanz verwendet wird, wird neuroleptische Aktivität bei einer $VB_{50}$ von 0,7 mg/kg p.o. und 0,095 mg/kg s.c., beim (–)-Enantiomeren der vorstehenden Verbindung wird neuroleptische Aktivität bei einer $VB_{50}$ von 0,48 mg/kg p.o. beobachtet.

Ähnlich wird, wenn

2,3,6-Trimethyl-4,4a,5,6,7,8,8a,9-octahydro-4a,8a-trans-1H-pyrrolo[2,3-g]isochinolin-4-on-Hydrochlorid

als Testsubstanz verwendet wird, neuroleptische Aktivität bei einer $VB_{50}$ von 0,48 mg/kg p.o. beobachtet.

Wenn

N-[2(3-Äthyl-4,4a,5,6,7,8,8a,9-octahydro-2-methyl-4-oxo-4a,8a-trans-1H-pyrrolo[2,3-g]isochinolin-6-yl)-äthyl]-4-fluorbenzamid

als Testsubstanz verwendet wird, wird eine neuroleptische Aktivität bei einer $VB_{50}$ von 3,5 mg/kg p.o. beobachtet.

Wenn
3-Äthyl-2-methyl-6-[4-(4-fluorphenyl)-4-oxobutyl]-4,4a,5,6,7,8,8a,9-octahydro-4a,8a-trans-1H-pyrrolo[2,3-g]isochinolin-4-on
als Testsubstanz verwendet wird, wird neuroleptische Aktivität bei einer $VB_{50}$ von 0,19 mg/kg p.o. beobachtet.

Ebenso wird, wenn
3-Äthyl-2,6-dimethyl-4,4a,5,6,7,8,8a,9-octahydro-4a,8a-trans-1H-pyrrolo[2,3-g]isochinolin-4-thion
als Substanz verwendet wird, neuroleptische Aktivität bei einer $VB_{50}$ von 0,94 mg/kg p.o. beobachtet.

Die Verbindungen der Formel A und ihre pharmazeutisch annehmbaren Säureadditionssalze haben antipsychotische Wirkungen, die denen von Haloperidol, Trifluorperazin und Molindon, die durch ihre therapeutische Verwendung und Eigenschaften bekannt sind, qualitativ ähneln. So zeigen die Verbindungen der Formel A ein mit antipsychotischen Wirkstoffen bekannter Wirksamkeit und Sicherheit verbundenes Aktivitätsmuster.

Die Verbindungen der Formel A und ihre pharmazeutisch annehmbaren Säureadditionssalze können in Form herkömmlicher pharmazeutischer Zubereitungen verwendet werden. Beispielsweise enthalten sie geeignete orale Dosierungseinheiten oder liegen im Bereich von 0,05 bis 50 mg, und geeignete orale Dosierungen bei warmblütigen Tieren liegen im Bereich von etwa 0,001 mg/kg pro Tag und etwa 10 mg/kg pro Tag. Für jedes bestimmte warmblütige Tier jedoch kann die spezifische Dosierung schwanken, und sie sollte nach den individuellen Bedürfnissen und dem fachkundigen Urteil der die Verabreichung einer Verbindung der Formel A oder eines pharmazeutisch annehmbaren Säureadditionssalzes hiervon vornehmenden oder überwachenden Person eingestellt werden. Ferner schwankt die Häufigkeit, mit der jede solche Dosierungsform verabreicht wird, in Abhängigkeit von der Menge an vorhandenem aktivem Medikament und den Erfordernissen der pharmakologischen Situation.

Für die offenbarte Verwendung werden die Verbindungen der Formel A und ihre pharmazeutisch annehmbaren Säureadditionssalze unter Einsatz herkömmlicher inerter pharmazeutischer Hilfsmaterialien zu Dosierungsformen zusammengestellt, die sich für orale oder parenterale Verabreichung eignen. Solche Dosierungsformen umfassen Tabletten, Suspensionen, Lösungen u.dgl. Ferner können die Verbindungen der Formel A in Form geeigneter harter oder weicher Kapseln eingearbeitet und verabreicht werden. Die Art der inerten Hilfsmaterialien, die zum Zusammenstellen der Verbindungen der Formel A und ihrer phamazeutisch annehmbaren Säureadditionssalze zu oralen und parenteralen Dosierungsformen verwendet werden, liegen für den Fachmann sofort auf der Hand. Diese Hilfsmaterialien, entweder anorganische oder organische, sind z.B. Wasser, Gelatine, Lactose, Stärke, Magnesiumstearat, Talkum, pflanzliche Öle, Harze, Polyalkylenglykole usw. Ferner können, wenn gewünscht, Konservierungsstoffe, Stabilisatoren, Benetzungsmittel, Emulgatoren, Salze zur Änderung des osmotischen Druckes, Puffer u.dgl. in solche Rezepturen eingearbeitet werden.

Da die Verbindungen der Formel A und ihre pharmazeutisch annehmbaren Säureadditionssalze ein symmetrisches Kohlenstoffatom besitzen, fallen sie gewöhnlich als Racemate an. Die Aufspaltung solcher Racemate in die optisch aktiven Isomeren kann nach bekannten Arbeitsweisen erfolgen. Manche Racemate können als Eutektika ausgefüllt und danach getrennt werden. Die chemische Aufspaltung wird jedoch bevorzugt. Danach bilden sich aus dem racemischen Gemisch Diastereomere mit einem optisch aktiven Spaltungsmittel, z.B. optisch aktiver Säure, wie (+)-Weinsäure, um ein diastereomeres Salz zu bilden. Die gebildeten Diastereomeren werden durch fraktioniertes Kristallisieren getrennt und können in die entsprechende optisch isomere Base überführt werden. So umfasst die Erfindung die optisch aktiven Isomeren der Verbindungen der Formel A sowie deren Racemate.

Ferner wird aufgrund der möglichen unterschiedlichen räumlichen Anordnungen ihrer Atome verständlich, dass die erfindungsgemässen Verbindungen in mehr als einer möglichen geometrisch isomeren Form erhalten werden können. Die Verbindungen der Formel A, wie beschrieben und beansprucht, sollen alle solchen isomeren Formen umfassen. So sind die hier gegebenen Beispiele als besondere Gemische geometrischer Isomerer oder einzelne geometrische Isomere veranschaulichend und nicht als Begrenzung des Umfangs der Erfindung zu verstehen.

Die folgenden Beispiele veranschaulichen die Erfindung noch eingehender. Alle Temperaturen sind in °C angegeben, sofern nicht anders bezeichnet.

Beispiel 1

Herstellung von N-2-(3,5-Dimethoxyphenyl)-äthylcarbaminsäureäthylester

In einen mit einem mechanischen Rührer und einem Zulauftrichter ausgestatteten 5-l-Dreihalsrundkolben wurden 32,63 g (3,5-Dimethoxyphenyl)äthylamin-Hydrochlorid, 600 ml Wasser, 600 ml Methylenchlorid und 150 ml 1n Natriumhydroxidlösung gebracht. Das Gemisch wurde gerührt und in einem Eisbad gekühlt, während 16,28 g Chlorameisensäureäthylester in 60 ml Methylenchlorid über 30 min zugetropft wurden. Während der Zugabe wurden insgesamt 150 ml 1n Natronlauge in 8 Teilmengen zugesetzt, um den pH-Wert zwischen 8 und 8 zu halten. Nach beendeter Zugabe wurde das Gemisch im Eisbad 1 h gerührt. Das Gemisch wurde in einen Scheidetrichter überführt, und die organische Schicht wurde abgetrennt. Die wässrige Lösung wurde mit 200 ml Methylenchlorid extrahiert, und die organischen Lösungen wurden vereinigt und mit 100 ml Wasser und 100 ml Salzlösung gewaschen und über wasserfreiem Natriumsulfat getrocknet und filtriert. Das Filtrat wurde am Rotationsverdampfer zu 37,1 g rohem N-2(3,5-Dimethoxyphenyl)äthylcarbaminsäureäthylester in Form eines farblosen Öls eingeengt.

Beispiel 1a

Herstellung von N-Methyl-(3,5-dimethoxyphenyl)-äthylamin-Hydrochlorid

In einen mit einem mechanischen Rührer, einem Zugabetrichter und einem Kühler ausgestatteten 3-l-Dreihalsrundkolben wurden 180 ml 70%ige Natriumdihydrobis(2-methoxyäthoxy)aluminatlösung und 700 ml trockenes Tetrahydrofuran gebracht. Die Lösung wurde in einem Eisbad gekühlt, und eine Lösung von 37,1 g rohen N-2-(3,5-Dimethoxyphenyl)äthylcarbaminsäureäthylesters in 100 ml trockenem Tetrahydrofuran wurden über 15 min zugesetzt. Nach der Zugabe wurde das Gemisch 1 h auf Rückfluss erwärmt und dann in einem Eisbad gekühlt, überschüssiges Hydrid durch Zutropfen von 100 ml einer 5%igen Natriumhydroxidlösung zersetzt. Nachdem die gesamte Base zugesetzt worden war, wurde die organische Schicht abgetrennt und die wässrige mit 100 ml Äther extrahiert. Die vereinigten organischen Lösungen wurden am Rotationsverdampfer zu einem Öl eingeengt, und das Öl wurde in 300 ml Äther gelöst. Die Ätherlösung wurde mit 50 ml Wasser, 50 ml Salzlösung gewaschen, über wasserfreiem Natriumsulfat getrocknet und filtriert. Das Filtrat wurde mit 70 ml ätherischem Chlorwasserstoff zur Fällung des Amin-Hydrochlorids versetzt. Der Feststoff wurde auf einem Büchner-Trichter gesammelt und aus 180 ml absolutem Äthanol und 270 ml Äther kristallisiert und lieferte 28,9 g N-Methyl-(3,5-dimethoxyphenyl)-äthylamin-Hydrochlorid als weissen, kristallinen Feststoff, Schmp. 160–164°.

Beispiel 2

Herstellung von N-Methyl-1,5-dimethoxycyclohexa-1,4-dien-3-äthylamin

185,2 g N-Methyl-(3,5-dimethoxyphenyl)äthylamin-Hydrochlorid wurden in 1600 ml Wasser gelöst und die Lösung mit 160 ml Ammoniumhydroxid alkalisch gemacht. Das Gemisch wurde mit 3 × 1000 ml Methylenchlorid extrahiert, und die vereinigten Extrakte wurden mit 1000 ml Salzlösung gewaschen und über wasserfreiem Natriumsulfat getrocknet. Verdampfen des Lösungsmittels am Rotationsverdampfer bei 35–40° lieferte 156,0 g freie Base.

In einen 12-l-Dreihalskolben, ausgestattet mit einem mechanischen Rührer und zwei Trockeneiskühlern, einer mit einem Gaseinleitungsrohr, der andere mit einem Natronkalk-Trockenrohr versehen, wurden 4,0 l wasserfreies Ammoniak kondensiert. Zu dem Ammoniak wurde eine Lösung von 156,0 g der freien Base in 400 ml t-Butanol und 400 ml wasserfreier Äther über 15 min zugegeben. Die gerührte Lösung wurde über 50 min mit insgesamt 33,6 g Lithiumdraht, in Längen von 6,35 cm (2,5 Zoll) geschnitten, versetzt. Die Zugabegeschwindigkeit wurde so gesteuert, dass pro min 12,7 cm Draht zugesetzt wurden. Nachdem das gesamte Lithium zugesetzt worden war, wurde das tiefblaue Gemisch 2 h unter Rückfluss gerührt. Dann wurden 2,8 l wasserfreier Äther zum Verdünnen des Gemisches zugesetzt, das Trockenrohr wurde entfernt, um den Wasserstoff abzulassen, und insgesamt 280 g Ammoniumchloridpulver wurden langsam über 30 min zugesetzt, bis die blaue Farbe verschwunden war. Der Trockeneiskühler wurde entfernt, und das Gemisch wurde gerührt, das Ammoniak konnte über Nacht verdampfen.

Der Rückstand wurde mit 2,8 l Eiswasser versetzt. Das Gemisch wurde in einen Scheidetrichter überführt, wobei mit 800 ml Äther gespült wurde, und die Schichten wurden getrennt. Die wässrige Schicht wurde mit 2 × 1,5 l Methylenchlorid extrahiert, und die Extrakte wurden vereinigt und mit 1 l Salzlösung gewaschen und über wasserfreiem Natriumsulfat getrocknet. Verdampfen der Lösungsmittel am Rotationsverdampfer bei 40° und schliesslich bei 40°/1,0 mm für 1,5 h lieferte 150,7 g Rohprodukt als gelbes Öl. Das rohe Öl wurde über eine 30,5 cm (12 Zoll)-Goodloe-Kolonne (Bad 150°) destilliert, wobei folgende Fraktionen aufgefangen wurden:

| Fraktion | Sdp. | Gewicht | GC-Reinheit |
|---|---|---|---|
| 1 | 40–80°/0,45 mm | 7,9 g | 4,6% |
| 2 | 80–85°/0,45–0,15 mm | 6,2 g | 50 % |
| 3 | 85–86°/0,15 mm | 21,2 g | 92 % |
| 4 | 86–87°/0,15 mm | 99,4 g | 100 % |

Die kombinierten Fraktionen 3 und 4 lieferten 120,6 g N-Methyl-1,5-dimethoxycyclohexa-1,4-edin-3-äthylamin als farbloses Öl.

Beispiel 3

Herstellung von 6-[2-(N-Methylamino)äthyl]-2-methyl-3-äthyl-6,7-dihydro-(5H)-4(1H,5H)-indolon

In einen 1-l-Dreihalsrundkolben, versehen mit einem mechanischen Rührer und einem Kühler, wurde eine Lösung von 60,0 g destilliertem N-Methyl-1,5-dimethoxycyclohexa-1,4-dien-3-äthylamin in 700 ml 70%iger wässriger Essigsäure gebracht. Das Reaktionsgemisch wurde 15 min rückflussgekocht, und 59,5 g Zinkstaub wurden in fünf Anteilen über 10 min zugegeben und dann das Gemisch weitere 15 min rückflussgekocht. Der unter Rückfluss kochenden Lösung wurde eine Lösung von 42,1 g 2-Isonitroso-3-pentanon in 175 ml 70%iger wässriger Essigsäure über einen Zeitraum von 1 h zugesetzt. Nach der Zugabe wurde das Gemisch

2,5 h rückflussgekocht und auf Raumtemperatur gekühlt. Das ausgefallene Zinkacetat wurde abfiltriert und der Filterkuchen mit 500 ml Methylenchlorid gewaschen. Das Filtrat wurde am Rotationsverdampfer eingeengt und der Rückstand bei 100°/1,0 mm 30 min erwärmt, um letzte Spuren Essigsäure zu entfernen. Der Rückstand wurde in 500 ml Wasser gelöst, und die Lösung wurde mit 2 × 150 ml Methylenchlorid extrahiert. Die Methylenchloridextrakte wurden verworfen, und die wässrige Schicht wurde mit 165 ml Ammoniumhydroxid basisch gemacht (pH 8 bis 9), und 500 ml Salzlösung wurden zugesetzt. Das Gemisch wurde mit 3 × 200 ml Methylenchlorid extrahiert, und die vereinigten Extrakte wurden mit 100 ml Salzlösung gewaschen und über wasserfreiem Natriumsulfat getrocknet. Verdampfen des Lösungsmittels lieferte 56,0 g rohes Tetrahydroindolon, das in 90 ml Toluol/Äthylacetat 2 : 1 gelöst wurde. Die Lösung wurde magnetgerührt und angeimpft und über Nacht unter Rühren kristallisieren gelassen. Die erste Ausbeutenmenge von 20,8 g wurde durch Filtrieren gesammelt, und die Mutterlauge wurde eingeengt und wieder aus einer gerührten Lösung kristallisiert, um eine zweite Ausbeutenmenge von 10,0 g zu ergeben. Die Mutterlauge wurde in 75 ml Methanol gelöst, und eine Lösung von 15,0 Oxalsäure in 50 ml Methanol wurde zugesetzt. Das Gemisch wurde 10 min auf dem Dampfbad erwärmt und dann gekühlt. Das feste Oxalat wurde abfiltriert und mit 10 ml Methanol gewaschen und in 50 ml Wasser gelöst. Die Lösung wurde mit Ammoniumhydroxid basisch gemacht und mit 2 × 50 ml Methylenchlorid extrahiert. Die Extrakte wurden mit 1 × 20 ml Salzlösung gewaschen und über wasserfreiem Natriumsulfat getrocknet und am Rotationsverdampfer zu 4,5 g zusätzlichem Rohprodukt eingeengt. Kristallisieren aus Toluol/Äthylacetat 2 : 1 lieferte 2,6 g zusätzliches kristallines Produkt. Die beiden Ausbeutenmengen und die aus dem Oxalat stammenden Kristalle wurden vereinigt und bei 25°/1 mm 2 h getrocknet, um 33,4 g 6-[2-(N-Methylamino)äthyl]-2-methyl-3-äthyl-6,7-dihydro-(5H)-4(1H, 5H)-indolon als schwach gelben Feststoff, Schmp. 114–120°, nach Dünnschichtchromatographie homogen, zu liefern.

Beispiel 4

Herstellung von
3-Äthyl-2,6-dimethyl-4,4a,5,6,7,8,8a,9-octahydro-4a,8a-trans-1H-pyrrolo[2,3-g]isochinolin-4-on-Hydrochlorid

In einen 500-ml-Rundkolben wurden 17,0 g 6-[2-(N-Methylamino)äthyl]-2-methyl-3-äthyl-6,7-dihydro-(5H)-4(1H, 5H)-indolon und 170 ml Methanol gebracht. Die Lösung wurde mit 20 ml 4n Chlorwasserstoff in Diäthyläther (hergestellt durch Einleiten von HCl-Gas in Diäthyläther in einem Eisbad und Titrieren) versetzt. Das Lösungsmittel wurde am Rotationsverdampfer entfernt und der verbleibende Rückstand bei 50°/1 mm 2 h getrocknet, um 19,7 g rohes Hydrochlorid zu liefern.

In einen mit einem mechanischen Rührer, Thermometer und Destillationskopf ausgestatteten 3-l-Dreihalskolben wurden die 19,7 g Hydrochlorid, 21,8 g Paraformaldehyd und 1000 ml Octanol gebracht. Das Reaktionsgemisch wurde auf Rückfluss erwärmt, und freigesetztes Wasser wurde destillativ entfernt, bis die Temperatur der Octanollösung in dem Kolben 175–180° erreichte, worauf der Destillationskopf entfernt und durch einen Rückflusskühler ersetzt wurde. Das Reaktionsgemisch wurde 1 h auf 175–180° erwärmt, und 6,54 g Paraformaldehyd wurden in drei Anteilen über 5 min zugesetzt. Wasser wurde wie zuvor herausdestilliert, bis die Temperatur 175–180° erreichte, und das Gemisch wurde noch 1 Stunde auf 175–180° erwärmt. Die dunkelbraune Lösung wurde gekühlt und in 1000 ml Wasser gegossen. Die Schichten wurden getrennt, und die organische Schicht wurde mit 2 × 400 ml 5%iger Salzsäure extrahiert. Die vereinigten wässrigen Extrakte wurden mit 2 × 150 ml Chloroform gewaschen und die Chloroformlösungen verworfen. Die wässrige Schicht wurde mit 120 ml Ammoniumhydroxid und 400 ml Chloroform versetzt. Die Schichten wurden getrennt und die wässrige Lösung mit 4 × 200 ml Chloroform extrahiert. Die vereinigten Chloroformextrakte wurden mit 200 ml Salzlösung gewaschen und über wasserfreiem Natriumsulfat getrocknet. Verdampfen des Lösungsmittels lieferte 12,0 g rohes Pyrrolo[2,3-g]isochinolin als ein 4a,8a-trans-, 4a,8a-cis-Gemisch (etwa 8 : 1) als dunkelgelbbraunen Feststoff. Der rohe Feststoff wurde in 100 ml Methylenchlorid/Methanol, 9 : 1, gelöst, und 300 ml Diäthyläther wurden zugesetzt. Der feine, feste Niederschlag, überwiegend das 4a,8a-trans-Isomere, wurde durch Filtrieren gesammelt, und das Filtrat wurde eingeengt und kristallisiert, um zweite und dritte Ausbeutenmengen an gelbbraunem Feststoff zu ergeben. Das vereinigte Material wurde bei 25°/1 mm 1 h getrocknet, um 8,20 g eines hellgrauen Feststoffs,
3-Äthyl-2,6-dimethyl-4,4a,5,6,7,8,8a,9-octahydro-4a,8a-trans-1H-pyrrolo[2,3-g]isochinolin-4-on,
Schmp. 203–226°, zu ergeben. Der teilweise gereinigte graue Feststoff wurde in 80 ml Methanol suspendiert, und 12 ml 4n Chlorwasserstoff in Diäthyläther wurden zugesetzt. Das Lösungsmittel wurde entfernt, und der Rückstand wurde aus 25 ml heissem, absolutem Äthanol kristallisiert. Die erste Ausbeutemenge wurde durch Filtrieren gesammelt, und die Mutterlauge wurde eingeengt und kristallisiert, um zweite und dritte Kristallmengen zu liefern. Der vereinigte Feststoff wurde in 120 ml Methanol gelöst, und 2,4 g Aktivkohle (Darco-G-60) wurden zugesetzt. Das Gemisch wurde auf einem Dampfbad 10 min erwärmt und der Kohlenstoff über Celite abfiltriert. Das Filtrat wurde konzentriert und aus 15 ml Äthanol umkristallisiert, um 3 Ausbeutenmengen an weissen Kristallen zu ergeben. Der vereinigte Feststoff wurde unter Vakuum bei 80°/0,05 mm 18 h getrocknet, um 5,4 g
3-Äthyl-2,6-dimethyl-4,4a,5,6,7,8,8a,9-octahydro-4a,8a-trans-1H-pyrrolo[2,3-g]isochinolin-4-on-Hydrochlorid
als weissen Feststoff, Schmp. 196–198°; Oxim-Semihydrat, Schmp. 131–133°, zu ergeben.

Beispiele 4a–c

Nach den Arbeitsweisen der Beispiele 3 und 4 wurden die in Tabelle I aufgeführten Verbindungen aus dem geeigneten Isonitrosoketon mit Abänderungen, wie angegeben, hergestellt. Jede Verbindung zeigte spektrale Merkmale, die mit der beschriebenen Struktur in Übereinstimmung stehen. Schmelzpunkte gelten für die freie Base oder das Hydrochlorid, wie angegeben. Isonitrosoketone wurden hergestellt, wie in der Literatur beschrieben [z.B. Ferris et al., J. Org. Chem., 24, 1726 (1959)], durch Nitrosieren des geeigneten Ketons. Die isolierten Verbindungen sind die 4a,-8a-trans-Isomeren.

Beispiel 5

Herstellung von

2-Methyl-3-äthyl-4,4a,5,6,7,8,8a,9-octahydro-4a,8a-trans-1H-pyrrolo[2,3-g]isochinolin-4-on

Ein Gemisch von

3-Äthyl-2,6-dimethyl-4,4a,5,6,7,8,8a,9-octahydro-1H-pyrrolo[2,3-g]isochinolin-4-on,

hergestellt wie in Beispiel 4 (4,92 g, 20 mMol), Chlorameisensäureäthylester (19,55 g, 180 mMol) und Kaliumbicarbonat (6,0 g, 60 mMol) in Diäthylketon (100 ml) wurde 3 h auf Rückfluss erwärmt. Das Gemisch wurde gekühlt, filtriert und das Filtrat am Rotationsverdampfer zur Trockne eingeengt und der Rückstand in Chloroform gelöst. Die Chloroformlösung wurde mit 5%iger wässriger Salzsäure, Salzlösung gewaschen und über wasserfreiem Natriumsulfat getrocknet. Verdampfen des Lösungsmittels lieferte 4,90 g rohes Carbamat, das chromatographisch an Aluminiumoxid III gereinigt wurde, um 3,70 g reines

6-Äthoxycarbonyl-3-äthyl-2-methyl-4,4a,5,6,7,8,-8a,9-octahydro-1H-pyrrolo[2,3-g]isochinolin-4-on

zu liefern.

Das Carbamat (3,7 g, 12,2 mMol), Eisessig (45 ml) und konzentrierte Salzsäure (60 ml) wurden 24 h auf Rückfluss erwärmt, gekühlt und am Rotationsverdampfer eingeengt. Der Rückstand wurde in Wasser gelöst und mit Chloroform extrahiert (verworfen), und die wässrige Schicht wurde mit Ammoniumhydroxid alkalisch gemacht, mit Chloroform extrahiert. Die vereinigten Extrakte wurden mit Salzlösung gewaschen und über wasserfreiem Natriumsulfat getrocknet und eingeengt, um 2,72 g rohes sec.-Amin zu liefern. Behandeln des rohen Amins in Äthanol mit äthanolischem Chlorwasserstoff lieferte das Hydrochlorid, das aus heissem Äthanol kristallisiert wurde, um 2,18 g (47% Ausbeute)

2-Methyl-3-äthyl-4,4a,5,6,7,8,8a,9-octahydro-4a,8a-trans-1H-pyrrolo[2,3-g]isochinolin-4-on-Hydrochlorid

als weisse Kristalle, Schmp. >250°, zu liefern.

Analyse ber. für $C_{14}H_{20}N_2O{\cdot}HCl$:

| | | | | |
|---|---|---|---|---|
| | C 62,56 | H 7,88 | N 10,42 | $Cl^-$ 12,19 |
| gef.: | C 62,50 | H 7,90 | N 10,19 | $Cl^-$ 13,33 |

Analog kann 2,3-Dimethyl-4,4a,5,6,7,8,8a,9-octahydro-4a,8a-trans-1H-pyrrolo[2,3-g]isochinolin-4-on aus 2,3,6-Trimethyl-4,4a,5,6,7,8,8a,9-octahydro-1H-pyrrolo[2,3-g]isochinolin-4-on hergestellt werden.

Beispiel 6

Herstellung von

3,4-Dihydro-1H-6,8-dimethoxy-2-methyl-isochinolin-Hydrochlorid

Eine Lösung von N-Methyl-(3,5-dimethoxyphenyl)äthylamin-Hydrochlorid (15,0 g, 64,7 mMol) in 30 ml Wasser wurde mit 35 ml 2n Natriumhydroxid behandelt und mit Methylenchlorid extrahiert. Die vereinigten Extrakte wurden am Rotationsverdampfer eingeengt und mit wässrigem Formaldehyd (65 ml, 37%ige Lösung) gemischt. Das Gemisch wurde 2 h rückflussgekocht, mit 2n Natriumhydroxid (15 ml) alkalisch gemacht und mit Methylenchlorid extrahiert. Die vereinigten Extrakte wurden mit Salzlösung gewaschen und über wasserfreiem Magnesiumsulfat getrocknet und zu einem gelben Produktöl (15,5 g) eingeengt. Das Öl wurde in 100 ml Äthanol gelöst und mit äthanolischem Chlorwasserstoff behandelt. Äther (75 ml) wurde zugesetzt, und das Salz kristallisierte aus und ergab 10,15 g 3,4-Dihydro-1H-6,8-dimethoxy-2-methylisochinolin-Hydrochlorid (64% Ausbeute).

Beispiel 7

Herstellung von

1,2,3,4,4a,7-Hexahydro-6,8-dimethoxy-2-methylisochinolin und Octahydro-2-methylisochinolin-6,8-dion

Ammoniak (150 ml) wurde in einen t-Butanol (9,1 g, 123 mMol) und Diäthyläther (50 ml) enthaltenden Kolben kondensiert. Zu der Lösung wurde

3,4-Dihydro-1H-6,8-dimethoxy-2-methylisochinolin-Hydrochlorid

(1,0 g, 4,1 mMol) gegeben. Nach 2 bis 3 min Rühren wurde Lithiumdraht (0,57 g, 82 mMol) in kurzen Stücken über 30 min zugesetzt. Die blaue Lösung wurde 2,5 h unter Rückfluss gerührt, und festes Ammoniumchlorid (4,5 g) wurde zugesetzt, bis die blaue Farbe verschwand. Äther (100 ml) wurde zugesetzt, und das Ammoniak konnte über Nacht abdampfen. Eiswasser (100 ml) wurde zugegeben, und die organische Phase wurde abgetrennt. Die wässrige Schicht wurde mit Äthylacetat und Chloroform extrahiert. Die vereinigten Extrakte wurden mit Salzlösung gewaschen und über wasserfreiem Magnesiumsulfat getrocknet und zu 1,2,3,4,4a,7-Hexa-hydro-6,8-dimethoxy-2-methylisochinolin (0,58 g, 68% Ausbeute) als gelbes Öl eingeengt.

Das Rohprodukt (1,05 g) in 20 ml 70%iger wässriger Essigsäure wurde 5 h rückflussgekocht und die Essigsäure am Rotationsverdampfer entfernt. Der Rückstand wurde in Wasser gelöst und mit Chloroform gewaschen. Die wässrige Phase wurde auf ein Volumen von 10 ml eingeengt und an Dowex AG 50WX 8 unter Elution mit 2molarem wässrigem Pyridin chromatographiert, um 0,11 g Octahydro-2-methylisochinolin-6,8-dion (11,6% Ausbeute) als blassgelben Feststoff zu ergeben. Behandeln mit Chlorwasserstoff in Methanol lieferte das Hydrochlorid, Schmp. 193–196°.

Tabelle I

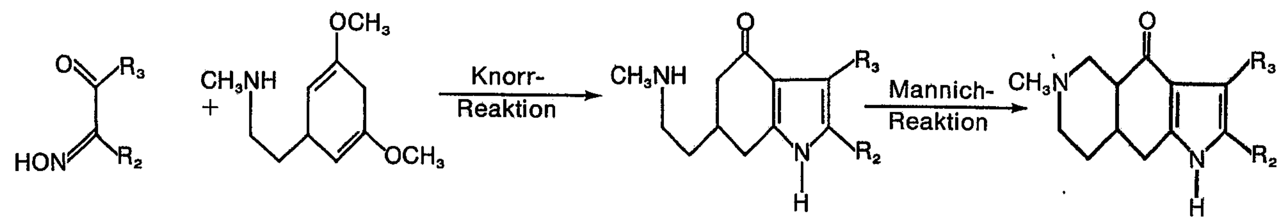


| Beispiel | $R_2$ | $R_3$ | Analyse ber. | gef. | Schmp. | kristallisiert aus | abweichende Arbeitsweise |
|---|---|---|---|---|---|---|---|
| 4a<br>3,6-Dimethyl-2-(2-propyl)-4,4a,-5,6,7,8,8a,9-octahydro-1H-pyrr-olo-[2,3-g]isochinolin-4-on | $CH(CH_3)_2$ | $CH_3$ | (.HCl)<br>C 64,74<br>H 8,49<br>N 9,44<br>$Cl^-$ 11,94 | (.HCl)<br>C 64,53<br>H 8,38<br>N 9,36<br>$Cl^-$ 12,16 | >280°, d | Methanol | – |
| 4b<br>2,6-Dimethyl-3-phenyl-4,4a,5,6,-7,8,8a,9-octahydro-1H-pyrr-olo-[2,3-g]-isochinolin-4-on | $CH_3$ | 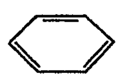 | C 77,23<br>H 7,53<br>N 9,52 | C 77,23<br>H 7,50<br>N 9,54 | >240°, d | Äthanol-<br>Äthylacetat | Mannich-Reaktion in Diäthylen-glykol-monoäthyläther, 155°, 1 h |
| 4c<br>2,3,6-Trimethyl-4,4a,5,6,7,8,8a,9-octahydro-1H-pyrrolo[2,3-g]iso-chinolin-4-on | $CH_3$ | $CH_3$ | (.HCl)<br>C 62,56<br>H 7,88<br>N 10,42<br>$Cl^-$ 13,19 | C 62,19<br>H 7,97<br>N 10,20<br>$Cl^-$ 13,41 | 275–80°, d | Äthanol-<br>Äther | Knorr-Reaktion in n-Butanol bei 170°/3,5 bar (50psi) |

Beispiel 8

Herstellung von
3-Äthyl-2,6-dimethyl-4,4a,5,6,7,8,8a,9-octahydro-1H-pyrrolo[2,3-g]isochinolin-4-on über Octahydro-2-methylisochinolin-6,8-dion

1,2,3,4,4a,7-Hexahydro-6,8-dimethoxy-2-methylisochinolin (0,56 g, 2,68 mMol) wurde auf 90 bis 100° in 70%iger wässriger Essigsäure (10 ml) zur Hydrolyse des Bis(enoläthers) zum Octahydro-2-methylisochinolin-6,8-dion erwärmt. Der heissen Lösung wurde Zinkstaub (0,6 g, 9,25 mMol) und 2-Isonitroso-3-pentanon (0,7 g, 6,1 mMol) zugesetzt. Das Gemisch wurde 3 h rückflussgekocht, gekühlt und filtriert, um Zink und Zinkacetat zu entfernen. Das Filtrat wurde am Rotationsverdampfer zur Trockne eingeengt, und der Rückstand wurde in Methylenchlorid gelöst. Die Lösung wurde mit Ammoniumhydroxid versetzt, und die Schichten wurden getrennt. Die organische Schicht wurde mit Salzlösung gewaschen und über wasserfreiem Natriumsulfat getrocknet und zum Rohprodukt eingeengt. Chromatographie des Rohprodukts an Aluminiumoxid III lieferte
3-Äthyl-2,6-dimethyl-4,4a,5,6,7,8,8a,9-octahydro-1H-pyrrolo[2,3-g]isochinolin-4-on
als weissen Feststoff (0,19 g, 29% Ausbeute).

Beispiel 9

Herstellung von
2,6-Dimethyl-3-isopropyl-4,4a,5,6,7,8,8a,9-octahydro-4a,8a-trans-1H-pyrrolo[2,3-g]isochinolin-4-on

Eine Lösung von rohem Octahydro-2-methylisochinolin-6,8-dion (etwa 12,5 mMol) und 2,4 g (18 mMol) 2-Isonitroso-4-methyl-3-pentanon in 40 ml 70%iger wässriger Essigsäure wurde mit 2,6 g (40 mMol) Zinkstaub behandelt und langsam auf Rückfluss erwärmt. Nach 1 h wurde das Gemisch leicht gekühlt, und weitere 0,4 g Isonitrosoketon und 1,0 g Zink wurden zugegeben und das Gemisch 1,5 h bei Rückfluss gerührt. Das Gemisch wurde dann gekühlt und filtriert, und das Filtrat wurde bei 50°/20 mm Hg zu einem gelben Öl eingeengt, das mit 50 ml Wasser verdünnt und mit Ammoniumhydroxid (auf pH 8 bis 9) alkalisch gemacht wurde. Das Gemisch wurde mit Chloroform extrahiert, und die Extrakte wurden mit Salzlösung gewaschen und über Natriumsulfat getrocknet und zu 2,6 g Rohprodukt eingeengt. Das Material wurde an 100 g Kieselgel (trockene Säule) unter Elution mit der organischen Phase eines durch Gleichgewichtseinstellung von 90 (Volumen-)Teilen Chloroform, 30 Teilen Methanol, 10 Teilen Wasser und 6 Teilen Essigsäure hergestellten Gemisches chromatographiert. Die produkthaltigen Eluatfraktionen wurden eingedampft, mit Wasser verdünnt, mit Ammoniumhydroxid (auf pH 8 bis 9) alkalisch gemacht und mit Chloroform extrahiert. Die Extrakte wurden über Natriumsulfat getrocknet und zu 1,0 g festen Produkts eingedampft, das zweimal aus Äthylacetat zu 470 mg reinem
2,6-Dimethyl-3-isopropyl-4,4a,5,6,7,8,8a,9-octahydro-4a,8a-trans-1H-pyrrolo[2,3-g]isochinolin-4-on
als kristallinem Feststoff, Schmp. 244–247°, umkristallisiert wurde.

Beispiel 10

Herstellung von
3,6-Dimethyl-2-(2-propenyl)-4,4a,5,6,7,8,8a,9-octahydro-4a,8a-trans-1H-pyrrolo[2,3-g]isochinolin-4-on

Ähnlich wie in Beispiel 9 beschrieben, lieferten 3-Isonitroso-5-hexen-2-on und 2-Methyl-octahydroisochinolin-6,8-dion 3,6-Dimethyl-2-(2-propenyl)-4,4a,5,6,7,8,8a,9-octahydro-4a,8a-trans-1H-pyrrolo[2,3-g]isochinolin-4-on,
Schmp. 221–223°.

Beispiel 11

Herstellung von
3-Cyclopropyl-2,6-dimethyl-4,4a,5,6,7,8,8a,9-octahydro-4a,8a-trans-1H-pyrrolo[2,3-g]isochinolin-4-on

Ähnlich wie in Beispiel 9 beschrieben, lieferten Cyclopropyl-2-isonitroso-1-propanon und 2-Methyl-octahydroisochinolin-6,8-dion
3-Cyclopropyl-2,6-dimethyl-4,4a,5,6,7,8,8a,9-octahydro-4a,8a-trans-1H-pyrrolo[2,3-g]isochinolin-4-on,
Schmp. 258–259° (Zers.).

Beispiel 12

Herstellung von
2-Benzyl-3,6-dimethyl-4,4a,5,6,7,8,8a,9-octahydro-4a,8a-trans-1H-pyrrolo[2,3-g]isochinolin-4-on

Ähnlich wie in Beispiel 9 beschrieben, lieferten 3-Isonitroso-4-phenyl-2-butanon und 2-Methyl-octahydroisochinolin-6,8-dion
2-Benzyl-3,6-dimethyl-4,4a,5,6,7,8,8a,9-octahydro-4a,8a-trans-1H-pyrrolo[2,3-g]isochinolin-4-on,
Schmp. 234–235°.

Beispiel 13

Herstellung von
6-Methyl-4,4a,5,6,7,8,8a,9-octahydro-4a,8a-trans-1H-pyrrolo[2,3-g]isochinolin-4-on

Ähnlich wie in Beispiel 9 beschrieben, mit der Ausnahme, dass kein Zink verwendet wurde, lieferten Aminoacetaldehyd-dimethylacetal und 2-Methyl-octahydroisochinolin-6,8-dion
6-Methyl-4,4a,5,6,7,8,8a,9-octahydro-4a,8a-trans-1H-pyrrolo[2,3-g]isochinolin-4-on,
Schmp. 208–210°.

Beispiel 14

Herstellung von
3-Äthyl-1,2,6-trimethyl-4,4a,5,6,7,8,8a,9-octahydro-4a,8a-trans-1H-pyrrolo[2,3-g]isochinolin-4-on

Flüssiges Ammoniak (80 ml) wurden in einen eine Suspension von
2,6-Dimethyl-3-äthyl-4,4a,5,6,7,8,8a,9-octahydro-1H-pyrrolo[2,3-g]isochinolin-4-on
(0,984 g, 4,0 mMol) in (24 ml) Äther enthaltenden Kolben kondensiert. Natriummetall (0,138 g, 6,0 mMol) wurde zugesetzt, und die Lösung wurde gerührt, bis das gesamte Natrium gelöst war. Eine

Lösung von Methyljodid (1,28 g, 9,0 mMol) in Äther (16 ml) wurde zugesetzt, und das Gemisch wurde bei Raumtemperatur gerührt, bis das Ammoniak verdampft war. Wasser und Chloroform wurden zugesetzt, und die wässrige Schicht wurde abgetrennt und mit Chloroform extrahiert. Die vereinigten Extrakte wurden mit Salzlösung gewaschen und über wasserfreiem Natriumsulfat getrocknet und zu 1,14 g Rohprodukt eingeengt, das an Aluminiumoxid III zu 0,572 g eines weissen festen Produkts chromatographiert wurde. Die chromatographierte freie Base wurde mit ätherischem Chlorwasserstoff in das Hydrochlorid überführt und aus Äthylacetat/Äthanol und Äthanol kristallisiert, um reines

3-Äthyl-1,2,6-trimethyl-4,4a,5,6,7,8,8a,9-octahydro-4a,8a-trans-1H-pyrrolo[2,3-g]isochinolin-4-on-Hydrochlorid

(0,33 g, 28% Ausbeute) als weisse Schmp. 241 bis 243°, zu ergeben.

Analyse, ber. für $C_{16}H_{24}N_2O \cdot HCl$:

| | | | | |
|---|---|---|---|---|
| | C 64,74 | H 8,49 | N 9,44 | $Cl^-$ 11,94 |
| gef: | C 64,72 | H 8,63 | N 9,29 | $Cl^-$ 12,03 |

Beispiel 15

Herstellung von

1-Benzoyl-2,6-dimethyl-3-äthyl-4,4a,5,6,7,8,8a,9-octahydro-4a,8a-trans-1H-pyrrolo[2,3-g]isochinolin-4-on

Zu einer Suspension von

3-Äthyl-2,6-dimethyl-4,4a,5,6,7,8,8a,9-octahydro-1H-pyrrolo[2,3-g]isochinolin-4-on

(492,7 mg, 2,0 mMol) in trockenem Tetrahydrofuran (10 ml) bei −30° wurde Butyllithium (1,6 ml, 2,4 mMol, 1,5 m Lösung in Hexan) über 2 bis 3 min mit Hilfe einer Spritze gegeben. Die Lösung wurde 1 h bei −30° gerührt, und Benzoylchlorid (336 mg, 2,4 mMol) wurde über 2 bis 3 min zugegeben. Die erhaltene Lösung wurde 1 h bei −25 bis −35° und 0,5 h bei Raumtemperatur gerührt. Die Lösung wurde in Eiswasser (30 ml) gegossen und mit Chloroform extrahiert. Die vereinigten Extrakte wurden mit Salzlösung gewaschen und über wasserfreiem Natriumsulfat getrocknet. Verdampfen des Lösungsmittels lieferte das Rohprodukt (0,9 g), das chromatographiert wurde (trockene Säule, Kieselgel, Elution mit einer Chloroform/wässrigen Methanol/Essigsäure-Lösung). Die Säulenfraktionen wurden mit Ammoniumhydroxid behandelt, mit Chloroform extrahiert, mit Wasser gewaschen und über wasserfreiem Natriumsulfat getrocknet. Verdampfen des Lösungsmittels ergab

1-Benzoyl-2,6-dimethyl-3-äthyl-4,4a,5,6,7,8,8a,9-octahydro-4a,8a-trans-1H-pyrrolo[2,3-g]isochinolin-4-on

als einen Feststoff, der aus Cyclohexan umkristallisiert wurde, um das reine Produkt, Schmp. 144–146°, zu ergeben.

Analyse, ber. für $C_{22}H_{26}N_2O_2$:

| | | | |
|---|---|---|---|
| | C 75,40 | H 7,48 | N 7,99 |
| gef.: | C 75,63 | H 7,79 | N 8,01 |

Unter Anwendung der Arbeitsweise des Beispiels 15 wurden die folgenden Verbindungen hergestellt. Die isolierten Verbindungen sind die 4a,8a-trans-Isomeren.

Aus

2,6-Dimethyl-3-äthyl-4,4a,5,6,7,8,8a,9-octahydro-1H-pyrrolo[2,3-g]isochinolin-4-on

und Benzylchlorid wurde

1-Benzyl-2,6-dimethyl-3-äthyl-4,4a,5,6,7,8,8a,9-octahydro-1H-pyrrolo[2,3-g]isochinolin-4-on

erhalten.

Aus

2,6-Dimethyl-3-äthyl-4,4a,5,6,7,8,8a,9-octahydro-1H-pyrrolo[2,3-g]isochinolin-4-on

und 1-Trimethylacetylchlorid wurde

2,6-Dimethyl-3-äthyl-1-(2,2-dimethyl-1-oxopropyl)-4,4a,5,6,7,8,8a,9-octahydro-1H-pyrrolo[2,3-g]isochinolin-4-on,

Schmp. 110–112°, erhalten.

Aus

2-Methyl-3-äthyl-6-(2-phenyläthyl)-4,4a,5,6,7,8,8a,9-octahydro-1H-pyrrolo[2,3-g]isochinolin-4-on

und Methyljodid wurde

3-Äthyl-1,2-dimethyl-6-(2-phenyläthyl)-4,4a,5,6,7,8,8a,9-octahydro-4a,8a-trans-1H-pyrrolo[2,3-g]isochinolin-4-on,

Schmp. 180–181°, erhalten.

Beispiel 16

Herstellung von

3-Äthyl-2-methyl-6-(2-propenyl)-4,4a,5,6,7,8,8a,9-octahydro-4a,8a-trans-1H-pyrrolo[2,3-g]isochinolin-4-on

Ein Gemisch aus 3-Äthyl-2-methyl-4,4a,5,6,7,-8,8a,9-octahydro-1H-pyrrolo[2,3-g]isochinolin-4-on (0,470 g, 2,03 mMol), Allylbromid (0,5 g, 4,13 mMol) und Kaliumcarbonat (0,85 g, 6,16 mMol) in Aceton (35 ml) wurde bei Raumtemperatur 2 h gerührt und filtriert. Das Filtrat wurde eingeengt und der Rückstand (0,53 g) an Aluminiumoxid III zu dem Produkt (0,40 g) chromatographiert. Dieses Produkt wurde mit ätherischem Chlorwasserstoff zum Hydrochlorid behandelt, das aus Äthanol/Äthylacetat umkristallisiert wurde, um

3-Äthyl-2-methyl-6-(2-propenyl)-4,4a,5,6,7,8,8a,9-octahydro-4a,8a-trans-1H-pyrrolo[2,3-g]isochinolin-4-on-Hydrochlorid

als weissen Feststoff, Schmp. 214–217°, zu ergeben.

Analyse, ber. für $C_{17}H_{24}N_2O \cdot HCl \cdot 0{,}5H_2O$:

| | | | | |
|---|---|---|---|---|
| | C 64,24 | H 8,25 | N 8,81 | $Cl^-$ 11,15 |
| gef.: | C 64,50 | H 8,48 | N 8,96 | $Cl^-$ 11,37 |

Beispiele 16a–dd

Nach der Arbeitsweise des Beispiels 16 wurden die in Tabelle II aufgeführten Verbindungen aus dem angegebenen

4,4a,5,6,7,8,8a,9-Octahydro-1H-pyrrolo[2,3-g]isochinolin-4-on

und dem bezeichneten Halogenid hergestellt. Jede Verbindung zeigte Spektraldaten, die mit der beschriebenen Struktur im Einklang standen. Schmelzpunkte sind für die freie Base oder das Hydrochlorid angegeben, wie bezeichnet. Die isolierten Verbindungen sind die 4a,8a-trans-Isomeren.

Tabelle II

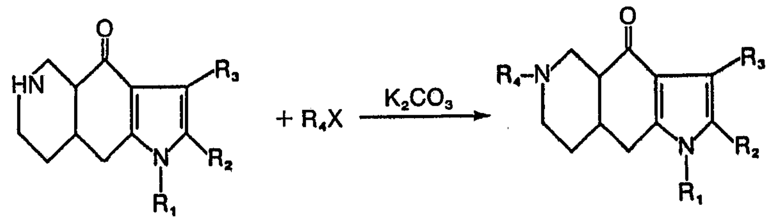


| Beispiel | $R_1$ | $R_2$ | $R_3$ | $R_4$ | X | Analyse ber. | gef. | Schmp. | kristalli- siert aus | Reaktionsbe- dingungen Temp. | Zeit |
|---|---|---|---|---|---|---|---|---|---|---|---|
| 16a<br>2-Methyl-3-äthyl-6-(cyclopropylmethyl-4,4a,5,6,7,8 8a,9-octahydro-1H-pyrrolo-[2,3-g]-isochinolin-4-on, HCl, 0,2 molar-Hydrat | H | $CH_3$ | $CH_2CH_3$ | 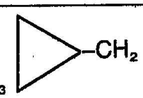 | Cl | (.HCl)<br>C 66,22<br>H 8,15<br>N 8,58<br>$Cl^-$ 10,85 | C 66,24<br>H 8,35<br>N 8,36<br>$Cl^-$ 10,51 | 215–9° | Äthanol/ Äthyl-acetat | Rückfluss in Aceton | 16 h |
| 16b<br>2-Methyl-3-äthyl-6-benzyl-4,4a,5,6,7,8,8a,9-octahydro-1H-pyrrolo[2,3-g]isochinolin-4-on-HCl | H | $CH_3$ | $CH_2CH_3$ | 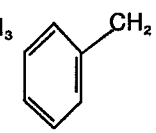 | Cl | (.HCl)<br>C 70,27<br>H 7,58<br>N 7,81<br>$Cl^-$ 9,88 | C 70,49<br>H 7,33<br>N 7,72<br>$Cl^-$ 10,03 | 193–7° | Äthanol | Rückfluss in Aceton | 2 h |
| 16c<br>rac. 2-Methyl-3-äthyl-6-[2-(dimethylamino)äthyl]-4,4a,5,6,7,8,8a,9-octahydro-4a,8a-trans-1H-pyrrolo-[2,3-g]isochinolin-4-on | H | $CH_3$ | $CH_2CH_3$ | $(CH_3)_2NCH_2CH_2$ | Cl | C 71,25<br>H 9,63<br>N 13,85 | C 71,03<br>H 9,73<br>N 13,66 | 184–6° | Äthyl-acetat | Rückfluss | 20 h in 3-Pentanon |
| 16d<br>rac.2-Methyl-3-äthyl-6-[4-(4-fluorphenyl)-4-oxobutyl]-4,4a,5,6,7,8,8a,9-octahydro-4a,8,trans-1H-pyrrolo-[2,3-g]isochinolin-4-on | H | $CH_3$ | $CH_2CH_3$ | 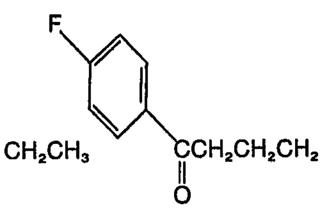 | Cl | C 72,70<br>H 7,37<br>N 7,07<br>F 4,79 | C 72,47<br>H 7,47<br>N 7,09<br>F 4,75 | 210–12° | Äthyl-acetat | Rückfluss | 24 h in 3-Pentanon |

Tabelle II (Fortsetzung)

| | | | | | | | | | | | | | |
|---|---|---|---|---|---|---|---|---|---|---|---|---|---|
| 16e<br>rac. 2-Methyl-3-äthyl-6-(2-phenyläthyl)-4,4a,5,6,7,8,8a,9-octahydro-4a,8a-trans-1H-pyrrolo[2,3-g]isochinolin-4-on | H | $CH_3$ | $CH_2CH_3$ | 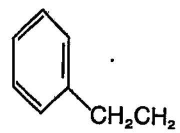 | Br | C<br>H<br>N | 78,53<br>8,39<br>8,33 | C<br>H<br>N | 78,53<br>8,40<br>8,35 | 239–40° | Äthyl-acetat/<br>Äthanol | Rückfluss | 2 h in 3-Pentanon |
| 16f<br>rac. 2-Methyl-3-äthyl-6-(2-äthoxyäthyl)-4,4a,5,6,7,8,8a,9-octahydro-4a,8a-trans-1H-pyrrolo-[2,3-g]isochinolin-4-on-Hydrochlorid | H | $CH_3$ | $CH_2CH_3$ | $CH_3CH_2OCH_2CH_2$ | Br | C<br>H<br>N<br>$Cl^-$ | 63,42<br>8,57<br>8,21<br>10,40 | C<br>H<br>N<br>$Cl^-$ | 63,13<br>8,71<br>8,1<br>10,64 | 213–5° | Äthanol/<br>Äthyl-acetat | Rückfluss | 2 h in 3-Pentanon |
| 16g<br>rac. 3,6-Diäthyl-2-methyl-4,4a,5,6,7,8,8a,9-octahydro-4a,8a-trans-1H-pyrrolo-[2,3-g]isochinolin-4-on | H | $CH_3$ | $CH_2CH_3$ | $CH_3CH_2$ | Br | C<br>H<br>N | 73,81<br>9,29<br>10,76 | C<br>H<br>N | 73,91<br>9,30<br>10,84 | 228–30° | Äthanol | Rückfluss | 3 h in Aceton |
| 16h<br>rac. 3-Äthyl-2-methyl-6-propyl-4,4a,5,6,7,8,8a,9-octahydro-4a,8a-trans-1H-pyrrolo[2,3-g]-isochinolin-4-on | H | $CH_3$ | $CH_2CH_3$ | $CH_3CH_2CH_2$ | Br | C<br>H<br>N | 74,41<br>9,55<br>10,21 | C<br>H<br>N | 74,29<br>9,40<br>10,21 | 226–8° | Äthanol | Rückfluss | 13 h in 3-Pentanon |
| 16i<br>4a,8a-trans-3-Äthyl-4-oxo-4a,5,7,8,8a,9-hexahydro-2-methyl-1H,4H-pyrrolo-[2,3-g]-isochinolin -6-essigsäure-äthylester | H | $CH_3$ | $CH_2CH_3$ | $CH_3CH_2OOCCH_2$ | Br | C<br>H<br>N | 67,90<br>8,23<br>8,80 | C<br>H<br>N | 68,09<br>8,28<br>8,80 | 215–6° | Äthanol/<br>Äthyl-acetat | Rückfluss | 2 h in 3-Pentanon |
| 16j<br>4a,8a-trans-1-[Äthyl-4a,5,7,8,8a,9-hexahydro-2-methyl-4-oxo-1H,4H-pyrrolo-[2,3-g]isochinolin-6-yl]-2-propanon | H | $CH_3$ | $CH_2CH_3$ | $H_3CC(=O)CH_2$ | Cl | C<br>H<br>N | 70,80<br>8,39<br>9,71 | N<br>H<br>N | 71,10<br>8,30<br>9,93 | 192–5° | Äthanol | Rückfluss | 2 h in 3-Pentanon |

Tabelle II (Fortsetzung)

| Beispiel | $R_1$ | $R_2$ | $R_3$ | $R_4$ | X | Analyse ber. | Analyse gef. | Schmp. | kristallisiert aus | Reaktionsbedingungen Temp. | Reaktionsbedingungen Zeit |
|---|---|---|---|---|---|---|---|---|---|---|---|
| 16k<br>rac. 2-Methyl-3-äthyl-6-[2-(4-fluorphenyl)-2-oxoäthyl]-4,4a, 5,6,7,8,8a,9-octahydro-4a,8a-trans-1H-pyrrolo[2,3-g]-isochinolin-4-on | H | $CH_3$ | $CH_2CH_3$ | 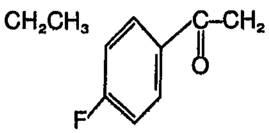 | Cl | C 71,72<br>H 6,84<br>N 7,60 | C 71,44<br>H 6;80<br>N 7,47 | 209–13° | Äthanol | Rückfluss | 3 h in 3-Pentanon |
| 16l<br>rac. 2-Methyl-3-äthyl-6-[3-(4-fluorphenyl)-3-oxo-propyl]-4,4a,5,6,7,8,8a,9-octahydro-4a,8a-trans-1H-pyrrolo[2,3-g]isochinolin-4-on | H | $CH_3$ | $CH_2CH_3$ | 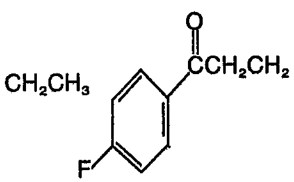 | Cl | C 72,23<br>H 7,12<br>N 7,32 | C 72,24<br>H 7,00<br>N 7,19 | 208–10° | Äthanol | Rückfluss | 3 h in 3-Pentanon |
| 16m<br>rac. 3-Äthyl-2-methyl-6-(3-phenoxypropyl)-4,4a,5,6,7,8,8a,9-octahydro-1H-4a,8a-trans-pyrrolo[2,3-g]isochinolin-4-on | H | $CH_3$ | $CH_2CH_3$ | 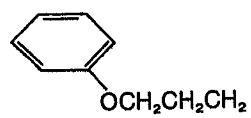 | Br | C 75,37<br>H 8,24<br>N 7,64 | C 75,64<br>H 8,18<br>N 7,52 | 202–3° | Äthanol | Rückfluss | 2 h in 3-Pentanon |
| 16n<br>rac. 6-(3-Diphenylpropyl)-3-äthyl-2-methyl-4,4a,5,6,7,8,8a-9-octahydro-4a,8a-trans-1H-pyrrolo[2,3-g]isochinolin-4-on | H | $CH_3$ | $CH_2CH_3$ | 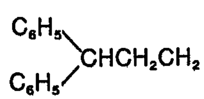 | Cl | C 81,65<br>H 8,03<br>N 6,57 | C 81,60<br>H 7,85<br>N 6,72 | 220–2° | | Rückfluss | 17 h in 3-Pentanon |

Tabelle II (Fortsetzung)

| | | | | | | | | | | | | | |
|---|---|---|---|---|---|---|---|---|---|---|---|---|---|
| 16o<br>rac. 3-Äthyl-6-[2-(4-methoxy-phenyl)-äthyl]-2-methyl-4,4a,5,6,7,8,8a,9-octahydro-4a,8a-trans-1H-pyrrolo[2,3-g]-isochinolin-4-on | H | $CH_3$ | $CH_2CH_3$ | 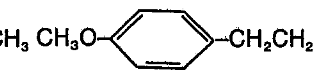 | Cl | C<br>H<br>N | 75,37<br>8,25<br>7,64 | C<br>H<br>N | 75,65<br>8,41<br>7,73 | 255–7° | Äthanol | Rückfluss | 24 h in 3-Pentanon |
| 16p<br>3-Äthyl-4,4a,5,6,7,8,8a,9-octa-hydro-2-methyl-6-[2-[tricyclo-[3,3,1,1/3,7/]decan-1-yl]-2-oxo-äthyl]-4a,8a-trans-1H-pyrrolo-[2,3-g]isochinolin-4-on | H | $CH_3$ | $CH_2CH_3$ | 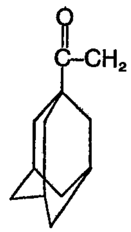 | Br | C<br>H<br>N | 76,43<br>8,88<br>6,86 | C<br>H<br>N | 76,34<br>8,75<br>6,62 | 237–40° | Äthanol | Rückfluss | 3 h in 3-Pentanon |
| 16q<br>rac. 3-Äthyl-2-methyl-6-(2-methylpropyl)-4,4a,5,6,7,8,8a,9-octahydro-4a,8a-trans-1H-pyrrolo[2,3-g]isochinolin-4-on | H | $CH_3$ | $CH_2CH_3$ | 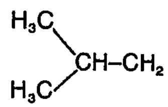 | Br | C<br>H<br>N | 74,96<br>9,79<br>9,71 | C<br>H<br>N | 75,24<br>9,89<br>9,70 | 213–15° | Äthanol | Rückfluss | 10 h in 3-Pentanon |
| 16r<br>rac. 3-Äthyl-6-[2-(4-chlorphe-nyl)-äthyl]-2-methyl-4,4a,5,6,7,8,8a,9-octahydro-4n,8n-trans-1H-pyrrolo-[2,3-g]isochinolin-4-on | H | $CH_3$ | $CH_2CH_3$ | 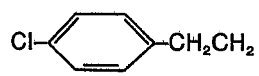 | Cl | C<br>H<br>N | 71,24<br>7,34<br>7,55 | C<br>H<br>N | 71,25<br>7,16<br>7,63 | 264–7° | 1,4-Dioxan | Rückfluss | 20 h in 3-Pentanon |
| 16s<br>rac. 3-Äthyl-6-[2-(äthenyloxy)-äthyl]-2-methyl-4,4a,5-6,7,8,8a,-9-octahydro-4a,8a-trans-1H-pyrrolo[2,3-g]isochinolin-4-on | H | $CH_3$ | $CH_2CH_3$ | $CH_2 = CHOCH_2CH_2$ | Cl | C<br>H<br>N | 71,49<br>8,67<br>9,26 | C<br>H<br>N | 71,31<br>8,59<br>9,25 | 174–7° | Acetonitril | Rückfluss | 10 h in 3-Pentanon |
| 16t<br>rac. 3-Äthyl-2-methyl-6-(3-phenyl-2-propenyl)-4,4a,5,6,-7,8,8a,9-octahydro-4a,8a-trans-1H-pyrrolo[2,3-g]isochinolin-4-on | H | $CH_3$ | $CH_2CH_3$ | $C_6H_5CH = CHCH_2$ | Br | C<br>H<br>N | 79,27<br>8,10<br>8,04 | C<br>H<br>N | 79,10<br>8,04<br>8,10 | 218–20° | Äthanol | Rückfluss | 4 h in 3-Pentanon |

Tabelle II (Fortsetzung)

| Beispiel | $R_1$ | $R_2$ | $R_3$ | $R_4$ | X | Analyse ber. | | Analyse gef. | | Schmp. | kristallisiert aus | Reaktionsbedingungen Temp. | Zeit |
|---|---|---|---|---|---|---|---|---|---|---|---|---|---|
| 16u<br>4a,8a-trans-3-Äthyl-4a,5,7,8,-8a,9-hexahydro-2-methyl-4-oxo-1H,4H-pyrrolo[2,3-g]isochinolin-6-propionsäureäthylester | H | $CH_3$ | $CH_2CH_3$ | $CH_3CH_2OOCCH_2CH_2$ | Br | C<br>H<br>N | 68,65<br>8,49<br>8,43 | C<br>H<br>N | 68,59<br>8,50<br>8,55 | 180–4° | Acetonitril | Rückfluss | 3 h in 3-Pentanon |
| 16v<br>rac.-6-(Cyclobutylmethyl)-3-äthyl-2-methyl-4,4a,5,6,7,-8,8a,9-octahydro-4a,8a-trans-1H-pyrrolo[2,3-g]isochinolin-4-on | H | $CH_3$ | $CH_2CH_3$ | 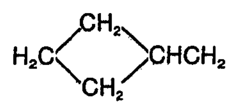 | Cl | C<br>H<br>N | 75,96<br>9,39<br>9,32 | C<br>H<br>N | 75,70<br>9,34<br>9,23 | 222–4° | Äthanol | Rückfluss | 18 h in 3-Pentanon |
| 16w<br>rac.-6-(4-Diphenylbutyl)-3-äthyl-2-methyl-4,4a,5,6,-7,8,8a,9-octahydro-4a,8a-trans-1H-pyrrolo[2,3-g]-isochinolin-4-on | H | $CH_3$ | $CH_2CH_3$ | 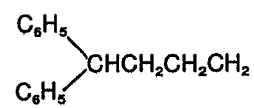 | Cl | C<br>H<br>N | 81,78<br>8,24<br>6,36 | C<br>H<br>N | 81,88<br>8,16<br>6,36 | 236–8° | Äthanol | Rückfluss | 18 h in 3-Pentanon |
| 16x<br>trans-[2-[3-Äthyl-4,4a,5,6,-7,8,8a,9-octahydro-2-methyl-4-oxo-1H-pyrrolo[2,3-g]isochinolin-6-yl]-äthyl]benzoesäureester | H | $CH_3$ | $CH_2CH_3$ | 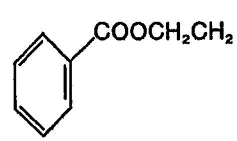 | Br | C<br>H<br>N | 72,60<br>7,42<br>7,36 | C<br>H<br>N | 72,31<br>7,58<br>7,22 | 206—8° | Äthanol | Rückfluss | 6 h in 3-Pentanon |
| 16y<br>rac. 3-Äthyl-2-methyl-6-[2-(N-methyl-2-pyrrolidinyl)-äthyl]-4,4a,5,6,7,8,8a,9-octahydro-4a,8a-trans-1H-pyrrolo-[2,3-g]isochinolin-4-on | H | $CH_3$ | $CH_2CH_3$ | 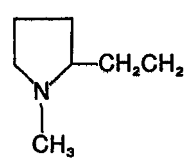 | Cl | C<br>H<br>N | 73,43<br>9,68<br>12,23 | C<br>H<br>N | 73,25<br>9,54<br>12,41 | 192–4° | Äthylacetat | Rückfluss | 20 h in 3-Pentanon |

Tabelle II (Fortsetzung)

| | | | | | | | | | | | |
|---|---|---|---|---|---|---|---|---|---|---|---|
| 16z<br>trans-[3-[3-Äthyl-4,4a,5,6-7,8,8a,9-octahydro-2-methyl-4-oxo-1H-pyrrolo[2,3-g]isochii-nolin-6-yl]propyl]benzoat | H | $CH_3$ | $CH_2CH_3$ | 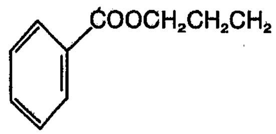 | Br | C 73,07<br>H 7,67<br>N 7,10 | C 73,12<br>H 7,63<br>N 7,07 | 189–90,5 | Äthanol | Rückfluss | 8 h in 3-Pentanon |
| 16aa<br>rac.-3-Äthyl-2-methyl-6-(2,3-dihydroxypropyl)-4,4a,5,6,7,8,8a,9-octahydro-4a,8a-trans-1H-pyrrolo-[2,3-g]isochinolin-4-on | H | $CH_3$ | $CH_2CH_2$ | 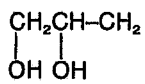 | Cl | C 66,64<br>H 8,55<br>N 9,14 | C 66,32<br>H 9,07<br>N 9,07 | 210–12° | Äthanol | Rückfluss | 20 h in 3-Pentanon |
| 16bb<br>3-Äthyl-2-methyl-6-[(2,2-dimethyl-1,3-dioxolan-4-yl) methyl]-4,4a,5,6,7,8,8a,9-octahydro-4a,8a-trans-1H-pyrrolo[2,3-g]isochinolin-4-on | H | $CH_3$ | $CH_2CH_3$ | 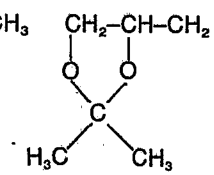 | Br | C 69,33<br>H 8,73<br>N 8,09 | C 69,29<br>H 8,74<br>N 8,12 | 233–5° | Äthanol | Rückfluss | 20 h in 3-Pentanon |
| 16cc<br>rac. 3-Äthyl-6-[2-(4-morpholinyl)äthyl]-2-methyl-4,4a,5,6,7,8,8a,9-octahydro-4a,8a-trans-1H-pyrrolo-[2,3-g]isochinolin-4-on | H | $CH_3$ | $CH_2CH_3$ | 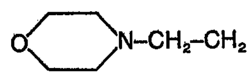 | Cl | C 69,53<br>H 9,04<br>N 12,16 | C 69,59<br>H 9,12<br>N 12,12 | 206–8° | Äthanol | Rückfluss | 8 h in 3-Pentanon |
| 16dd<br>rac. 6-Benzyl-2,3-dimethyl-4,4a,5,6,7,8,8a,9-octahydro-1H-4a,8a-transpyrrolo[2,3-g]isochi-nolin-4-on | H | $CH_3$ | $CH_3$ | 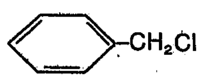 | Cl | C 77,89<br>H 7,84<br>N 9,08 | C 77,61<br>H 7,63<br>N 9,13 | 245,5–6° | Äthanol | Rückfluss | 3 h in Aceton |

Beispiel 17

Herstellung von

3-Äthyl-2-methyl-6-(2-hydroxy-2-phenyläthyl)-4,4a,5,6,7,8,8a,9-octahydro-4a,8a-trans-1H-pyrrolo[2,3-g]isochinolin-4-on

Ein Gemisch von 3-Äthyl-2-methyl-4,4a,5,6,7,8,8a,9-octahydro-1H-pyrrolo[2,3-g]isochinolin-4-on (0,83 g, 3,58 mMol) und Styroloxid (0,51 g, 4,22 mMol) in Methanol (25 ml) wurde 2,5 h rückflussgekocht, gekühlt und filtriert. Das Filtrat wurde eingeengt, und der Rückstand wurde an Aluminiumoxid III zu 0,69 g Rohprodukt chromatographiert. Umkristallisieren aus Äthylacetat/Äthanol lieferte 0,195 g 3-Äthyl-2-methyl-6-(2-hydroxy-2-phenyläthyl)-4,4a,5,6,7,8,8a,9-octahydro-4a,8a-trans-1H-pyrrolo[2,3-g]isochinolin-4-on als weissen Feststoff, Schmp. 218,5–220°.

Analyse, ber. für $C_{22}H_{28}N_2O_2$:

| | | | |
|---|---|---|---|
| | C 74,97 | H 8,01 | N 7,95 |
| gef.: | C 74,87 | H 7,92 | N 7,95 |

Beispiele 17a bis 17f

Nach der Arbeitsweise des Beispiels 17 wurden die in Tabelle III aufgeführten Verbindungen aus 3-Äthyl-2-methyl-4,4a,5,6,7,8,8a,9-octahydro-1H-pyrrolo[2,3-g]isochinolin-4-on und dem angegebenen Epoxid hergestellt. Jede Verbindung zeigte Spektraldaten, die mit der beschriebenen Struktur übereinstimmten. Schmelzpunkte sind für die freie Base oder das Hydrochlorid, wie bezeichnet angegeben. Die isolierten Verbindungen sind die 4a,8a-trans-Isomeren.

Beispiel 18

Spaltung racemischen

3-Äthyl-2,6-dimethyl-4,4a,5,6,7,8,8a,9-octahydro-4a,8a-trans-1H-pyrrolo[2,3-g]isochinolin-4-ons

Die racemische freie Base (hergestellt wie in Beispiel 4) (1,20 g) wurde in Methanol gelöst, und eine Lösung von d-(+)-Weinsäure (0,74 g) in Methanol wurde zugesetzt. Die Lösung wurde eingeengt und zweimal aus Methanol umkristallisiert. Das kristalline d-(+)-Tartrat wurde mit Ammoniumhydroxid zur Freisetzung der freien Base behandelt, und die freie Base wurde mit wasserfreiem ätherischem Chlorwasserstoff behandelt, um das Hydrochlorid zu ergeben. Nach zweimaligem Umkristallisieren aus Äthanol und Trocknen bei 80°/0,005 mm wurden 0,15 g des (−)-Enantiomeren als weisser kristalliner Feststoff, Schmp. 240–245°, erhalten.

Drehung: $[\alpha]_D^{25}$ −120,78° (c 0,81%, Wasser)

Analyse ber. für $C_{15}H_{22}N_2O \cdot HCl \cdot 0{,}25H_2O$

| | | | |
|---|---|---|---|
| | C 62,70 | H 8,24 | N 9,75 |
| gef.: | C 62,44 | H 8,33 | N 9,67 |

Die Mutterlaugen aus der Kristallisation des d-(+)-Tartrats wurden mit Ammoniumhydroxid zur Freisetzung der freien Base behandelt, die mit einer Lösung von 1-(−)-Weinsäure (0,46 g) in Methanol behandelt wurde. Die Lösung wurde eingeengt und zweimal aus Methanol umkristallisiert, wie oben beschrieben in die freie Base und das Hydrochlorid überführt, um 0,10 g des (+)-Enantiomeren als weissen, kristallinen Feststoff, Schmp. 240–244°, zu ergeben.

Drehung: $[\alpha]_D^{25}$ +121,38° (c 0,44%, Wasser)

Analyse ber. für $C_{15}H_{22}N_2O \cdot HCl \cdot 0{,}25H_2O$

| | | | |
|---|---|---|---|
| | C 62,70 | H 8,24 | N 9,75 |
| gef.: | C 63,02 | H 8,20 | N 9,88 |

Beispiel 19

Herstellung von

N-[2-(3-Äthyl-4,4a,5,6,7,8,8a,9-octahydro-2-methyl-4-oxo-4a,8a-trans-1H-pyrrolo[2,3-g]isochinolin-6-yl)-äthyl]-4-fluorbenzamid

N-[2-(3-Äthyl-4,4a,5,6,7,8,8a,9-octahydro-2-methyl-4-oxo-4a,8a-trans-1H-pyrrolo[2,3-g]isochinolin-6-yl)äthyl]-4-fluorbenzamid wurde durch Erwärmen von 3-Äthyl-2-methyl-4,4a,5,6,7,8,8a,9-octahydro-1H-pyrrolo[2,3-g]isochinolin-4-on und 1-(4-Fluorbenzoyl)-aziridin in einem Gemisch aus Benzol und Methanol für 2 h hergestellt. Das Rohprodukt kristallisierte aus Äthanol als weisser Feststoff, Schmp. 252–253°. Das Ausgangs-Aziridin wurde aus Aziridin und p-Fluorbenzoylchlorid und Natriumbicarbonat in Wasser hergestellt.

Beispiel 20

Herstellung von

3-Äthyl-2,6-dimethyl-4,4a,5,6,7,8,8a,9-octahydro-4a,8a-trans-1H-pyrrolo[2,3-g]isochinolin-4-thion

Ein Gemisch von 2,48 g (0,01 Mol) 3-Äthyl-2,6-dimethyl-4,4a,5,6,7,8,8a,9-octahydro-1H-pyrrolo[2,3-g]isochinolin-4-on und 2,44 g (0,008 Mol) $P_4S_{10}$ in 100 ml Dioxan wurde 17 h gerührt und rückflussgekocht. Das Dioxan wurde unter vermindertem Druck verdampft, und 150 ml Wasser und genügend Ammoniumhydroxid wurden zugesetzt, um den pH auf 8 bis 9 zu bringen. Das Gemisch wurde mit Chloroform extrahiert, und die Extrakte wurden mit Wasser gewaschen und über Natriumsulfat getrocknet. Verdampfen des Lösungsmittels ergab das rohe Thion (3,3 g) als harzartiges Material. Trockensäulenchromatographie lieferte 1,2 g festes Thion, das zweimal aus Acetonitril umkristallisiert wurde, um reines 3-Äthyl-2,6-dimethyl-4,4a,5,6,7,8,8a,9-octahydro-4a,8a-trans-1H-pyrrolo[2,3-g]isochinolin-4-thion, Schmp. 194–196° (Zers.) zu ergeben.

Beispiel 21

Herstellung von

3-Äthyl-2-methyl-4,4a,5,6,7,8,8a,9-octahydro-1H-pyrrolo[2,3-g]isochinolin-4-thion

Ein Gemisch von 1,45 g (6 mMol) 3-Äthyl-2-methyl-4,4a,5,6,7,8,8a,9-octahydro-1H-pyrrolo[2,3-g]isochinolin-4-on und 1,77 g (4 mMol) Phosphorpentasulfid in 60 ml Dioxan wurde 10 h gerührt und rückflussgekocht. Das Gemisch wurde gekühlt, und die Dioxanlösung wurde von einem dunklen Rückstand dekantiert, der in 75 ml Wasser gelöst wurde. Die Lösung wurde (auf pH 8 bis 9) mit Ammoniumhydroxid alkalisch gemacht, und die wässrige Mischung wurde mit Chloroform extrahiert. Die Extrakte

Tabelle III

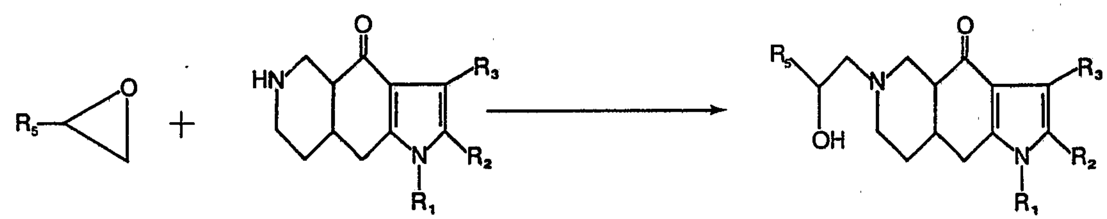

| Beispiel | $R_1$ | $R_2$ | $R_3$ | $R_5$ | Analyse ber. | Analyse gef. | Schmp. | kristallisiert aus | Reaktionsbedingungen Temp. | Zeit |
|---|---|---|---|---|---|---|---|---|---|---|
| 17a<br>2-Methyl-3-äthyl-6-[2-hydroxy-2-(4-chlorphenyl)-äthyl]-4,4a,5,6,7,8a,9-octahydro-1H-pyrrolo[2,3-g]-isochinolin-4-on | H | $CH_3$ | $CH_2CH_3$ | 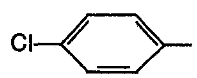 | C 68,29<br>H 7,03<br>N 7,24<br>Cl 9,16 | C 68,11<br>H 7,03<br>N 7,18<br>Cl 9,38 | 214–5° | Äthanol-Äthyl-acetat | Rückfluss | 3 h in Methanol |
| 17b<br>2-Methyl-3-äthyl-6-(2-hydroxyäthyl)-4,4a,5,6,7,8,8a,9-octa-hydro-1H-pyrrolo-[2,3-g]isochi-nolin-4-on | H | $CH_3$ | $CH_2CH_3$ | H | C 69,53<br>H 8,75<br>N 10,14 | C 68,98<br>H 8,78<br>N 9,96 | 215–8° | Äthanol | 25° | 2 h in Me-thanol |
| 17c<br>rac. 2-Methyl-3-äthyl-6-[2-hydroxy-3-[4-(1,1-dimethyl-äthyl)phenoxy]propyl]-4,4a,5,6,-7,8,8a,9-octahydro-4a, 8a-trans-III-pyrrolo[2,3-g]-isochinolin-4-on | H | $CH_3$ | $CH_2CH_3$ | 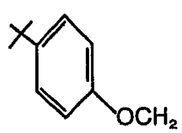 | C 73,94<br>H 8,73<br>N 6,39 | C 73,83<br>H 8,84<br>N 6,30 | 225–7° | Äthyl-acetat-Äthanol | Rückfluss | 2 h in Me-thanol |
| 17d<br>rac. 2-Methyl-3-äthyl-6-(2-hydroxy-3-methylbutyl)-4,4a,5,6,7,8,8a,9-octahydro-4a,8a-trans-III-pyrrolo-[2,3-g]isochinolin-4-on | H | $CH_3$ | $CH_2CH_3$ | 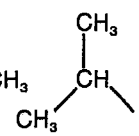 | C 71,66<br>H 9,50<br>N 8,80 | C 71,49<br>H 9,72<br>N 8,77 | 197–9° | Äthanol-Äthyl-acetat | Rückfluss | 5 h in Äthanol |

Tabelle III (Fortsetzung)

| Beispiel | $R_1$ | $R_2$ | $R_3$ | $R_5$ | Analyse ber. | Analyse gef. | Schmp. | kristallisiert aus | Reaktionsbedingungen Temp. | Reaktionsbedingungen Zeit |
|---|---|---|---|---|---|---|---|---|---|---|
| 17e<br>rac. 2-Methyl-3-äthyl-6-(2-hydroxy-3,3-dimethylbutyl)-4,4a,5,6,7,8,8a,9-octahydro-4a,8a-trans-1H-pyrrolo-[2,3-g]isochinolin-4-on | H | $CH_3$ | $CH_2CH_3$ | 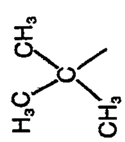 | C 72,25<br>N 9,70<br>H 8,43 | C 72,31<br>H 9,73<br>N 8,20 | 232–4° | Äthanol-Äthylacetat | Rückfluss | über Nacht in Methanol |
| 17f<br>rac. 2-Methyl-3-äthyl-6-[2-hydroxy-3-(4-chlorphenoxy)-propyl]-4,4a,5,6,7,8,8a,9-octahydro-4a,8a-trans-1H-pyrrolo[2,3-g]isochinolin-4-on | H | $CH_3$ | $CH_2CH_3$ | 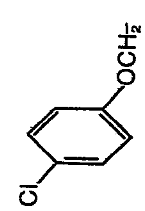 | C 66,26<br>H 7,01<br>N 6,72<br>Cl 8,50 | C 66,11<br>H 7,05<br>N 6,81<br>Cl 8,26 | 211–13° | Äthanol | Rückfluss | 3 h in Äthanol |

wurden mit Wasser gewaschen und über Natriumsulfat getrocknet. Verdampfen des Lösungsmittels ergab 420 mg rohes Thion, das (Trockensäule, Kieselgel) zusammen mit 100 mg zusätzlichem Rohprodukt, erhalten durch Heisswasserbehandlung der Rückstände aus der ursprünglichen Isolierung, gefolgt von der gleichen Chloroformextraktion, chromatographiert wurde. Elution der trockenen Säule mit der organischen Phase eines durch Gleichgewichtseinstellung von 90 (Volumen-)Teilen Chloroform, 30 Teilen Methanol, 10 Teilen Wasser und 6 Teilen Essigsäure hergestellten Gemisches ergab gereinigtes Thion nach Verdampfen, Lösen in Wasser, Neutralisieren mit Ammoniumhydroxid auf pH 8 bis 9 und Extraktion mit Chloroform. Nach dem Waschen mit Wasser und Trocknen über Natriumsulfat lieferte das Verdampfen des Chloroforms 250 mg
3-Äthyl-2-methyl-4,4a,5,6,7,8,8a,9-octahydro-1H-pyrrolo[2,3-g]isochinolin-4-thion
als gelben Feststoff, Schmp. 190–194°. Umkristallisation aus Acetonitril lieferte reines 4a,8a-trans-Isomer, Schmp. 203–205°.

Beispiel 22

Herstellung von
3-Äthyl-2-methyl-6-(2-phenyläthyl)-4,4a,5,6,7,8,-8a,9-octahydro-4a,8a-trans-1H-pyrrolo[2,3-g]-isochinolin-4-thion

Ein Gemisch von 248 mg (1 mMol)
3-Äthyl-2-methyl-4,4a,5,6,7,8,8a,9-octahydro-1H-pyrrolo[2,3-g]isochinolin-4-thion,
276 mg Kaliumcarbonat und 222 mg 2-Bromäthylbenzol in 15 ml 3-Pentanon wurde 3 h gerührt und rückflussgekocht. Das Lösungsmittel wurde am Rotationsverdampfer entfernt, 25 ml Wasser wurden zugesetzt, und das Gemisch wurde mit Chloroform extrahiert. Reinigen durch Trockensäulenchromatographie, wie in Beispiel 21 im einzelnen angegeben, lieferte 100 mg gereinigtes Produkt, das 50 mg
3-Äthyl-2-methyl-6-(2-phenyläthyl)-4,4a,5,6,7,8,8a,9-octahydro-4a,8a-trans-1H-pyrrolo[2,3-g]isochinolin-4-thion,
Schmp. 164–166° (Zers.) nach Umkristallisieren aus Acetonitril lieferte.

Beispiel 23
Kapsel-Zusammensetzung

| Bestandteile | mg/Kapsel 0,5 | 5,0 | 10,0 |
|---|---|---|---|
| 3-Äthyl-2,6-dimethyl-4,4a5,6,7,8,8a,9-octa-hydro-4a,8a-trans-1H-pyrrolo[2,3-g]isochinolin-4-on, Hydrochlorid | 0,5 | 5,0 | 10,0 |
| Lactose | 183,5 | 179,0 | 218,0 |
| Stärke | 30,0 | 30,0 | 50,0 |
| Talcum | 5,0 | 5,0 | 10,0 |
| Magnesiumstearat | 1,0 | 1,0 | 2,0 |
| insgesamt | 220 mg | 220 mg | 290 mg |

Arbeitsweise: Der aktive Bestandteil, Lactose und Stärke werden in einem geeigneten Mischer gemischt. Dann wird in einer geeigneten Mühle vermahlen. Schliesslich wird mit Talcum und Magnesiumstearat gemischt und auf einer Kapselmaschine gefüllt.

Beispiel 24
Tabletten-Zusammensetzung (Direktkomprimieren)

| Bestandteile | mg/Tablette 0,5 | 5,0 | 10,0 |
|---|---|---|---|
| 3-Äthyl-2,6-dimethyl-4,4a,5,6,7,8,8a,9-octa-hydro-4a,8a-trans-1H-pyrrolo[2,3-g]isochinolin-4-on, Hydrochlorid | 0,5 | 5,0 | 10,0 |
| Lactose | 85,5 | 81,0 | 103,0 |
| mikrokristalline Cellulose (Avicel) | 30,0 | 30,0 | 45,0 |
| modifizierte Stärke | 7,5 | 7,5 | 10,0 |
| Magnesiumstearat | 1,5 | 1,5 | 2,0 |
| insgesamt | 125 mg | 125 mg | 170 mg |

Arbeitsweise: Der aktive Bestandteil, Lactose, mikrokristalline Cellulose (Avicel) und modifizierte Stärke werden in einem geeigneten Mischer 10 bis 15 min gemischt. Magnesiumstearat wird als Vorgemisch zugesetzt und 4 min gemischt. Dann wird auf geeigneter Presse komprimiert.

Beispiel 25

Tabletten-Zusammensetzung (Nassgranulieren)

| Bestandteile | mg/Tablette 0,5 | 5,0 | 10,0 |
|---|---|---|---|
| 3-Äthyl-2,6-dimethyl-4,4a,5,6,7,8,8a,9-octa-hydro-4a,8a-trans-1H-pyrrolo[2,3-g]isochinolin-4-on, Hydrochlorid | 0,5 | 5,0 | 10,0 |
| Lactose | 103,5 | 99,0 | 148,0 |
| modifizierte Stärke | 10,0 | 10,0 | 20,0 |
| vorgelatinierte Stärke | 10,0 | 10,0 | 20,0 |
| Magnesiumstearat | 1,0 | 1,0 | 2,0 |
| insgesamt | 125 mg | 125 mg | 200 mg |

Arbeitsweise: Der aktive Bestandteil, Lactose, modifizierte und vorgelatinierte Stärke werden in einem geeigneten Mischer gemischt, granuliert wird mit Wasser. Dann wird getrocknet und vermahlen. Schliesslich wird mit Magnesiumstearat gemischt und auf einer geeigneten Presse komprimiert.

**Patentansprüche für die Vertragsstaaten: BE, CH, DE, FR, GB, IT, LU, NL, SE**

1. Octahydro-1H-pyrrolo[2,3-g]isochinolin der allgemeinen Formel

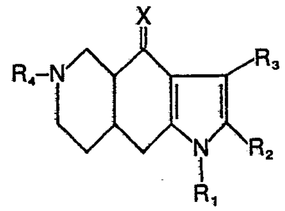

A

worin $R_1$ Wasserstoff, $(C_1–C_7)$-Alkyl, $(C_1–C_7)$-Alkanoyl oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Benzoyl oder Phenyl-$(C_1–C_7)$-alkyl, $R_2$ und $R_3$ unabhängig voneinander Wasserstoff, $(C_1–C_7)$-Alkyl, $(C_3–C_6)$-Cycloalkyl, $(C_2–C_7)$-Alkenyl,$(C_1–C_7)$-Alkanoyl oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Benzoyl, Phenyl oder Phenyl-$(C_1–C_7)$-alkyl und $R_4$ Wasserstoff,
$(C_1–C_7)$-Alkyl,
Hydroxy-$(C_1–C_7)$-alkyl,
Phenyl-hydroxy-$(C_1–C_7)$-alkyl,
Halogenphenyl-hydroxy-$(C_1–C_7)$-alkyl,
$(C_1–C_7)$-Alkylphenyl-hydroxy-$(C_1–C_7)$-alkyl,
$(C_1–C_7)$-Alkoxyphenyl-hydroxy-$(C_1–C_7)$-alkyl,
$(C_1–C_7)$-Alkoxy-$(C_1–C_7)$-alkyl,
$(C_1–C_7)$-Alkoxy-hydroxy-$(C_1–C_7)$-alkyl,
$(C_1–C_7)$-Alkanoyloxy-$(C_1–C_7)$-alkyl,
$(C_1–C_7)$-Alkoxycarbonyl-$(C_1–C_7)$-alkyl,
$(C_2–C_7)$-Alkenyl, $(C_3–C_6)$-Cycloalkyl-$C_1–C_7)$-alkyl,
$(C_2–C_7)$-Alkinyl, Thienyl-$(C_1–C_7)$-alkyl,
Furyl-$(C_1–C_7)$-alkyl,
$(C_1–C_7)$-Alkanoyl-$(C_1–C_7)$-alkyl,
$(C_3–C_6)$-Cycloalkoxy-$(C_1–C_7)$-alkyl,
$(C_3–C_6)$-Cycloalkylhydroxy-$(C_1–C_7)$-alkyl,
$(C_2–C_7)$-Alkenyloxy-$(C_1–C_7)$-alkyl,
N-$(C_1–C_7)$-Alkyl-pyrrolidinyl-$(C_1–C_7)$-alkyl,
Trifluoralkyl mit 2 bis 6 Kohlenstoffatomen oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes
Phenoxy-hydroxy-$(C_1–C_7)$-alkyl,
Benzoyloxy-$(C_1–C_7)$-alkyl,
Benzoyl-$(C_1–C_7)$-alkyl, Phenyl-$(C_1–C_7)$-alkyl,
Phenylcarboxamido-$(C_1–C_7)$-alkyl,
Phenyl-$(C_2–C_7)$-alkenyl,
Phenoxy-$(C_1–C_7)$-alkyl oder

Phenyl-N-imidazolonyl-$(C_1–C_7)$-alkyl oder 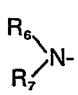

$(C_1–C_7)$-Alkyl sind, worin $R_6$ und $R_7$ unabhängig voneinander Wasserstoff oder $(C_1–C_7)$-Alkyl oder zusammen mit dem Stickstoffatom ein 5- oder 6gliedriger gesättigter heterocyclischer Ring, der als Ringglied noch ein Sauerstoffatom oder ein gegebenenfalls durch $(C_1–C_7)$-Alkyl oder Hydroxy-$(C_1–C_7)$-alkyl substituiertes Stickstoffatom enthalten kann, sind und X O oder S ist, dessen optisches oder geometrisches Isomer oder dessen pharmazeutisch annehmbares Säureadditionssalz.

2. Verbindung nach Anspruch 1, worin $R_1$ Wasserstoff, $(C_1–C_7)$-Alkyl, $(C_1–C_7)$-Alkanoyl oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Benzoyl oder Phenyl-$(C_1–C_7)$-alkyl, $R_2$ und $R_3$ unabhängig voneinander Wasserstoff, $(C_1–C_7)$-Alkyl, $(C_3–C_6)$-Cycloalkyl, $(C_2–C_7)$-Alkenyl oder gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Phenyl-$(C_1–C_7)$-alkyl und $R_4$ Wasserstoff,
$(C_1–C_7)$-Alkyl, Hydroxy-$(C_1–C_7)$-alkyl,
Phenyl-hydroxy-$(C_1–C_7)$-alkyl,
Halogenphenyl-hydroxy-$(C_1–C_7)$-alkyl,
$(C_1–C_7)$-Alkylphenyl-hydroxy-$(C_1–C_7)$-alkyl,
$(C_1–C_7)$-Alkoxyphenyl-hydroxy-$(C_1–C_7)$-alkyl,
$(C_1–C_7)$-Alkoxy-$(C_1–C_7)$-alkyl,
$(C_1–C_7)$-Alkoxy-hydroxy-$(C_1–C_7)$-alkyl,
$(C_1–C_7)$-Alkanoyloxy-$(C_1–C_7)$-alkyl,
$(C_1–C_7)$-Alkoxycarbonyl-$(C_1–C_7)$-alkyl,
$(C_2–C_7)$-Alkenyl,
$(C_3–C_6)$-Cycloalkyl-$(C_1–C_7)$-alkyl, $(C_2–C_7)$-Alkinyl,
Thienyl-$(C_1–C_7)$-alkyl, Furyl-$(C_1–C_7)$-alkyl
oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Phenoxy-hydroxy-$(C_1–C_7)$-alkyl, Benzoyloxy-$(C_1–C_7)$-alkyl, Phenyl-$(C_1–C_7)$-alkyl oder Benzoyl-$(C_1–C_7)$-alkyl oder

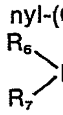N-$(C_1–C_7)$-Alkyl und X O sind, deren optisches oder geometrisches Isomer oder deren pharmazeutisch annehmbares Säureadditionssalz.

3. Verbindung nach Anspruch 1 oder 2, worin $R_1$ Wasserstoff ist.

4. Verbindung nach einem der Ansprüche 1 bis 3, worin $R_2$ und $R_3$ $(C_1–C_7)$-Alkyl sind.

5. Verbindung nach einem der Ansprüche 1 bis 4, worin
$R_4$ $(C_1–C_7)$-Alkyl, Hydroxy-$(C_1–C_7)$-alkyl,
Phenyl-hydroxy-$(C_1–C_7)$-alkyl,
Halogenphenyl-hydroxy-$(C_1–C_7)$-alkyl,
$(C_1–C_7)$-Alkoxy-$(C_1–C_7)$-alkyl
oder jeweils gegebenenfalls am Phenylring durch Halogen, Trifluormethyl, $(C_1–C_7)$-alkyl, $(C_1–C_7)$-Alkoxy, Nitro, Amino, $(C_1–C_7)$-Alkylamino oder Di-$(C_1–C_7)$-alkylamino substituiertes Phenoxy-$(C_1–C_7)$-alkyl, Benzoyl-$(C_1–C_7)$-alkyl oder Phenyl-$(C_1–C_7)$-alkyl ist.

6. Verbindung nach einem der Ansprüche 1 und 3 bis 5, worin X O ist.

7. Verbindung nach einem der Ansprüche 1 bis 6, die das trans-Isomere ist.

8. 3-Äthyl-2,6-dimethyl-4,4a,5,6,7,8,8a,9-octahydro-4a,8a-trans-1H-pyrrolo[2,3-g]isochinolin-4-on.

9. 2-Methyl-3-äthyl-6-(2-phenyläthyl)-4,4a,5,-6,7,-8,8a,9-octahydro-4a,8a-trans-1H-pyrrolo[2,3-g]isochinolin-4-on.

10. 2-Methyl-3-äthyl-6-(2-äthoxyäthyl)-4,4a,5,6,-7,8,8a,9-octahydro-4a,8a-trans-1H-pyrr-olo[2,3-g]isochinolin-4-on.

11. 2-Methyl-3-äthyl-6-[4-(4-fluorphenyl)-4-oxobutyl]-4,4a,5,6,7,8,8a,9-octahydro-4a,8a-trans-1H-pyrrolo[2,3-g]isochinolin-4-on.

12. 3-Äthyl-2-methyl-6-(2-hydroxy-3,3-dimethylbutyl)-4,4a,5,6,7,8,8a,9-octahydro-1H-pyrrolo-[2,3-g]isochinolin-4-on.

13. Verbindung gemäss Anspruch 1, worin $R_1$ Wasserstoff, $R_2$ Methyl, $R_3$ Äthyl, X O und $R_4$ 2-Hydroxy-2-phenyläthyl, Benzyl, 3-(4-Fluorphenyl)-3-oxopropyl, 3-Phenoxypropyl, 2-Hydroxy-3-methylbutyl, 2-Hydroxyäthyl, 2-Propenyl, Cyclopropylmethyl, Äthyl, 2-Oxo-2-(4-fluorphenyl)-äthyl sind.

14. Verbindung gemäss Anspruch 1, worin $R_1$ Wasserstoff, $R_2$ und $R_3$ Methyl, X O und $R_4$ Methyl, 2-Phenyläthyl, 4-(4-Fluorphenyl)-4-oxobutyl sind.

15. Ein Octahydro-1H-pyrrolo[2,3-g]isochinolin gemäss einem der Ansprüche 1 bis 14 als pharmazeutischer Wirkstoff.

16. Ein Octahydro-1H-pyrrolo[2,3-g]isochinolin gemäss einem der Ansprüche 1 bis 14 als neuroleptischer/antipsychotischer Wirkstoff.

17. 3-Äthyl-2,6-dimethyl-4,4a,5,6,7,8,8a,9-octahydro-4a,8a-trans-1H-pyrrolo[2,3-g]isochinolin-4-on als pharmazeutischer Wirkstoff.

18. 3-Äthyl-2,6-dimethyl-4,4a,5,6,7,8,8a,9-octahydro-4a,8a-trans-1H-pyrrolo[2,3-g]isochinolin-4-on als neuroleptischer/antipsychotischer Wirkstoff.

19. Verfahren zur Herstellung von Verbindungen der Formel A in Anspruch 1, von deren optischen und geometrischen Isomeren und von deren pharmazeutisch annehmbaren Säureadditionssalzen, dadurch gekennzeichnet, dass man

a) zur Herstellung einer Verbindung der allgemeinen Formel

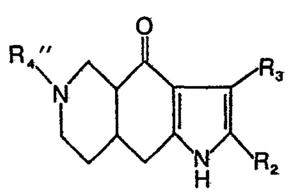

Ia,

worin $R_4''$ $(C_1-C_7)$-alkyl, $(C_1-C_7)$-alkoxy-$(C_1-C_7)$-alkyl oder $(C_3-C_6)$-Cycloalkyl-$(C_1-C_7)$-alkyl und $R_2$ und $R_3$ wie zuvor beschrieben sind,
eine Verbindung der allgemeinen Formel

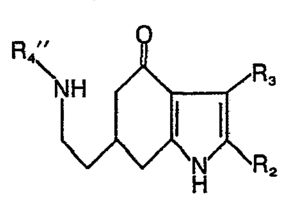

IX,

worin $R_2$, $R_3$ und $R_4''$ wie zuvor beschrieben sind, mit Formaldehyd behandelt, oder

b) zur Herstellung einer Verbindung der obigen Formel Ia eine Verbindung der allgemeinen Formel

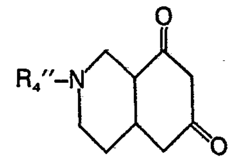

XII,

worin $R_4''$ wie zuvor beschrieben ist,
mit einer Verbindung der allgemeinen Formel

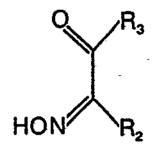

VII

in Gegenwart eines Reduktionsmittels, oder mit einer Verbindung der allgemeinen Formel

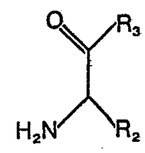

VIII,

worin $R_2$ und $R_3$ wie zuvor beschrieben sind, oder einer Vorstufe von dieser, behandelt, oder

c) zur Herstellung einer Verbindung der allgemeinen Formel

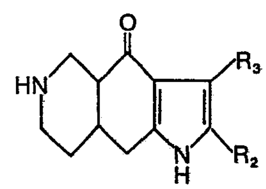

Ib

worin $R_2$ und $R_3$ wie zuvor beschrieben sind,
eine Verbindung der obigen Formel Ia, worin $R_4''$ Methyl ist, N-demethyliert, oder

d) zur Herstellung einer Verbindung der allgemeinen Formel

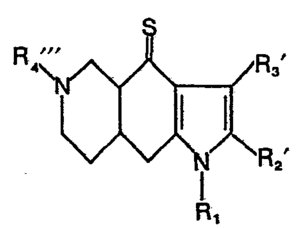

IIc'',

worin $R_2'$ und $R_3'$ unabhängig voneinander Wasserstoff, $(C_1-C_7)$-Alkyl, $(C_3-C_6)$-Cycloalkyl, $(C_2-C_7)$-Alkenyl oder jeweils gegebenenfalls am Phenylring durch Halogen, Trifluormethyl, $(C_1-C_7)$-Alkyl, $(C_1-C_7)$-Alkoxy, Nitro, Amino, $(C_1-C_7)$-Alkylamino oder Di-$(C_1-C_7)$-alkylamino substituiertes Phenyl oder Phenyl-$(C_1-C_7)$-alkyl, $R_4'''$ Wasserstoff,

($C_1$–$C_7$)-Alkyl, ($C_1$–$C_7$)-Alkoxy-($C_1$–$C_7$)-alkyl, ($C_2$–$C_7$)-Alkenyl, ($C_3$–$C_6$)-Cycloalkyl-($C_1$–$C_7$)-alkyl, ($C_2$–$C_7$)-Alkinyl, Thienyl-($C_1$–$C_7$)-alkyl, Furyl-($C_1$–$C_7$)-alkyl, ($C_2$–$C_7$)-Alkenyloxy-($C_1$–$C_7$)-alkyl oder jeweils gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$-Alkylamino oder Di-($C_1$–$C_7$)-alkylamino substituiertes Phenyl-($C_1$–$C_7$)-alkyl, Phenyl-($C_2$–$C_7$)-alkenyl oder Phenoxy-($C_1$–$C_7$)-alkyl oder Trifluoralkyl mit 2 bis 6 Kohlenstoffatomen und $R_1$ wie zuvor beschrieben sind, eine Verbindung der allgemeinen Formel

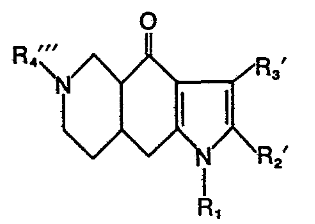

Ic″,

worin $R_1$, $R_2'$, $R_3'$ und $R_4'''$ wie zuvor beschrieben sind, mit Phosphorpentasulfid behandelt, oder

e) zur Herstellung einer Verbindung der allgemeinen Formel

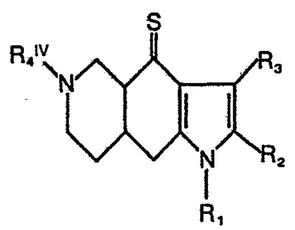

IIc‴,

worin $R_4^{IV}$ Hydroxy-($C_1$–$C_7$)-alkyl, Phenyl-hydroxy-($C_1$–$C_7$)-alkyl, Halogenphenyl-hydroxy-($C_1$–$C_7$)-alkyl, ($C_1$–$C_7$)-Alkyl-phenyl-hydroxy-($C_1$–$C_7$)-alkyl, ($C_1$–$C_7$)-Alkoxyphenyl-hydroxy-($C_1$–$C_7$)-alkyl, ($C_1$–$C_7$)-Alkoxy-hydroxy-($C_1$–$C_7$)-alkyl, ($C_1$–$C_7$)-Alkanoyloxy-($C_1$–$C_7$)-alkyl, ($C_1$–$C_7$)-Alkoxycarbonyl-($C_1$–$C_7$)-alkyl, ($C_1$–$C_7$)-Alkanoyl-($C_1$–$C_7$)-alkyl, ($C_3$–$C_6$)-Cycloalkoxy-($C_1$–$C_7$)-alkyl, ($C_3$–$C_6$)-Cycloalkyl-hydroxy-($C_1$–$C_7$)-alkyl, N-($C_1$–$C_7$)-Alkyl-pyrrolidinyl-($C_1$–$C_7$)-alkyl oder jeweils gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Phenyl-N-imidazolonyl-($C_1$–$C_7$)-alkyl, Phenoxy-hydroxy-($C_1$–$C_7$)-alkyl, Benzoyloxy-($C_1$–$C_7$)-alkyl, Benzoyl-($C_1$–$C_7$)-alkyl oder Phenylcarboxamido-($C_1$–$C_7$)-alkyl oder

$R_6$, $R_7$ >N-($C_1$–$C_7$-Alkyl bedeutet und $R_1$, $R_2$, $R_3$, $R_6$ und $R_7$ wie zuvor beschrieben sind,

die Schutzgruppe oder -gruppen in einer Verbindung der allgemeinen Formel

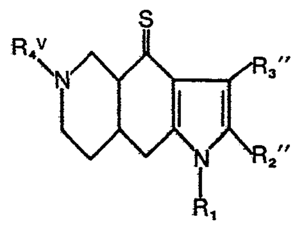

IIf,

worin $R_2''$ und $R_3''$ die gleiche Bedeutung wie $R_2$ und $R_3$ haben, ausgenommen wenn $R_2$ und $R_3$ ($C_1$–$C_7$)-Alkanoyl oder gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Benzoyl bedeuten, sind $R_2''$ und $R_3''$ in geschützter Form, $R_4^V$ die gleiche Bedeutung wie $R_4^{IV}$ hat, aber in geschützter Form, und $R_1$ wie zuvor beschrieben ist, abspaltet, oder

f) zur Herstellung einer Verbindung der allgemeinen Formel

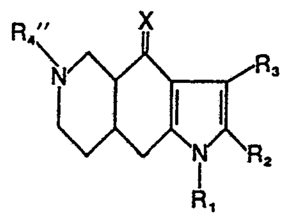

. Ac,

worin $R_1'$ ($C_1$–$C_7$)-Alkyl, ($C_1$–$C_7$)-Alkanoyl oder jeweils gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Benzoyl oder Phenyl-($C_1$–$C_7$)-alkyl und $R_2$, $R_3$, $R_4''$ und X wie zuvor beschrieben sind,

eine Verbindung der allgemeinen Formel

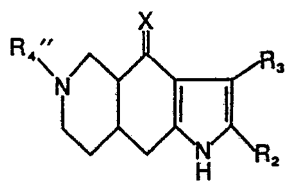

Aa,

worin $R_2$, $R_3$, $R_4''$ und X wie zuvor beschrieben sind, am Pyrrol-Stickstoffatom substituiert, oder

g) zur Herstellung einer Verbindung der allgemeinen Formel

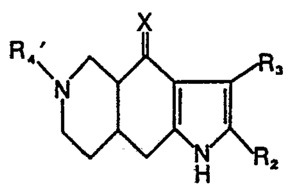

Ad,

worin $R_4'$

($C_1$–$C_7$)-Alkyl, Hydroxy-($C_1$–$C_7$)-alkyl, Phenyl-hydroxy-($C_1$–$C_7$)-alkyl, Halogenphenyl-hydroxy-($C_1$–$C_7$)-alkyl, ($C_1$–$C_7$)-Alkylphenyl-hydroxy-($C_1C_7$)-alkyl,

$(C_1–C_7)$-Alkoxyphenyl-hydroxy-$(C_1–C_7)$-alkyl, $(C_1–C_7)$-Alkoxy-$(C_1–C_7)$-alkyl, $(C_1–C_7)$-Alkoxy-hydroxy-$(C_1–C_7)$-alkyl, $(C_1–C_7)$-Alkanoyloxy-$(C_1–C_7)$-alkyl, $(C_1–C_7)$-Alkoxycarbonyl-$(C_1–C_7)$-alkyl, $(C_2–C_7)$-Alkenyl, $(C_3–C_6)$-Cycloalkyl-$(C_1–C_7)$-alkyl, $(C_2–C_7)$-Alkinyl, Thienyl-$(C_1–C_7)$-alkyl, Furyl-$(C_1–C_7)$-alkyl, $(C_1–C_7)$-Alkanoyl-$(C_1–C_7)$-alkyl, $(C_3–C_6)$-Cycloalkoxy-$(C_1–C_7)$-alkyl, $(C_3–C_6)$-Cycloalkyl-hydroxy-$(C_1–C_7)$-alkyl, $(C_2–C_7)$-Alkenyloxy-$(C_1–C_7)$-alkyl, N-$(C_1–C_7)$-Alkyl-pyrrolidinyl-$(C_1–C_7)$-alkyl, Trifluoralkyl mit 2 bis 6 Kohlenstoffatomen oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Phenoxy-hydroxy-$(C_1–C_7)$-alkyl, Benzoyloxy-$(C_1–C_7)$-alkyl, Benzoyl-$(C_1–C_7)$-alkyl, Phenyl-$(C_1–C_7)$-alkyl, Phenylcarboxamido-$(C_1–C_7)$-alkyl, Phenyl-$(C_2–C_7)$-alkenyl, Phenoxy-$(C_1–C_7)$-alkyl oder Phenyl-N-imidazolonyl-$(C_1–C_7)$-alkyl oder 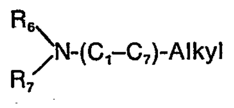

bedeutet und $R_2$, $R_3$, $R_6$, $R_7$ und X wie zuvor beschrieben sind,
eine Verbindung der allgemeinen Formel

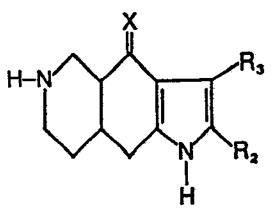

Ab,

worin $R_2$, $R_3$ und X wie zuvor beschrieben sind, am Isochinolin-Stickstoffatom substituiert, oder

h) zur Herstellung einer Verbindung der allgemeinen Formel

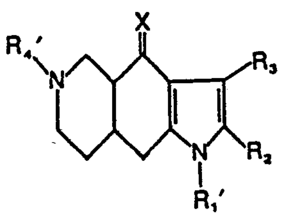

Af,

worin $R_1'$, $R_2$, $R_3$, $R_4'$ und X wie zuvor beschrieben sind,
eine Verbindung der folgenden Formel Ae am Isochinolin-Stickstoffatom substituiert, oder

i) zur Herstellung einer Verbindung der allgemeinen Formel

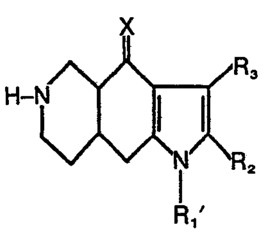

Ae,

worin $R_1'$, $R_2$, $R_3$ und X wie zuvor beschrieben sind,
die Schutzgruppe in einer Verbindung der allgemeinen Formel

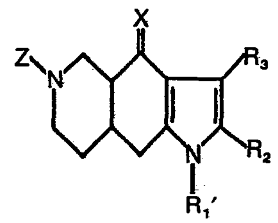

Ah,

worin Z eine Schutzgruppe ist und $R_1'$, $R_2$, $R_3$ und X wie zuvor beschrieben sind, abspaltet, oder

j) zur Herstellung einer Verbindung der allgemeinen Formel

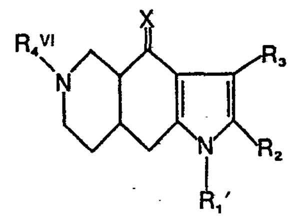

Af',

worin $R_4^{VI}$ Wasserstoff, $(C_1–C_7)$-Alkyl, $(C_1–C_7)$-Alkoxy-$(C_1–C_7)$-alkyl, $(C_1–C_7)$-Alkanoyloxy-$(C_1–C_7)$-alkyl, $(C_1–C_7)$-Alkoxycarbonyl-$(C_1–C_7)$-alkyl, $(C_2–C_7)$-Alkenyl, $(C_3–C_6)$-Cycloalkyl-$(C_1–C_7)$-alkyl, $(C_2–C_7)$-Alkinyl, Thienyl-$(C_1–C_7)$-alkyl, Furyl-$(C_1–C_7)$-alkyl, $(C_1–C_7)$-Alkanoyl-$(C_1–C_7)$-alkyl, $(C_3–C_6)$-Cycloalkyloxy-$(C_1–C_7)$-alkyl, $(C_2–C_7)$-Alkenyloxy-$(C_1–C_7)$-alkyl, N-$(C_1–C_7)$-Alkyl-pyrrolidinyl-$(C_1–C_7)$-alkyl, Trifluoralkyl mit 2 bis 6 Kohlenstoffatomen oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Phenyl-N-imidazolonyl-$(C_1–C_7)$-alkyl, Benzoyloxy-$(C_1–C_7)$-alkyl, Phenyl-$(C_1–C_7)$-alkyl, Phenyl-carboxamido-$(C_1–C_7)$-alkyl, Benzoyl-$(C_1–C_7)$-alkyl, Phenyl-$(C_2–C_7)$-alkenyl oder Phenoxy-$(C_1–C_7)$-alkyl bedeutet und $R_1'$, $R_2$, $R_3$ und X wie zuvor beschrieben sind,
eine Verbindung der allgemeinen Formel

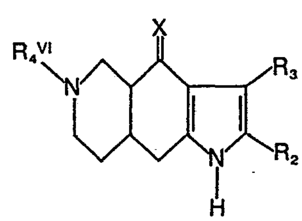

Ad',

worin $R_2$, $R_3$, $R_4^{VI}$ und X wie zuvor beschrieben sind, am Pyrrol-Stickstoffatom substituiert, oder

k) zur Herstellung einer Verbindung der allgemeinen Formel

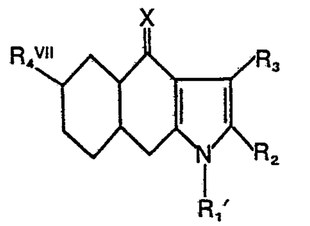

Af''

worin $R_4^{VII}$ Hydroxy-($C_1$–$C_7$)-alkyl, Phenyl-hydroxy-($C_1$–$C_7$)-alkyl, Halogenphenyl-hydroxy-($C_1$–$C_7$)-alkyl, ($C_1$–$C_7$)-Alkyl-phenyl-hydroxy-($C_1$–$C_7$)-alkyl, ($C_1$–$C_7$)-Alkoxyphenyl-hydroxy-($C_1$–$C_7$)-alkyl, ($C_1$–$C_7$)-Alkoxy-hydroxy-($C_1$–$C_7$)-alkyl,

($C_3$–$C_6$)-Cycloalkyl-hydroxy-($C_1$–$C_7$)-alkyl, 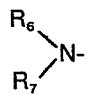

($C_1$–$C_7$)-Alkyl oder gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Phenoxy-hydroxy-($C_1$–$C_7$)-alkyl bedeutet und $R_1'$, $R_2$, $R_3$, $R_6$, $R_7$ und X wie zuvor beschrieben sind, die Schutzgruppe in einer Verbindung der allgemeinen Formel

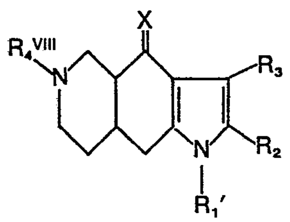

Af'''

worin $R_4^{VIII}$ die gleiche Bedeutung wie $R_4^{VII}$ hat, jedoch in geschützter Form, und $R_1'$, $R_2$, $R_3$ und X wie zuvor beschrieben sind, abspaltet, oder

l) das erhaltene Gemisch der cis- und trans-Isomeren zu einem Endverhältnis, das überwiegend das trans-Isomere aufweist, isomerisiert, oder

m) das trans-Isomere aus dem erhaltenen Gemisch abtrennt, oder

n) ein erhaltenes racemisches Gemisch in die optischen Antipoden aufspaltet, und

o) wenn gewünscht, eine erhaltene Verbindung oder ein pharmazeutisch nicht annehmbares Säureadditionssalz in ein pharmazeutisch annehmbares Säureadditionssalz überführt.

20. Arzneimittel, enthaltend ein Octahydro-1H-pyrrolo[2,3-g]-isochinolin der Formel A in Anspruch 1, ein optisches oder geometrisches Isomeres oder ein pharmazeutisch annehmbares Säureadditionssalz dieser Verbindung.

21. Neuroleptisches/antipsychotisches Arzneimittel, enthaltend ein Octahydro-1H-pyrrolo[2,3-g]isochinolin der Formel A in Anspruch 1, ein optisches oder geometrisches Isomeres oder ein pharmazeutisch annehmbares Säureadditionssalz dieser Verbindung.

22. Arzneimittel, enthaltend
3-Äthyl-2,6-dimethyl-4,4a,5,6,7,8,8a,9-octahydro-4a,8a-trans-1H-pyrrolo[2,3-g]isochinolin-4-on.

23. Neuroleptisches/antipsychotisches Arzneimittel, enthaltend
3-Äthyl-2,6-dimethyl-4,4a,5,6,7,8,8a,9-octahydro-4a,8a-trans-1H-pyrrolo[2,3-g]isochinolin-4-on.

**Patentansprüche für den Vertragsstaat: AT**

1. Verfahren zur Herstellung von Octahydro-1H-pyrrolo(2,3-g]isochinolinen der allgemeinen Formel

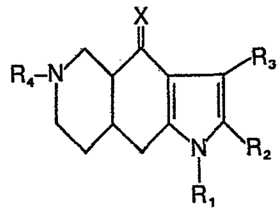

A

worin $R_1$ Wasserstoff, ($C_1$–$C_7$)-Alkyl, ($C_1$–$C_7$)-Alkanoyl oder jeweils gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Benzoyl oder Phenyl-($C_1$–$C_7$)-alkyl, $R_2$ und $R_3$ unabhängig voneinander Wasserstoff, ($C_1$–$C_7$)-Alkyl, ($C_3$–$C_6$)-Cycloalkyl, ($C_2$–$C_7$)-Alkenyl, ($C_1$–$C_7$)-Alkanoyl oder jeweils gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Benzoyl, Phenyl oder Phenyl-($C_1$–$C_7$)-alkyl und $R_4$ Wasserstoff, ($C_1$–$C_7$)-Alkyl, Hydroxy-($C_1$–$C_7$)-alkyl, Phenyl-hydroxy-($C_1$–$C_7$)-alkyl, Halogenphenyl-hydroxy-($C_1$–$C_7$)-alkyl, ($C_1$–$C_7$)-Alkylphenyl-hydroxy-($C_1$–$C_7$)-alkyl, ($C_1$–$C_7$)-Alkoxyphenyl-hydroxy-($C_1$–$C_7$)-alkyl, ($C_1$–$C_7$)-Alkoxy-($C_1$–$C_7$)-alkyl, ($C_1$–$C_7$)-Alkoxy-hydroxy-($C_1$–$C_7$)-alkyl, ($C_1$–$C_7$)-Alkanoyloxy-($C_1$–$C_7$)-alkyl, ($C_1$–$C_7$)-Alkoxycarbonyl-($C_1$–$C_7$)-alkyl, ($C_2$–$C_7$)-Alkenyl, ($C_3$–$C_6$)-Cycloalkyl-($C_1$–$C_7$)-alkyl, ($C_2$–$C_7$)-Alkinyl, Thienyl-($C_1$–$C_7$)-alkyl, Furyl-($C_1$–$C_7$)-alkyl, ($C_1$–$C_7$)-Alkanoyl-($C_1$–$C_7$)-alkyl, ($C_3$–$C_6$)-Cycloalkoxy-($C_1$–$C_7$)-alkyl, ($C_3$–$C_6$)-Cycloalkyl-hydroxy-($C_1$–$C_7$)-alkyl, ($C_2$–$C_7$)-Alkenyloxy-($C_1$–$C_7$)-alkyl, N-($C_1$–$C_7$)-Alkyl-pyrrolidinyl-($C_1$–$C_7$)-alkyl, Trifluoralkyl mit 2 bis 6 Kohlenstoffatomen oder jeweils gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Phenoxy-hydroxy-($C_1$–$C_7$)-alkyl, Benzoyloxy-($C_1$–$C_7$)-alkyl, Benzoyl-($C_1$–$C_7$)-alkyl, Phenyl-($C_1$–$C_7$)-alkyl, Phenylcarboxamido-($C_1$–$C_7$)-alkyl, Phenyl-($C_2$–$C_7$)-alkenyl, Phenoxy-($C_1$–$C_7$)-alkyl oder Phenyl-N-imidazolonyl-($C_1$–$C_7$)-alkyl oder 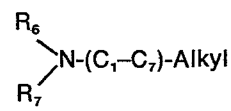

sind, worin $R_6$ und $R_7$ unabhängig voneinander Wasserstoff oder $(C_1–C_7)$-Alkyl oder zusammen mit dem Stickstoffatom ein 5- oder 6gliedriger gesättigter heterocyclischer Ring, der als Ringglied noch ein Sauerstoffatom oder ein gegebenenfalls durch $(C_1–C_7)$-Alkyl oder Hydroxy-$(C_1–C_7)$-alkyl substituiertes Stickstoffatom enthalten kann, sind und X O oder S ist, der optischen und geometrischen Isomeren dieser Verbindungen und deren pharmazeutisch annehmbare Säureadditionssalze, dadurch gekennzeichnet, dass man

a) zur Herstellung einer Verbindung der allgemeinen Formel

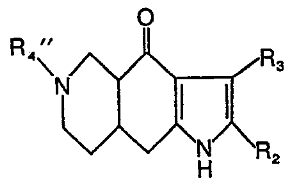

Ia,

worin $R_4''$ $(C_1–C_7)$-Alkyl, $(C_1–C_7)$-Alkoxy-$(C_1–C_7)$-alkyl oder $(C_3–C_6)$-Cycloalkyl-$(C_1–C_7)$-alkyl und $R_2$ und $R_3$ wie zuvor beschrieben sind,
eine Verbindung der allgemeinen Formel

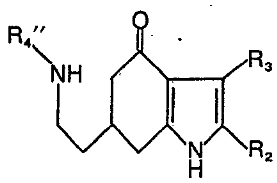

IX,

worin $R_2$, $R_3$ und $R_4''$ wie zuvor beschrieben sind, mit Formaldehyd behandelt, oder

b) zur Herstellung einer Verbindung der obigen Formel Ia eine Verbindung der allgemeinen Formel

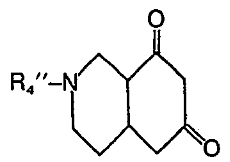

XII,

worin $R_4''$ wie zuvor beschrieben ist,
mit einer Verbindung der allgemeinen Formel

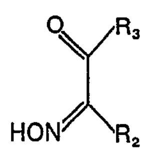

VII

in Gegenwart eines Reduktionsmittels, oder mit einer Verbindung der allgemeinen Formel

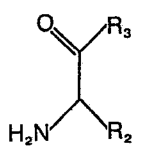

VIII,

worin $R_2$ und $R_3$ wie zuvor beschrieben sind, oder einer Vorstufe von dieser, behandelt, oder

c) zur Herstellung einer Verbindung der allgemeinen Formel

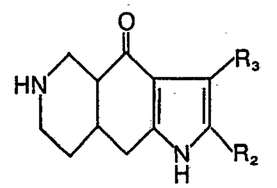

Ib

worin $R_2$ und $R_3$ wie zuvor beschrieben sind, eine Verbindung der obigen Formel Ia, worin $R_4''$ Methyl ist, N-demethyliert, oder

d) zur Herstellung einer Verbindung der allgemeinen Formel

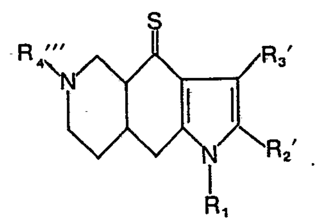

IIc'',

worin $R_2'$ und $R_3'$ unabhängig voneinander Wasserstoff, $(C_1–C_7)$-Alkyl, $(C_3–C_6)$-Cycloalkyl, $(C_2–C_7)$-Alkenyl oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Phenyl oder Phenyl-$(C_1–C_7)$-alkyl, $R_4'''$ Wasserstoff, $(C_1–C_7)$-Alkyl, $(C_1–C_7)$-Alkoxy-$(C_1–C_7)$alkyl, $(C_2–C_7)$-Alkenyl, $(C_3–C_6)$-Cycloalkyl-$(C_1–C_7)$-alkyl, $(C_2–C_7)$-Alkinyl, Thienyl-$(C_1–C_7)$-alkyl, Furyl-$(C_1–C_7)$-alkyl, $(C_2–C_7)$-Alkenyloxy-$(C_1–C_7)$-alkyl oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Phenyl-$(C_1–C_7)$-alkyl, Phenyl-$(C_2–C_7)$-alkenyl oder Phenoxy-$(C_1–C_7)$-alkyl oder Trifluoralkyl mit 2 bis 6 Kohlenstoffatomen und $R_1$ wie zuvor beschrieben sind, eine Verbindung der allgemeinen Formel

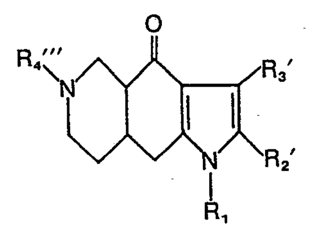

Ic'',

worin $R_1$, $R_2'$, $R_3'$ und $R_4'''$ wie zuvor beschrieben sind, mit Phosphorpentasulfid behandelt, oder

e) zur Herstellung einer Verbindung der allgemeinen Formel

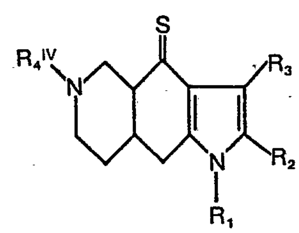

IIc''',

worin $R_4^{IV}$
Hydroxy-($C_1$–$C_7$)-alkyl,
Phenyl-hydroxy-($C_1$–$C_7$)-alkyl,
Halogenphenyl-hydroxy-($C_1$–$C_7$)-alkyl,
($C_1$–$C_7$)-Alkyl-phenyl-hydroxy-($C_1$–$C_7$)-alkyl,
($C_1$–$C_7$)-Alkoxyphenyl-hydroxy-($C_1$–$C_7$)-alkyl,
($C_1$–$C_7$)-Alkoxy-hydroxy-($C_1$–$C_7$)-alkyl,
($C_1$–$C_7$)-Alkanoyloxy-($C_1$–$C_7$)-alkyl,
($C_1$–$C_7$)-Alkoxycarbonyl-($C_1$–$C_7$)-alkyl,
($C_1$–$C_7$)-Alkanoyl-($C_1$–$C_7$)-alkyl,
($C_3$–$C_6$)-Cycloalkoxy-($C_1$–$C_7$)-alkyl,
($C_3$–$C_6$)-Cycloalkyl-hydroxy-($C_1$–$C_7$)-alkyl,
N-($C_1$–$C_7$)-Alkyl-pyrrolidinyl-($C_1$–$C_7$)-alkyl
oder jeweils gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$-alkylamino substituiertes Phenyl-N-imidazolonyl-($C_1$–$C_7$)-alkyl, Phenoxy-hydroxy-($C_1$–$C_7$)-alkyl, Benzoyloxy-($C_1$–$C_7$)-alkyl, Benzoyl-($C_1$–$C_7$)-alkyl oder Phenylcarboxamido-($C_1$–$C_7$)-alkyl oder

$R_6$, $R_7$ >N($C_1$–$C_7$)-Alkyl bedeutet und $R_1$, $R_2$, $R_3$, $R_6$ und $R_7$ wie zuvor beschrieben sind,
die Schutzgruppe oder -gruppen in einer Verbindung der allgemeinen Formel

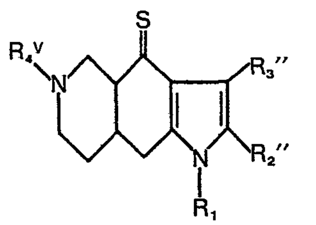

IIf,

worin $R_2''$ und $R_3''$ die gleiche Bedeutung wie $R_2$ und $R_3$ haben, ausgenommen wenn $R_2$ und $R_3$ ($C_1$–$C_7$)-Alkanoyl oder gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Benzoyl bedeuten, sind $R_2''$ und $R_3''$ in geschützter Form, $R_4^{V}$ die gleiche Bedeutung wie $R_4^{IV}$ hat, aber in geschützter Form, und $R_1$ wie zuvor beschrieben ist, abspaltet oder

f) zur Herstellung einer Verbindung der allgemeinen Formel

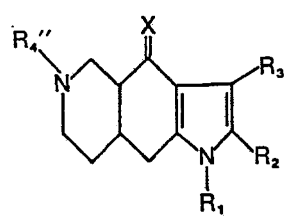

. Ac,

worin $R_1'$ ($C_1$–$C_7$)-Alkyl, ($C_1$–$C_7$)-Alkanoyl oder jeweils gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Benzoyl oder Phenyl-($C_1$–$C_7$)-alkyl und $R_2$, $R_3$, $R_4''$ und X wie zuvor beschrieben sind,

eine Verbindung der allgemeinen Formel

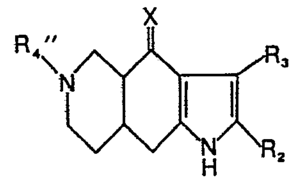

Aa,

worin $R_2$, $R_3$, $R_4''$ und X wie zuvor beschrieben sind, am Pyrrol-Stickstoffatom substituiert, oder

g) zur Herstellung einer Verbindung der allgemeinen Formel

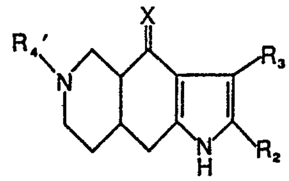

Ad,

worin $R_4'$
($C_1$–$C_7$)-Alkyl, Hydroxy-($C_1$–$C_7$)-alkyl,
Phenyl-hydroxy-($C_1$–$C_7$)-alkyl,
Halogenphenyl-hydroxy-($C_1$–$C_7$)-alkyl,
($C_1$–$C_7$)-Alkylphenyl-hydroxy-($C_1$–$C_7$)-alkyl,
($C_1$–$C_7$)-Alkoxyphenyl-hydroxy-($C_1$–$C_7$)-alkyl,
($C_1$–$C_7$)-Alkoxy-($C_1$–$C_7$)-alkyl,
($C_1$–$C_7$)-Alkoxy-hydroxy-($C_1$–$C_7$)-alkyl,
($C_1$–$C_7$)-Alkanoyloxy-($C_1$–$C_7$)-alkyl,
($C_1$–$C_7$)-Alkoxycarbonyl-($C_1$–$C_7$)-alkyl,
($C_2$–$C_7$)-Alkenyl, ($C_3$–$C_6$)-Cycloalkyl-($C_1$–$C_7$)-alkyl,
($C_2$–$C_7$)-Alkinyl,
Thienyl-($C_1$–$C_7$)-alkyl,
Furyl-($C_1$–$C_7$)-alkyl,
($C_1$–$C_7$)-Alkanoyl-($C_1$–$C_7$)-alkyl,
($C_3$–$C_6$)-Cycloalkoxy-($C_1$–$C_7$)-alkyl,
($C_3$–$C_6$)-Cycloalkyl-hydroxy-($C_1$–$C_7$)-alkyl,
($C_2$–$C_7$)-Alkenyloxy-($C_1$–$C_7$)-alkyl,
N-($C_1$–$C_7$)-Alkyl-pyrrolidinyl-($C_1$–$C_7$)-alkyl,
Trifluoralkyl mit 2 bis 6 Kohlenstoffatomen oder jeweils gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Phenoxy-hydroxy-($C_1$–$C_7$)-alkyl, Benzoyloxy-($C_1$–$C_7$)-alkyl, Benzoyl-($C_1$–$C_7$)-alkyl, Phenyl-($C_1$–$C_7$)-alkyl, Phenylcarboxamido-($C_1$–$C_7$)-alkyl, Phenyl-($C_2$–$C_7$)-alkenyl, Phenoxy-($C_1$–$C_7$)-alkyl oder Phenyl-N-imidazolonyl-($C_1$–$C_7$)-alkyl oder $R_6$, $R_7$ >N-($C_1$–$C_7$)-Alkyl bedeutet und $R_2$, $R_3$, $R_6$, $R_7$ und X wie zuvor beschrieben sind,

eine Verbindung der allgemeinen Formel

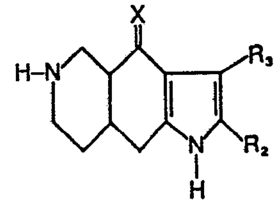

Ab,

worin $R_2$, $R_3$ und X wie zuvor beschrieben sind, am Isochinolin-Stickstoffatom substituiert, oder

h) zur Herstellung einer Verbindung der allgemeinen Formel

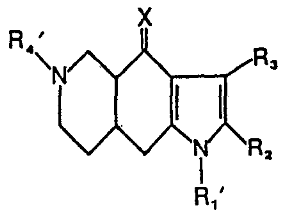

Af,

worin $R_1'$, $R_2$, $R_3$, $R_4'$ und X wie zuvor beschrieben sind, eine Verbindung der folgenden Formel Ae am Isochinolin-Stickstoffatom substituiert, oder

i) zur Herstellung einer Verbindung der allgemeinen Formel

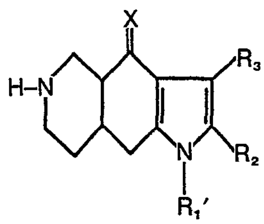

Ae,

worin $R_1'$, $R_2$, $R_3$ und X wie zuvor beschrieben sind,
die Schutzgruppe in einer Verbindung der allgemeinen Formel

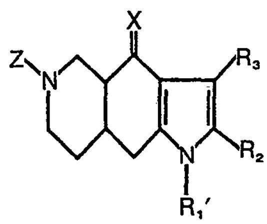

Ah,

worin Z eine Schutzgruppe ist und $R_1'$, $R_2$, $R_3$ und X wie zuvor beschrieben sind, abspaltet, oder

j) zur Herstellung einer Verbindung der allgemeinen Formel

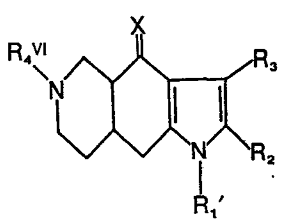

Af',

worin $R_4^{VI}$ Wasserstoff,
($C_1$–$C_7$)-Alkyl, ($C_1$–$C_7$)-Alkoxy-($C_1$–$C_7$)-alkyl,
($C_1$–$C_7$)-Alkanoyloxy-($C_1$–$C_7$)-alkyl,
($C_1$–$C_7$)-Alkoxycarbonyl-($C_1$–$C_7$)-alkyl,
($C_2$–$C_7$)-Alkenyl,
($C_3$–$C_6$)-Cycloalkyl-($C_1$–$C_7$)-alkyl,
($C_2$–$C_7$)-Alkinyl, Thienyl-($C_1$–$C_7$)-alkyl,
Furyl-($C_1$–$C_7$)-alkyl,
($C_1$–$C_7$)-Alkanoyl-($C_1$–$C_7$)-alkyl,
($C_3$–$C_6$)-Cycloalkyloxy-($C_1$–$C_7$)-alkyl,
($C_2$–$C_7$)-Alkenyloxy-($C_1$–$C_7$)-alkyl,
N-($C_1$–$C_7$)-Alkyl-pyrrolidinyl-($C_1$–$C_7$)-alkyl,
Trifluoralkyl mit 2 bis 6 Kohlenstoffatomen oder jeweils gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Phenyl-N-imidazolonyl-($C_1$–$C_7$)-alkyl, Benzoyloxy-($C_1$–$C_7$)-alkyl, Phenyl-($C_1$–$C_7$)-alkyl, Phenylcarboxamido-($C_1$–$C_7$)-alkyl, Benzoyl-($C_1$–$C_7$)-alkyl, Phenyl-($C_2$–$C_7$)-alkenyl oder Phenoxy-($C_1$–$C_7$)-alkyl bedeutet und $R_1'$, $R_2$, $R_3$ und X wie zuvor beschrieben sind,
eine Verbindung der allgemeinen Formel

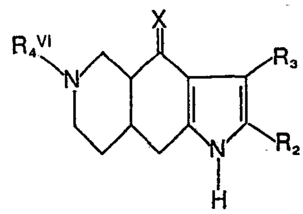

Ad',

worin $R_2$, $R_3$, $R_4^{VI}$ und X wie zuvor beschrieben sind, am Pyrrol-Stickstoffatom substituiert, oder

k) zur Herstellung einer Verbindung der allgemeinen Formel

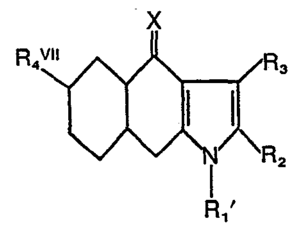

Af''

worin $R_4^{VII}$ Hydroxy-($C_1$–$C_7$)-alkyl,
Phenyl-hydroxy-($C_1$–$C_7$)-alkyl,
Halogenphenyl-hydroxy-($C_1$–$C_7$)-alkyl,
($C_1$–$C_7$)-Alkylphenyl-hydroxy-($C_1$–$C_7$)-alkyl,
($C_1$–$C_7$)-Alkoxyphenyl-hydroxy-($C_1$–$C_7$)-alkyl,
($C_1$–$C_7$)-Alkoxy-hydroxy-($C_1$–$C_7$)-alkyl,
($C_3$–$C_6$)-Cycloalkyl-hydroxy-($C_1$–$C_7$)-alkyl, $\underset{R_7}{\overset{R_6}{>}}$N-($C_1$–$C_7$)-Alkyl oder gegebenenfalls am Phenylring durch Halogen, Trifluormethyl, ($C_1$–$C_7$)-Alkyl, ($C_1$–$C_7$)-Alkoxy, Nitro, Amino, ($C_1$–$C_7$)-Alkylamino oder Di-($C_1$–$C_7$)-alkylamino substituiertes Phenoxy-hydroxy-($C_1$–$C_7$)-alkyl bedeutet und $R_1'$, $R_2$, $R_3$, $R_6$, $R_7$ und X wie zuvor beschrieben sind,
die Schutzgruppe in einer Verbindung der allgemeinen Formel

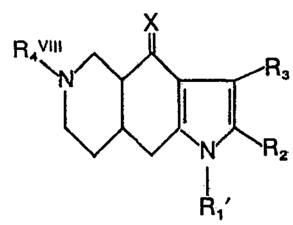

Af'''

worin $R_4^{VIII}$ die gleiche Bedeutung wie $R_4^{VII}$ hat, jedoch in geschützter Form, und $R_1'$, $R_2$, $R_3$ und X wie zuvor beschrieben sind, abspaltet, oder

l) das erhaltene Gemisch der cis- und trans-Isomeren zu einem Endverhältnis, das überwiegend das trans-Isomere aufweist, isomerisiert, oder

m) das trans-Isomere aus dem erhaltenen Gemisch abtrennt, oder

n) ein erhaltenes racemisches Gemisch in die optischen Antipoden aufspaltet, und

o) wenn gewünscht, eine erhaltene Verbindung oder ein pharmazeutisch nicht annehmbares Säureadditionssalz in ein pharmazeutisch annehmbares Säureadditionssalz überführt.

2. Verfahren nach Anspruch 1, bei dem Verbindungen der Formel A, worin $R_1$ Wasserstoff, $(C_1–C_7)$-Alkyl, $(C_1–C_7)$-Alkanoyl oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Benzoyl oder Phenyl-$(C_1–C_7)$-alkyl, $R_2$ und $R_3$ unabhängig voneinander Wasserstoff, $(C_1–C_7)$-Alkyl, $(C_3–C_6)$-Cycloalkyl, $(C_2–C_7)$-Alkenyl oder gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes
Phenyl-$(C_1–C_7)$-alkyl und $R_4$ Wasserstoff,
$(C_1–C_7)$-Alkyl, Hydroxy-$(C_1–C_7)$-alkyl,
Phenyl-hydroxy-$(C_1–C_7)$-alkyl,
Halogenphenyl-hydroxy-$(C_1–C_7)$-alkyl,
$(C_1–C_7)$-Alkylphenyl-hydroxy-$(C_1–C_7)$-alkyl,
$(C_1–C_7)$-Alkoxyphenyl-hydroxy-$(C_1–C_7)$-alkyl,
$(C_1–C_7)$-Alkoxy-$(C_1–C_7)$-alkyl,
$(C_1–C_7)$-Alkoxy-hydroxy-$(C_1–C_7)$-alkyl,
$(C_1–C_7)$-Alkanoyloxy-$(C_1–C_7)$-alkyl,
$(C_1–C_7)$-Alkoxycarbonyl-$(C_1–C_7)$-alkyl,
$(C_2–C_7)$-Alkenyl, $(C_3–C_6)$-Cycloalkyl-$(C_1–C_7)$-alkyl,
$(C_2–C_7)$-Alkinyl, Thienyl-$(C_1–C_7)$-alkyl,
Furyl-$(C_1–C_7)$-alkyl oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Phenoxy-hydroxy-$(C_1–C_7)$-alkyl, Benzoyloxy-$(C_1–C_7)$-alkyl, Phenyl-$(C_1–C_7)$-alkyl

oder Benzoyl-$(C_1–C_7)$-alkyl oder 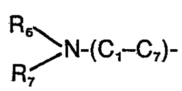

Alkyl und X O sind, deren optische und geometrische Isomere und deren pharmazeutisch annehmbare Säureadditionssalze hergestellt werden, dadurch gekennzeichnet, dass die Verbindungen nach den Ausführungsformen c), l), m), n) und o) gemäss Anspruch 1 oder nach der Ausführungsform a) oder b), worin eine Verbindung der Formel IX oder XII als Ausgangsmaterial eingesetzt wird, worin $R_4''$ Methyl ist, oder nach der Ausführungsform f), worin eine Verbindung der Formel Aa als Ausgangsmaterial eingesetzt wird, worin $R_4''$ Methyl und X O sind, oder nach den Ausführungsformen g), h), i), j) oder k), worin eine Verbindung der Formel Ab, Ae, Ah, Ad' oder Af''' als Ausgangsmaterial eingesetzt wird, wobei X O ist, hergestellt werden.

3. Verfahren nach Anspruch 1 oder 2, dadurch gekennzeichnet, dass es für $R_1$ mit der Bedeutung Wasserstoff durchgeführt wird.

4. Verfahren nach einem der Ansprüche 1 bis 3, dadurch gekennzeichnet, dass es für $R_2$ und $R_3$ mit der Bedeutung $(C_1–C_7)$-Alkyl durchgeführt wird.

5. Verbindung nach einem der Ansprüche 1 bis 4, worin $R_4$ $(C_1–C_7)$-Alkyl, Hydroxy-$(C_1–C_7)$-alkyl, Phenyl-hydroxy-$(C_1–C_7)$-alkyl, Halogenphenyl-hydroxy-$(C_1–C_7)$-alkyl, $(C_1–C_7)$-Alkoxy-$(C_1–C_7)$-alkyl oder jeweils gegebenenfalls am Phenylring durch Halogen, Trifluormethyl, $(C_1–C_7)$-Alkyl, $(C_1–C_7)$-Alkoxy, Nitro, Amino, $(C_1–C_7)$-Alkylamino oder Di-$(C_1–C_7)$-alkylamino substituiertes Phenoxy-$(C_1–C_7)$-alkyl, Benzoyl-$(C_1–C_7)$-alkyl oder Phenyl-$(C_1–C_7)$-alkyl ist.

6. Verfahren nach einem der Ansprüche 1 und 3 bis 5, dadurch gekennzeichnet, dass es für X mit der Bedeutung O durchgeführt wird.

7. Verfahren nach einem der Ansprüche 1 bis 6, dadurch gekennzeichnet, dass die Verbindung in Form des trans-Isomeren hergestellt wird.

8. Verfahren nach Anspruch 2, dadurch gekennzeichnet, dass
3-Äthyl-2,6-dimethyl-4,4a,5,6,7,8,8a,9-octahydro-4a,8a-trans-1H-pyrrolo[2,3-g]isochinolin-4-on
hergestellt wird.

9. Verfahren nach Anspruch 1, dadurch gekennzeichnet, dass
2-Methyl-3-äthyl-6-(2-phenyläthyl)-4,4a,5,6,7,8,-8a,9-octahydro-4a,8a-trans-1H-pyrrolo[2,3-g]isochinolin-4-on
hergestellt wird.

10. Verfahren nach Anspruch 1, dadurch gekennzeichnet, dass
2-Methyl-3-äthyl-6-(2-äthoxyäthyl)-4,4a,5,6,7,8,-8a,9-octahydro-4a,8a-trans-1H-pyrrolo[2,3-g]isochinolin-4-on
hergestellt wird.

11. Verfahren nach Anspruch 1, dadurch gekennzeichnet, dass
2-Methyl-3-äthyl-6-[4-(4-fluorphenyl)-4-oxobutyl]-4,4a,5,6,7,8,8a,9-octahydro-4a,8a-trans-1H-pyrrolo[2,3-g]isochinolin-4-on
hergestellt wird.

12. Verfahren nach Anspruch 1, dadurch gekennzeichnet, dass
2-Methyl-3-äthyl-6-(2-hydroxy-3,3-dimethylbutyl)-4,4a,5,6,7,8,8a,9-octahydro-4a,8a-trans-1H-pyrrolo[2,3-g]isochinolin-4-on
hergestellt wird.

**Claims for the Contracting States: BE, CH, DE, FR, GB, IT, LU, NL, SE.**

1. An octahydro-1H-pyrrolo[2,3-g]isoquinoline of the general formula

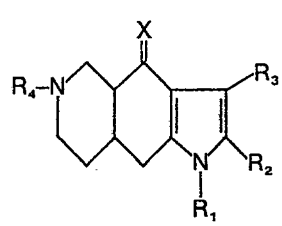 A

wherein $R_1$ is hydrogen, $(C_1–C_7)$-alkyl, $(C_1–C_7)$-alkanoyl or benzoyl or phenyl-$(C_1–C_7)$-alkyl each of which is optionally substituted on the phenyl ring by halogen, trifluoromethyl, $(C_1–C_7)$-alkyl,

$(C_1–C_7)$-alkoxy, nitro, amino, $(C_1–C_7)$-alkylamino or di-$(C_1–C_7)$-alkylamino, $R_2$ and $R_3$ independently of each other are hydrogen, $(C_1–C_7)$-alkyl, $(C_3–C_6)$-cycloalkyl, $(C_2–C_7$-alkenyl, $(C_1–C_7)$-alkanoyl or benzoyl, phenyl or phenyl-$(C_1–C_7)$-alkyl each of which is optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkyl-amino and $R_4$ is hydrogen,
$(C_1–C_7)$-alkyl, hydroxy-$(C_1–C_7)$-alkyl,
phenyl-hydroxy-$(C_1–C_7)$-alkyl,
halophenyl-hydroxy-$(C_1–C_7)$-alkyl,
$(C_1–C_7)$-alkylphenyl-hydroxy-$(C_1–C_7)$-alkyl,
$(C_1–C_7)$-alkoxyphenyl-hydroxy-$(C_1–C_7)$-alkyl,
$(C_1–C_7)$-alkoxy-$(C_1–C_7)$-alkyl,
$(C_1–C_7)$-alkoxy-hydroxy-$(C_1–C_7)$-alkyl,
$(C_1–C_7)$-alkanoyloxy-$(C_1–C_7)$-alkyl,
$(C_1–C_7)$-alkoxycarbonyl-$(C_1–C_7)$-alkyl,
$(C_2–C_7)$-alkenyl, $(C_3–C_6)$-cycloalkyl-$(C_1–C_7)$-alkyl, $(C_2–C_7)$-alkynyl, thienyl-$(C_1–C_7)$-alkyl, furyl-$(C_1–C_7)$-alkyl,
$(C_1–C_7)$-alkanoyl-$(C_1–C_7)$-alkyl,
$(C_3–C_6)$-cycloalkoxy-$(C_1–C_7)$-alkyl,
$(C_3–C_6)$-cycloalkyl-hydroxy-$(C_1–C_7)$-alkyl,
$(C_2–C_7)$-alkenyloxy-$(C_1–C_7)$-alkyl,
N-$(C_1–C_7)$-alkyl-pyrrolidinyl-$(C_1–C_7)$-alkyl,
trifluoroalkyl with 2 to 6 carbon atoms or phenoxy-hydroxy-$(C_1–C_7)$-alkyl, benzoyloxy-$(C_1–C_7)$-alkyl, benzoyl-$(C_1–C_7)$-alkyl, phenyl-$(C_1–C_7)$-alkyl, phenylcarboxamido-$(C_1–C_7)$-alkyl, phenyl-$(C_2–C_7)$-alkenyl, phenoxy-$(C_1–C_7)$-alkyl or phenyl-N-imidazolonyl-$(C_1–C_7)$-alkyl each of which is optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkyl-amino, or 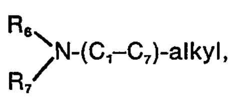, wherein $R_6$ and $R_7$ independently of each other are hydrogen or $(C_1–C_7)$-alkyl or together with the nitrogen atom are a 5- or membered saturated heterocyclic ring which can further contain as a ring member an oxygen atom or a nitrogen atom optionally substituted by $(C_1–C_7)$-alkyl or hydroxy-$(C_1–C_7)$-alkyl, and X is O or S, an optical or geometric isomer thereof or a pharmaceutically acceptable acid addition salt thereof.

2. A compound according to claim 1, wherein $R_1$ is hydrogen, $(C_1–C_7)$-alkyl, $(C_1–C_7)$-alkanoyl or benzoyl or phenyl-$(C_1–C_7)$-alkyl each of which is optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino, $R_2$ and $R_3$ independently of each other are hydrogen, $(C_1–C_7)$-alkyl, $(C_3–C_6)$-cycloalkyl, $(C_2–C_7)$-alkenyl or phenyl-$(C_1–C_7)$-alkyl optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino and $R_4$ is hydrogen,
$(C_1–C_7)$-alkyl, hydroxy-$(C_1–C_7)$-alkyl,
phenyl-hydroxy-$(C_1–C_7)$-alkyl,
halophenyl-hydroxy-$(C_1–C_7)$-alkyl,
$(C_1–C_7)$-alkylphenyl-hydroxy-$(C_1–C_7)$-alkyl,
$(C_1–C_7)$-alkoxyphenyl-hydroxy-$(C_1–C_7)$-alkyl, $(C_1–C_7)$-alkoxy-$(C_1–C_7)$-alkyl,
$(C_1–C_7)$-alkoxy-hydroxy-$(C_1–C_7)$-alkyl,
$(C_1–C_7)$-alkanoyloxy-$(C_1–C_7)$-alkyl,
$(C_1–C_7)$-alkoxycarbonyl-$(C_1–C_7)$-alkyl,
$(C_2–C_7)$-alkenyl, $(C_3–C_6)$-cycloalkyl-$(C_1–C_7)$-alkyl, $(C_2–C_7)$-alkynyl,
thienyl-$(C_1–C_7)$-alkyl, furyl-$(C_1–C_7)$-alkyl
or phenoxy-hydroxy-$(C_1–C_7)$-alkyl,
benzoyloxy-$(C_1–C_7)$-alkyl, phenyl-$(C_1–C_7)$-alkyl or benzoyl-$(C_1–C_7)$-alkyl each of which is optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkyl-amino, or 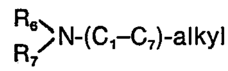 and X is O, an optical or geometric isomer thereof or a pharmaceutically acceptable acid addition salt thereof.

3. A compound according to claim 1 or 2, wherein $R_1$ is hydrogen.

4. A compound according to any one of claims 1 to 3, wherein $R_2$ and $R_3$ are $(C_1–C_7)$-alkyl.

5. A compound according to any one of claims 1 to 4, wherein $R_4$ is $(C_1–C_7)$-alkyl, hydroxy-$(C_1–C_7)$-alkyl, phenyl-hydroxy-$(C_1–C_7)$-alkyl, halophenyl-hydroxy-$(C_1–C_7)$-alkyl, $(C_1–C_7)$-alkoxy-$(C_1–C_7)$-alkyl or phenoxy-$(C_1–C_7)$-alkyl, benzoyl-$(C_1–C_7)$-alkyl or phenyl-$(C_1–C_7)$-alkyl each of which is optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkyl-amino.

6. A compound according to any one of claims 1 and 3 to 5, wherein X is O.

7. A compound according to any one of claims 1 to 6, which is the trans isomer.

8. 3-Ethyl-2,6-dimethyl-4,4a,5,6,7,8,8a,9-octa-hydro-4a,8a-trans-1H-pyrrolo-[2,3-g]isoquinolin-4-one.

9. 2-Methyl-3-ethyl-6-(2-phenylethyl)-4,4a,5,6,7,8,8a,9-octahydro-4a,8a-trans-1H-pyrrolo[2,3-g]isoquinolin-4-one.

10. 2-Methyl-3-ethyl-6-(2-ethoxyethyl)-4,4a,5,6,7,8,8a,9-octahydro-4a,8a-trans-1H-pyrrolo[2,3-g]isoquinolin-4-one.

11. 2-Methyl-3-ethyl-6-[4-(4-fluorophe-nyl)-4-oxo-butyl]-4,4a,5,6,7,8,8a,9-octa-hydro-4a,8a-trans-1H-pyrrolo[2,3-g]iso-quinolin-4-one.

12. 3-Ethyl-2-methyl-6-(2-hydroxy-3,3-dimethylbutyl)-4,4a,5,6,7,8,8a,9-octa-hydro-1H-pyrrolo[2,3-g)isoquinolin-4-one.

13. A compound according to claim 1, wherein $R_1$ is hydrogen, $R_2$ is methyl, $R_3$ is ethyl, X is O and $R_4$ is
2-hydroxy-2-phenylethyl, benzyl,
3-(4-fluorophenyl)-3-oxo-propyl,
3-phenoxypropyl, 2-hydroxy-3-methylbutyl,
2-hydroxy-ethyl, 2 propenyl,
cyclopropylmethyl, ethyl,
2-oxo-2-(4-fluorophenyl)-ethyl.

14. A compound according to claim 1, wherein $R_1$ is hydrogen, $R_2$ and $R_3$ are methyl, X is O and $R_4$ is methyl, 2-phenethyl, 4-(4-fluorophenyl)-4-oxobutyl.

15. An octahydro-1H-pyrrolo[2,3-g]isoquinoline according to any one of claims 1 to 14 as a pharmaceutically active substance.

16. An octahydro-1H-pyrrolo[2,3-g]isoquinoline according to any one of claims 1 to 14 as a neuroleptic/antipsychotic active substance.

17. 3-Ethyl-2,6-dimethyl-4,4a,5,6,7,8,8a,9-octahydro-4a,8a-trans-1H-pyrrolo[2,3-g]isoquinolin-4-one as a pharmaceutically active substance.

18. 3-Ethyl-2,6-dimethyl-4,4a,5,6,7,8,8a,9-octahydro-4a,8a-trans-1H-pyrrolo-[2,3-g]isoquinolin-4-one as a neuroleptic/antipsychotic active substance.

19. A process for the manufacture of compounds of formula A in claim 1, of optical and geometric isomers thereof and of pharmaceutically acceptable acid addition salts thereof, characterized by

a) for the manufacture of a compound of the general formula

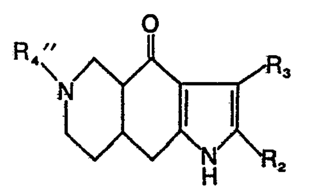

Ia,

wherein $R_4''$ is $(C_1–C_7)$-alkyl, $(C_1–C_7)$-alkoxy-$(C_1–C_7)$-alkyl or $(C_3–C_6)$-cyclo-alkyl-$(C_1–C_7)$-alkyl and $R_2$ and $R_3$ are as described previously, treating a compound of the general formula

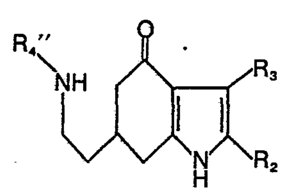

IX,

wherein $R_2$, $R_3$ and $R_4''$ are as described previously, with formaldehyde, or

b) for the manufacture of a compound of formula Ia above, treating a compound of the general formula

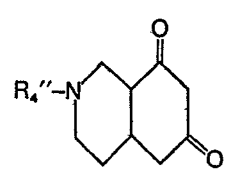

XII,

wherein $R_4''$ is as described previously, with a compound of the general formula

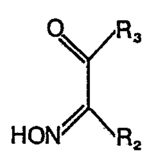

VII

in the presence of a reducing agent, or with a compound of the general formula

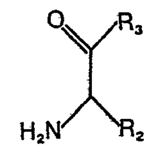

VIII,

wherein $R_2$ and $R_3$ are as described previously, or a precursor thereof, or

c) for the manufacture of a compound of the general formula

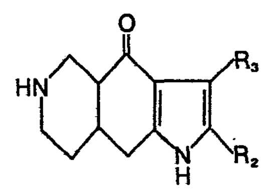

Ib

wherein $R_2$ and $R_3$ are as described previously, N-demethylating a compound of formula Ia above wherein $R_4''$ is methyl, or

d) for the manufacture of a compound of the general formula

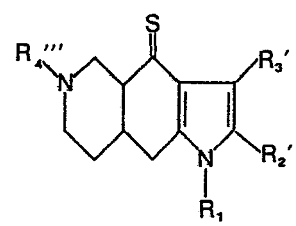

IIc'',

wherein $R_2'$ and $R_3'$ independently of each other are hydrogen, $(C_1–C_7)$-alkyl, $(C_3–C_6)$-cycloalkyl, $(C_2–C_7)$-alkenyl or phenyl or phenyl-$(C_1–C_7)$-alkyl each of which is optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino, $R_4'''$ is hydrogen, $(C_1–C_7)$-alkyl, $(C_1–C_7)$-alkoxy-$(C_1–C_7)$-alkyl, $(C_2–C_7)$-alkenyl, $(C_3–C_6)$-cycloalkyl-$(C_1–C_7)$-alkyl, $(C_2–C_7)$-alkynyl, thienyl-$(C_1–C_7)$-alkyl, furyl-$(C_1–C_7)$-alkyl, $(C_2–C_7)$-alkenyloxy-$(C_1–C_7)$-alkyl or phenyl-$(C_1–C_7)$-alkyl, phenyl-$(C_2–C_7)$-alkenyl or phenoxy-$(C_1–C_7)$-alkyl each of which is optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino, or trifluoroalkyl with 2 to 6 carbon atoms and $R_1$ is as described previously, treating a compound of the general formula

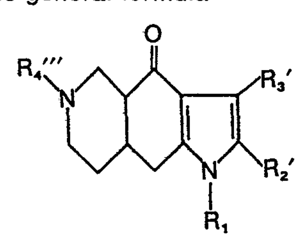

Ic'',

wherein $R_1$, $R_2'$, $R_3'$ and $R_4'''$ are as described previously, with phosphorus pentasulphide, or

e) for the manufacture of a compound of the general formula

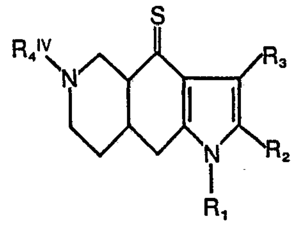

IIc''',

wherein $R_4^{IV}$ is
hydroxy-($C_1$–$C_7$)-alkyl,
phenyl-hydroxy-($C_1$–$C_7$)-alkyl,
halophenyl-hydroxy-($C_1$–$C_7$)-alkyl,
($C_1$–$C_7$)-alkylphenyl-hydroxy-($C_1$–$C_7$)-alkyl,
($C_1$–$C_7$)-alkoxyphenyl-hydroxy-($C_1$–$C_7$)-alkyl,
($C_1$–$C_7$-alkoxy-hydroxy-($C_1$–$C_7$)-alkyl,
($C_1$–$C_7$)-alkanoyloxy-($C_1$–$C_7$)-alkyl,
($C_1$–$C_7$)-alkoxycarbonyl-($C_1$–$C_7$)-alkyl,
($C_1$–$C_7$)-alkanoyl-($C_1$–$C_7$)-alkyl,
($C_3$–$C_6$)-cycloalkoxy-($C_1$–$C_7$)-alkyl,
($C_3$–$C_6$)-cycloalkyl-hydroxy-($C_1$–$C_7$)-alkyl,
N-($C_1$–$C_7$)-alkyl-pyrrolidinyl-($C_1$–$C_7$)-alkyl
or phenyl-N-imidazolonyl-($C_1$–$C_7$)-alkyl,
phenoxy-hydroxy-($C_1$–$C_7$)-alkyl,
benzoyloxy-($C_1$–$C_7$)-alkyl,
benzoyl-($C_1$–$C_7$)-alkyl or phenyl-carboxamido-($C_1$–$C_7$)-alkyl each of which is optionally substituted on the phenyl ring by halogen, trifluoromethyl, ($C_1$–$C_7$)-alkyl, ($C_1$–$C_7$)-alkoxy, nitro, amino, ($C_1$–$C_7$)-alkylamino or di-($C_1$–$C_7$)-alkyl-amino, or

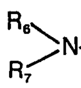N-($C_1$–$C_7$)-alkyl and $R_1$, $R_2$, $R_3$, $R_6$ and $R_7$ are as described previously, cleaving off the protecting group or protecting groups in a compound of the general formula

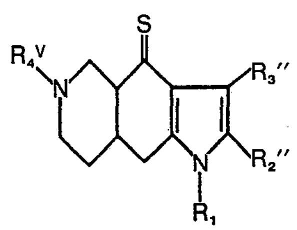

IIf,

wherein $R_2''$ and $R_3''$ have the same significance as $R_2$ and $R_3$ except when $R_2$ and $R_3$ are ($C_1$–$C_7$)-alkanoyl or benzoyl optionally substituted on the phenyl ring by halogen, trifluoromethyl, ($C_7$–$C_7$)-alkyl, ($C_1$–$C_7$)-alkoxy, nitro, amino, ($C_1$–$C_7$)-alkylamino or di-($C_1$–$C_7$)-alkylamino, $R_2''$ and $R_3''$ are in protected form, $R_4^V$ has the same significance as $R_4^{IV}$, but in protected form, and $R_1$ is as described previously, or

f) for the manufacture of a compound of the general formula

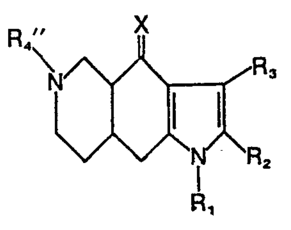

. Ac,

wherein $R_1'$ is ($C_1$–$C_7$)-alkyl, ($C_1$–$C_7$)-alkanoyl or benzoyl or phenyl-($C_1$–$C_7$)-alkyl each of which is optionally substituted on the phenyl ring by halogen, trifluoromethyl, ($C_1$–$C_7$)-alkyl, ($C_1$–$C_7$)-alkoxy, nitro, amino, ($C_1$–$C_7$)-alkylamino or di-($C_1$–$C_7$)-alkylamino and $R_2$, $R_3$, $R_4''$ and X are as described previously, substituting a compound of the general formula

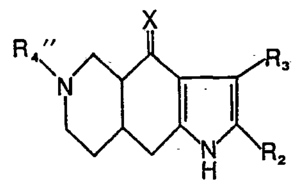

Aa,

wherein $R_2$, $R_3$, $R_4''$ and X are as described previously, at the pyrrole nitrogen atom, or

g) for the manufacture of a compound of the general formula

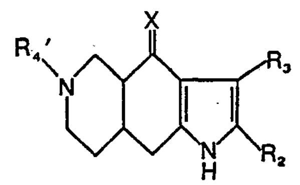

Ad,

wherein $R_4'$ is
($C_1$–$C_7$)-alkyl, hydroxy-($C_1$–$C_7$)-alkyl, phenyl-hydroxy-($C_1$–$C_7$)-alkyl,
halophenyl-hydroxy-($C_1$–$C_7$)-alkyl,
($C_1$–$C_7$)-alkylphenyl-hydroxy-($C_1$–$C_7$)-alkyl,
($C_1$–$C_7$)-alkoxyphenyl-hydroxy-($C_1$–$C_7$)-alkyl,
($C_1$–$C_7$)-alkoxy-($C_1$–$C_7$)-alkyl,
($C_1$–$C_7$)-alkoxy-hydroxy-($C_1$–$C_7$)-alkyl,
($C_1$–$C_7$)-alkanoyloxy-($C_1$–$C_7$)-alkyl,
($C_1$–$C_7$)-alkoxycarbonyl-($C_1$–$C_7$)-alkyl,
($C_2$–$C_7$)-alkenyl, ($C_3$–$C_6$)-cycloalkyl-($C_1$–$C_7$)-alkyl, ($C_2$–$C_7$)-alkynyl,
thienyl-($C_1$–$C_7$)-alkyl, furyl-($C_1$–$C_7$)-alkyl,
($C_1$–$C_7$)-alkanoyl-($C_1$–$C_7$)-alkyl,
($C_3$–$C_6$)-cycloalkoxy-($C_1$–$C_7$)-alkyl,
($C_3$–$C_6$)-cycloalkyl-hydroxy-($C_1$–$C_7$)-alkyl,
($C_2$–$C_7$)-alkenyloxy-($C_1$–$C_7$)-alkyl,
N-($C_1$–$C_7$)-alkyl-pyrrolidinyl-($C_1$–$C_7$)-alkyl,
trifluoro-alkyl with 2 to 6 carbon atoms or
phenoxy-hydroxy-($C_1$–$C_7$)-alkyl,
benzoyloxy-($C_1$–$C_7$)-alkyl,
benzoyl-($C_1$–$C_7$)-alkyl, phenyl-($C_1$–$C_7$)-alkyl,
phenylcarboxamido-($C_1$–$C_7$)-alkyl,
phenyl-($C_2$–$C_7$)-alkenyl,
phenoxy-($C_1$–$C_7$)-alkyl or phenyl-N-imidazolonyl-($C_1$–$C_7$)-alkyl each of which is optionally substituted on the phenyl ring by halogen, trifluoromethyl, ($C_1$–$C_7$)-alkyl, ($C_1$–$C_7$)-alkoxy, nitro, amino, ($C_1$–$C_7$)-alkylamino or di-($C_1$–$C_7$)-alkylamino, or

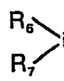N-($C_1$–$C_7$)-alkyl and $R_2$, $R_3$, $R_6$, $R_7$ and X are as

described previously, substituting a compound of the general formula

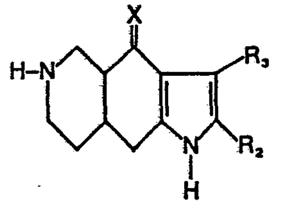

Ab,

wherein $R_2$, $R_3$ and X are as described previously, at the isoquinoline nitrogen atom, or

h) for the manufacture of a compound of the general formula

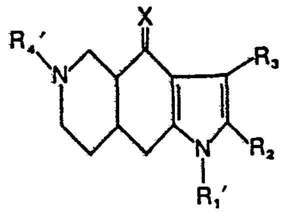

Af,

wherein $R_1'$, $R_2$, $R_3$, $R_4'$ and X are as described previously, substituting a compound of the following formula Ae at the isoquinoline nitrogen atom, or

i) for the manufacture of a compound of the general formula

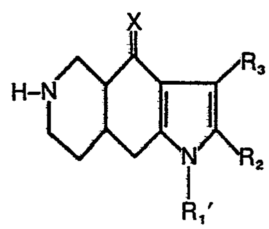

Ae,

wherein $R_1'$, $R_2$, $R_3$ and X are as described previously, cleaving off the protecting group in a compound of the general formula

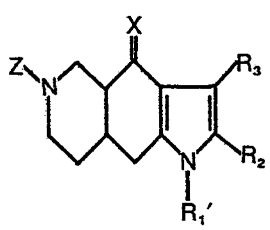

Ah,

wherein Z is a protecting group and $R_1'$, $R_2$, $R_3$ and X are as described previously,

j) for the manufacture of a compound of the general formula

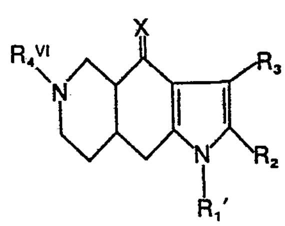

Af',

wherein $R_4^{VI}$ is hydrogen, ($C_1$–$C_7$)-alkyl, ($C_1$–$C_7$)-alkoxy-($C_1$–$C_7$)-alkyl, ($C_1$–$C_7$)-alkanoyloxy-($C_1$–$C_7$)-alkyl, ($C_1$–$C_7$)-alkoxycarbonyl-($C_1$–$C_7$)-alkyl, ($C_2$–$C_7$)-alkenyl, ($C_3$–$C_6$)-cycloalkyl-($C_1$–$C_7$)-alkyl, ($C_2$–$C_7$)-alkynyl, thienyl-($C_1$–$C_7$)-alkyl, furyl-($C_1$–$C_7$)-alkyl, ($C_1$–$C_7$)-alkanoyl-($C_1$–$C_7$)-alkyl, ($C_3$–$C_6$)-cycloalkyloxy-($C_1$–$C_7$)-alkyl, ($C_2$–$C_7$)-alkenyloxy-($C_1$–$C_7$)-alkyl, N-($C_1$–$C_7$)-alkyl-pyrrolidinyl-($C_1$–$C_7$)-alkyl, trifluoroalkyl with 2 to 6 carbon atoms or phenyl-N-imidazolonyl-($C_1$–$C_7$)-alkyl, benzoyloxy-($C_1$–$C_7$)-alkyl, phenyl-($C_1$–$C_7$)-alkyl, phenylcarboxamido-($C_1$–$C_7$)-alkyl, benzoyl-($C_1$–$C_7$)-alkyl, phenyl-($C_2$–$C_7$)-alkenyl or phenoxy-($C_1$–$C_7$)-alkyl each of which is optionally substituted on the phenyl ring by halogen, trifluoromethyl, ($C_1$–$C_7$)-alkyl, ($C_1$–$C_7$)-alkoxy, nitro, amino, ($C_1$–$C_7$)-alkylamino or di-($C_1$–$C_7$)-alkylamino and $R_1'$, $R_2$, $R_3$ and X are as described previously, substituting a compound of the general formula

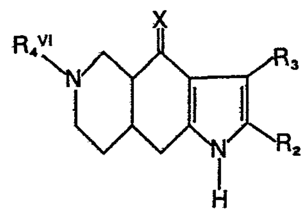

Ad',

wherein $R_2$, $R_3$, $R_4^{VI}$ and X are as described previously, at the pyrrole nitrogen atom, or

k) for the manufacture of a compound of the general formula

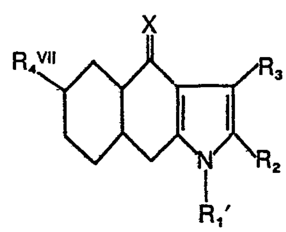

Af''

wherein $R_4^{VII}$ is hydroxy-($C_1$–$C_7$)-alkyl, phenyl-hydroxy-($C_1$–$C_7$)-alkyl, halophenyl-hydroxy-($C_1$–$C_7$)-alkyl, ($C_1$–$C_7$)-alkyl-phenyl-hydroxy-($C_1$–$C_7$)-alkyl, ($C_1$–$C_7$)-alkoxyphenyl-hydroxy-($C_1$–$C_7$)-alkyl, ($C_1$–$C_7$)-alkoxy-hydroxy-($C_1$–$C_7$)-alkyl, ($C_3$–$C_6$)-cycloalkyl-hydroxy-($C_1$–$C_7$)-alkyl,

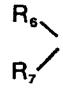N-($C_1$–$C_7$)-alkyl or phenoxy-hydroxy-($C_1$–$C_7$)-alkyl optionally substituted on the phenyl ring by halogen, trifluoromethyl, ($C_1$–$C_7$)-alkyl, ($C_1$–$C_7$)-alkoxy, nitro, amino, ($C_1$–$C_7$)-alkylamino or di-($C_1$–$C_7$)-alkylamino and $R_1'$, $R_2$, $R_3$, $R_6$, $R_7$ and X are

as described previously, cleaving off the protecting group in a compound of the general formula

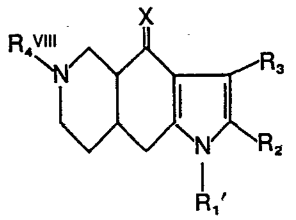

Af'''

wherein $R_4^{VIII}$ has the same significance as $R_4^{VII}$, but in protected form, and $R_1'$, $R_2$, $R_3$ and X are as described previously, or

l) isomerizing the mixture of cis and trans isomers obtained to a final ratio which exhibits predominantly the trans isomer, or

m) separating the trans isomer from the mixture obtained, or

n) resolving a racemic mixture obtained into the optical antipodes, and

o) if desired, converting an obtained compound or a pharmaceutically non-acceptable acid addition salt into a pharmaceutically acceptable acid addition salt.

20. A medicament containing an octahydro-1H-pyrrolo[2,3-g]isoquinoline of formula A in claim 1, an optical or geometric isomer or a pharmaceutically acceptable acid addition salt of this compound.

21. A neuroleptic/antipsychotic medicament containing an octahydro-1H-pyrrolo[2,3-g]isoquinoline of formula A in claim 1, an optical or geometric isomer or a pharmaceutically acceptable acid addition salt of this compound.

22. A medicament containing
3-ethyl-2,6-dimethyl-4,4a,5,6,7,8,8a,9-octahydro-4a,8a-trans-1H-pyrrolo[2,3-g]-isoquinolin-4-one.

23. A neuroleptic/antipsychotic medicament containing
3-ethyl-2,6-dimethyl-4,4a,5,6,7,8,8a,9-octahydro-4a,8a-trans-1H-pyrrolo[2,3-g]isoquinolin-4-one.

**Claims for the Contracting State: AT.**

1. A process for the manufacture of octahydro-1H-pyrrolo[2,3-g]isoquinolines of the general formula

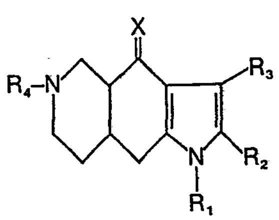

A

wherein $R_1$ is hydrogen, $(C_1–C_7)$-alkyl, $(C_1–C_7)$-alkanoyl or benzoyl or phenyl-$(C_1–C_7)$-alkyl each of which is optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino, $R_2$ and $R_3$ independently of each other are hydrogen, $(C_1–C_7)$-alkyl, $(C_3–C_6)$-cycloalkyl, $(C_2–C_7)$-alkenyl, $(C_1–C_7)$-alkanoyl or benzoyl, phenyl or phenyl-$(C_1–C_7)$-alkyl each of which is optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino and $R_4$ is hydrogen,
$(C_1–C_7)$-alkyl, hydroxy-$(C_1–C_7)$-alkyl,
phenyl-hydroxy-$(C_1–C_7)$-alkyl,
halophenyl-hydroxy-$(C_1–C_7)$-alkyl,
$(C_1–C_7)$-alkylphenyl-hydroxy-$(C_1–C_7)$-alkyl,
$(C_1–C_7)$-alkoxyphenyl-hydroxy-$(C_1–C_7)$-alkyl,
$(C_1–C_7)$-alkoxy-$(C_1–C_7)$-alkyl,
$(C_1–C_7)$-alkoxy-hydroxy-$(C_1–C_7)$-alkyl,
$(C_1–C_7)$-alkanoyloxy-$(C_1–C_7)$-alkyl,
$(C_1–C_7)$-alkoxycarbonyl-$(C_1–C_7)$-alkyl,
$(C_2–C_7)$-alkenyl,
$(C_3–C_6)$-cycloalkyl-$(C_1–C_7)$-alkyl,
$(C_2–C_7)$-alkynyl, thienyl-$(C_1–C_7)$-alkyl,
furyl-$(C_1–C_7)$-alkyl,
$(C_1–C_7)$-alkanoyl-$(C_1–C_7)$-alkyl,
$(C_3–C_6)$-cycloalkoxy-$(C_1–C_7)$-alkyl,
$(C_3–C_6)$-cycloalkyl-hydroxy-$(C_1–C_7)$-alkyl,
$(C_2–C_7)$-alkenyloxy-$(C_1–C_7)$-alkyl,
N-$(C_1–C_7)$-alkyl-pyrrolidinyl-$(C_1–C_7)$-alkyl,
trifluoroalkyl with 2 to 6 carbon atoms or
phenoxy-hydroxy-$(C_1–C_7)$-alkyl,
benzoyloxy-$(C_1–C_7)$-alkyl,
benzoyl-$(C_1–C_7)$-alkyl,
phenyl-$(C_1–C_7)$-alkyl,
phenylcarboxamido-$(C_1–C_7)$-alkyl,
phenyl-$(C_2–C_7)$-alkenyl,
phenoxy-$(C_1–C_7)$-alkyl or phenyl-N-imidazolonyl-$(C_1–C_7)$-alkyl each of which is optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino, or

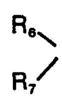N-$(C_1–C_7)$-alkyl, wherein $R_6$ and $R_7$ independently of each other are hydrogen or $(C_1–C_7)$-alkyl or together with the nitrogen atom are a 5- or 6-membered saturated heterocyclic ring which can further contain as a ring member an oxygen atom or a nitrogen atom optionally substituted by $(C_1–C_7)$-alkyl or hydroxy-$(C_1–C_7)$-alkyl, and X is O or S, the optical and geometric isomers of these compounds and their pharmaceutically acceptable acid addition salts, characterized by

a) for the manufacture of a compound of the general formula

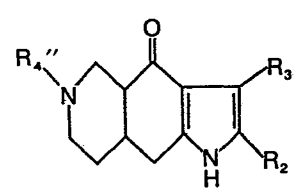

Ia,

wherein $R_4''$ is $(C_1–C_7)$-alkyl, $(C_1–C_7)$-alkoxy-$(C_1–C_7)$-alkyl or $(C_3–C_6)$-cyclo-alkyl-$(C_1–C_7)$-alkyl and $R_2$ and $R_3$ are as described previously, treating a compound of the general formula

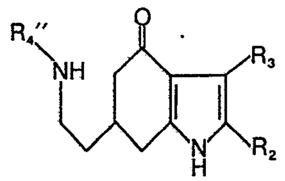

IX,

wherein $R_2$, $R_3$ and $R_4''$ are as described previously, with formaldehyde, or

b) for the manufacture of a compound of formula Ia above, treating a compound of the general formula

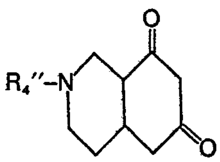

XII,

wherein $R_4''$ is as described previously, with a compound of the general formula

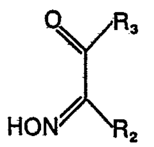

VII

in the presence of a reducing agent, or with a compound of the general formula

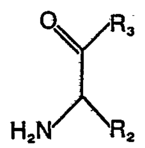

VIII,

wherein $R_2$ and $R_3$ are as described previously, or a precursor thereof, or

c) for the manufacture of a compound of the general formula

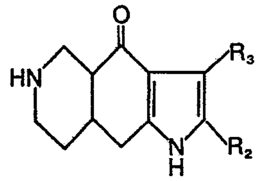

Ib

wherein $R_2$ and $R_3$ are as described previously,

N-demethylating a compound of formula Ia above wherein $R_4''$ is methyl, or

d) for the manufacture of a compound of the general formula

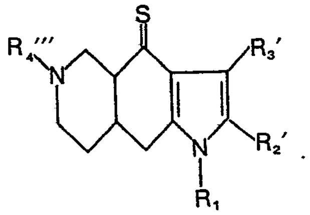

IIc'',

wherein $R_2'$ and $R_3'$ independently of each other are hydrogen, ($C_1$–$C_7$)-alkyl, ($C_3$–$C_6$)-cycloalkyl, ($C_2$–$C_7$)-alkenyl or phenyl or phenyl-($C_1$–$C_7$)-alkyl each of which is otpionally substituted on the phenyl ring by halogen, trifluoromethyl, ($C_1$–$C_7$)-alkyl, ($C_1$–$C_7$)-alkoxy, nitro, amino, ($C_1$–$C_7$)-alkylamino or di-($C_1$–$C_7$)-alkylamino, $R_4'''$ is hydrogen,
($C_1$–$C_7$)-alkyl,
($C_1$–$C_7$)-alkoxy-($C_1$–$C_7$)-alkyl,
($C_2$–$C_7$)-alkenyl,
($C_3$–$C_6$)-cycloalkyl-($C_1$–$C_7$)-alkyl,
($C_2$–$C_7$)-alkynyl,
thienyl-($C_1$–$C_7$)-alkyl,
furyl-($C_1$–$C_7$)-alkyl,
($C_2$–$C_7$)-alkenyloxy-($C_1$–$C_7$)-alkyl
or phenyl-($C_1$–$C_7$)-alkyl, phenyl-($C_2$–$C_7$)-alkenyl or phenoxy-($C_1$–$C_7$)-alkyl each of which is optionally substituted on the phenyl ring by halogen, trifluoro-methyl, ($C_1$–$C_7$)-alkyl, ($C_1$–$C_7$)-alkoxy, nitro, amino, ($C_1$–$C_7$)-alkylamino or di-($C_1$–$C_7$)-alkylamino, or trifluoroalkyl with 2 to 6 carbon atoms and $R_1$ is as described previously, treating a compound of the general formula

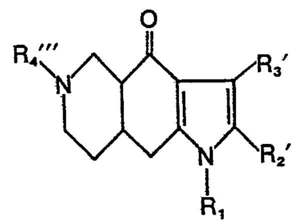

Ic'',

wherein $R_1$, $R_2'$, $R_3'$ and $R_4'''$ are as described previously, with phosphorus pentasulphide, or

e) for the manufacture of a compound of the general formula

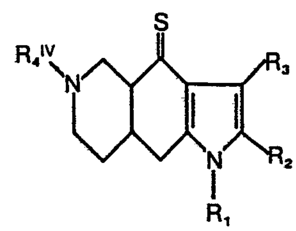

IIc''',

wherein $R_4^{IV}$ is
hydroxy-($C_1$–$C_7$)-alkyl,
phenyl-hydroxy-($C_1$–$C_7$)-alkyl,
halophenyl-hydroxy-($C_1$–$C_7$)-alkyl,
($C_1$–$C_7$)-alkyl-phenyl-hydroxy-($C_1$–$C_7$)-alkyl,
($C_1$–$C_7$)-alkoxyphenyl-hydroxy-($C_1$–$C_7$)-alkyl,
($C_1$–$C_7$)-alkoxy-hydroxy-($C_1$–$C_7$)-alkyl,
($C_1$–$C_7$)-alkanoyloxy-($C_1$–$C_7$)-alkyl,
($C_1$–$C_7$)-alkoxycarbonyl-($C_1$–$C_7$)-alkyl,
($C_1$–$C_7$)-alkanoyl-($C_1$–$C_7$)-alkyl,
($C_3$–$C_6$)-cycloalkoxy-($C_1$–$C_7$)-alkyl,
($C_3$–$C_6$)-cycloalkyl-hydroxy-($C_1$–$C_7$)-alkyl,
N-($C_1$–$C_7$)-alkyl-pyrrolidinyl-($C_1$–$C_7$)-alkyl
or phenyl-N-imidazolonyl-($C_1$–$C_7$)-alkyl, phenoxy-hydroxy-($C_1$–$C_7$)-alkyl, benzoyloxy-($C_1$–$C_7$)-alkyl, benzoyl-($C_1$–$C_7$)-alkyl or phenylcarboxamido-($C_1$–$C_7$)-alkyl each of which is optionally substituted on the phenyl ring by halogen, trifluoromethyl, ($C_1$–$C_7$)-alkyl, ($C_1$–$C_7$)-alkoxy, nitro, amino, ($C_1$–$C_7$)-alkylamino or di-($C_1$–$C_7$)-alkylamino, or

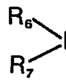N-($C_1$–$C_7$)-alkyl and $R_1$, $R_2$, $R_3$, $R_6$ and $R_7$ are

as described previously, cleaving off the protecting group or protecting groups in a compound of the general formula

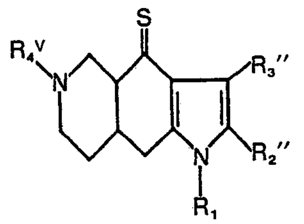

IIf,

wherein $R_2''$ and $R_3''$ have the same significance as $R_2$ and $R_3$ except when $R_2$ and $R_3$ are $(C_1–C_7)$-alkanoyl or benzoyl optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino, $R_2''$ and $R_3''$ are in protected form, $R_4^V$ has the same significance as $R_4^{IV}$, but in protected form, and $R_1$ is as described previously, or

f) for the manufacture of a compound of the general formula

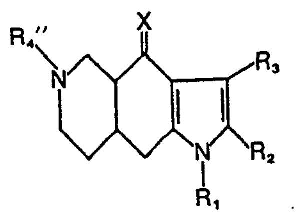

. Ac,

wherein $R_1'$ is $(C_1–C_7)$-alkyl, $(C_1–C_7)$-alkanoyl or benzoyl or phenyl-$(C_1–C_7)$-alkyl each of which is optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino and $R_2$, $R_3$, $R_4''$ and X are as described previously, substituting a compound of the general formula

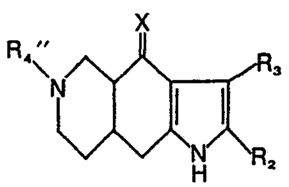

Aa,

wherein $R_2$, $R_3$, $R_4''$ and X are as described previously, at the pyrrole nitrogen atom, or

g) for the manufacture of a compound of the general formula

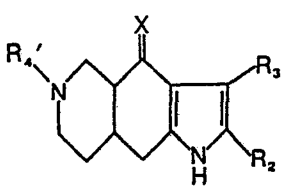

Ad,

wherein $R_4'$ is
$(C_1–C_7)$-alkyl, hydroxy-$(C_1–C_7)$-alkyl,
phenyl-hydroxy-$(C_1–C_7)$-alkyl,
halophenyl-hydroxy-$(C_1–C_7)$-alkyl,
$(C_1–C_7)$-alkylphenyl-hydroxy-$(C_1–C_7)$-alkyl,
$(C_1–C_7)$-alkoxyphenyl-hydroxy-$(C_1–C_7)$-alkyl,
$(C_1–C_7)$-alkoxy-$(C_1–C_7)$-alkyl,
$(C_1–C_7)$-alkoxy-hydroxy-$(C_1–C_7)$-alkyl,
$(C_1–C_7)$-alkanoyloxy-$(C_1–C_7)$-alkyl,
$(C_1–C_7)$-alkoxycarbonyl-$(C_1–C_7)$-alkyl,
$(C_2–C_7)$-alkenyl,
$(C_3–C_6)$-cycloalkyl-$(C_1–C_7)$-alkyl,
$(C_2–C_7)$-alkynyl, thienyl-$(C_1–C_7)$-alkyl,
furyl-$(C_1–C_7)$-alkyl,
$(C_1–C_7)$-alkanoyl-$(C_1–C_7)$-alkyl,
$(C_3–C_6)$-cycloalkoxy-$(C_1–C_7)$-alkyl,
$(C_3–C_6)$-cycloalkyl-hydroxy-$(C_1–C_7)$-alkyl,
$(C_2–C_7)$-alkenyloxy-$(C_1–C_7)$-alkyl,
N-$(C_1–C_7)$-alkyl-pyrrolidinyl-$(C_1–C_7)$-alkyl,
trifluoro-alkyl with 2 to 6 carbon atoms or
phenoxy-hydroxy-$(C_1–C_7)$-alkyl,
benzoyloxy-$(C_1–C_7)$-alkyl,
benzoyl-$(C_1–C_7)$-alkyl, phenyl-$(C_1–C_7)$-alkyl,
phenylcarboxamido-$(C_1–C_7)$-alkyl,
phenyl-$(C_2–C_7)$-alkenyl,
phenoxy-$(C_1–C_7)$-alkyl or phenyl-N-imidazolonyl-$(C_1–C_7)$-alkyl each of which is otpionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino, or 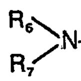-$(C_1–C_7)$-alkyl and $R_2$, $R_3$, $R_6$, $R_7$ and X are as described previously, substituting a compound of the general formula

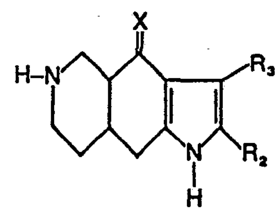

Ab,

wherein $R_2$, $R_3$ and X are as described previously, at the isoquinoline nitrogen atom, or

h) for the manufacture of a compound of the general formula

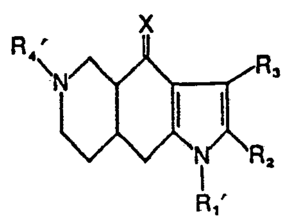

Af,

wherein $R_1'$, $R_2$, $R_3$, $R_4'$ and X are as described previously, substituting a compound of following formula Ae at the isoquinoline nitrogen atom, or

i) for the manufacture of a compound of the general formula

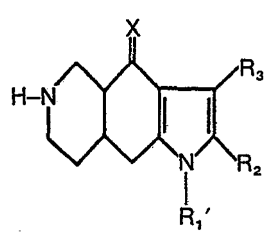

Ae,

wherein $R_1'$, $R_2$, $R_3$ and X are as described previously, cleaving off the protecting group in a compound of the general formula

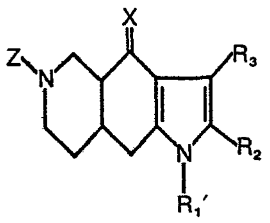

Ah,

wherein Z is a protecting group and $R_1'$, $R_2$, $R_3$ and X are as described previously, or

j) for the manufacture of a compound of the general formula

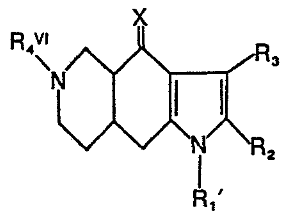

Af',

wherein $R_4^{VI}$ is hydrogen, $(C_1–C_7)$-alkyl, $(C_1–C_7)$-alkoxy-$(C_1–C_7)$-alkyl, $(C_1–C_7)$-alkanoyloxy-$(C_1–C_7)$-alkyl, $(C_1–C_7)$-alkoxycarbonyl-$(C_1–C_7)$-alkyl, $(C_2–C_7)$-alkenyl, $(C_3–C_6)$-cycloalkyl-$(C_1–C_7)$-alkyl, $(C_2–C_7)$-alkynyl, thienyl-$(C_1–C_7)$-alkyl, furyl-$(C_1–C_7)$-alkyl, $(C_1–C_7)$-alkanoyl-$(C_1–C_7)$-alkyl, $(C_3–C_6)$-cycloalkyloxy-$(C_1–C_7)$-alkyl, $(C_2–C_7)$-alkenyloxy-$(C_1–C_7)$-alkyl, N-$(C_1–C_7)$-alkyl-pyrrolidinyl-$(C_1–C_7)$-alkyl, trifluoroalkyl with 2 to 6 carbon atoms or phenyl-N-imidazolonyl-$(C_1–C_7)$-alkyl, benzoyloxy-$(C_1–C_7)$-alkyl, phenyl-$(C_1–C_7)$-alkyl, phenylcarboxamido-$(C_1–C_7)$-alkyl, benzoyl-$(C_1–C_7)$-alkyl, phenyl-$(C_2–C_7)$-alkenyl or phenoxy-$(C_1–C_7)$-alkyl each of which is otpionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino and $R_1'$, $R_2$, $R_3$ and X are as described previously, substituting a compound of the general formula

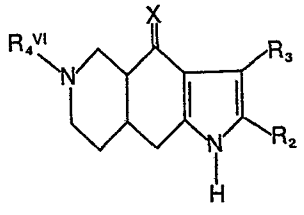

Ad',

wherein $R_2$, $R_3$, $R_4^{VI}$ and X are as described previously, at the pyrrole nitrogen atom, or

k) for the manufacture of a compound of the general formula

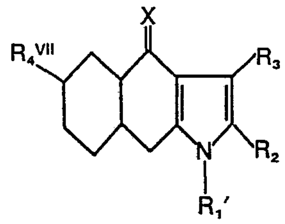

Af''

wherein $R_4^{VII}$ is hydroxy-$(C_1–C_7)$-alkyl, phenyl-hydroxy-$(C_1–C_7)$-alkyl, halophenyl-hydroxy-$(C_1–C_7)$-alkyl, $(C_1–C_7)$-alkyl-phenyl-hydroxy-$(C_1–C_7)$-alkyl, $(C_1–C_7)$-alkoxyphenyl-hydroxy-$(C_1–C_7)$-alkyl, $(C_1–C_7)$-alkoxy-hydroxy-$(C_1–C_7)$-alkyl, $(C_3–C_6)$-cycloalkyl-hydroxy-$(C_1–C_7)$-alkyl, $R_6R_7$>N-$(C_1–C_7)$-alkyl or phenoxy-hydroxy-$(C_1–C_7)$-alkyl optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino and $R_1'$, $R_2$, $R_3$, $R_6$, $R_7$ and X are as described previously, cleaving off the protecting group in a compound of the general formula

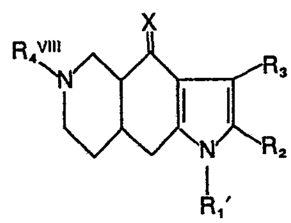

Af'''

wherein $R_4^{VIII}$ has the same significance as $R_4^{VII}$, but in protected form, and $R_1'$, $R_2$, $R_3$ and X are as described previously, or

l) isomerizing the mixture of cis and trans isomers obtained to a final ratio which exhibits predominantly the trans isomer, or

m) separating the trans isomer from the mixture obtained, or

n) resolving a racemic mixture obtained into the optical antipodes, and

o) when desired, converting an obtained compound or a pharmaceutically non-acceptable addition salt into a pharmaceutically acceptable acid addition salt.

2. A process according to claim 1 in which compounds of formula A wherein $R_1$ is hydrogen, $(C_1–C_7)$-alkyl, $(C_1–C_7)$-alkanoyl or benzoyl or phenyl-$(C_1–C_7)$-alkyl each of which is optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino, $R_2$ and $R_3$ independently of each other are hydrogen, $(C_1–C_7)$-alkyl, $(C_3–C_6)$-cycloalkyl, $(C_2–C_7)$-alkenyl or phenyl-$(C_1–C_7)$-alkyl optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino and

$R_4$ is hydrogen, $(C_1–C_7)$-alkyl, hydroxy-$(C_1–C_7)$-alkyl, phenyl-hydroxy-$(C_1–C_7)$-alkyl, halophenyl-hydroxy-$(C_1–C_7)$-alkyl, $(C_1–C_7)$-alkylphenyl-hydroxy-$(C_1–C_7)$-alkyl, $(C_1–C_7)$-alkoxyphenyl-hydroxy-$(C_1–C_7)$-alkyl, $(C_1–C_7)$-alkoxy-$(C_1–C_7)$-alkyl, $(C_1–C_7)$-alkoxy-hydroxy-$(C_1–C_7)$-alkyl, $(C_1–C_7)$-alkanoyloxy-$(C_1–C_7)$-alkyl, $(C_1–C_7)$-alkoxycarbonyl-$(C_1–C_7)$-alkyl, $(C_2–C_7)$-alkenyl, $(C_3–C_6)$-cycloalkyl-$(C_1–C_7)$-alkyl, $(C_2–C_7)$-alkynyl, thienyl-$(C_1–C_7)$-alkyl, furyl-$(C_1–C_7)$-alkyl or phenoxy-hydroxy-$(C_1–C_7)$-alkyl, benzoyloxy-$(C_1–C_7)$-alkyl, phenyl-$(C_1–C_7)$-alkyl or benzoyl-$(C_1–C_7)$-alkyl optionally substituted on the phenylring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino, or 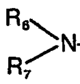N-$(C_1–C_7)$-alkyl and X is O, optical and geometric isomers thereof and pharmaceutically acceptable acid addition salts thereof are manufactured, characterized in that the compounds are manufactured according to embodiments c), l), m), n) and o) in accordance with claim 1 or according to embodiment a) or b) in which a compound of formula IX or X wherein $R_4''$ is methyl or used as the starting material or according to embodiment f) in which a compound of formula Aa wherein $R_4''$ is methyl and X is O is used as the starting material or according to embodiments g), h), i), j) or k) in which a compound of formula Ab, Ae, Ah, Ad' or Af''' wherein X is O is used as the starting material.

3. A process according to claim 1 or 2, characterized in that it is carried out for $R_1$ with the significance hydrogen.

4. A process according to any one of claims 1 to 3, characterized in that it is carried out for $R_2$ and $R_3$ with the significance $(C_1–C_7)$-alkyl.

5. A process according to any one of claims 1 to 4, wherein $R_4$ is $(C_1–C_7)$-alkyl, hydroxy-$(C_1–C_7)$-alkyl, phenyl-hydroxy-$(C_1–C_7)$-alkyl, halophenyl-hydroxy-$(C_1–C_7)$-alkyl, $(C_1–C_7)$-alkoxy-$(C_1–C_7)$-alkyl or phenoxy-$(C_1–C_7)$-alkyl, benzoyl-$(C_1–C_7)$-alkyl or phenyl-$(C_1–C_7)$-alkyl each of which is optionally substituted on the phenyl ring by halogen, trifluoromethyl, $(C_1–C_7)$-alkyl, $(C_1–C_7)$-alkoxy, nitro, amino, $(C_1–C_7)$-alkylamino or di-$(C_1–C_7)$-alkylamino.

6. A process according to any one of claims 1 and 3 to 5, characterized in that it is carried out for X with the significance O.

7. A process according to any one of claims 1 to 6, characterized in that the compound is manufactured in the form of the trans isomer.

8. A process according to claim 2, characterized in that
3-ethyl-2,6-dimethyl-4,4a,5,6,7,8,8a,9-octahydro-4a,8a-trans-1H-pyrrolo[2,3-g]isoquinolin-4-one
is manufactured.

9. A process according to claim 1, characterized in that
2-methyl-3-ethyl-6-(2-phenylethyl)-4,4a,5,6,7,8,-8a,9-octahydro-4a,8a-trans-1H-pyrrolo(2,3-g]-isoquinolin-4-one
is manufactured.

10. A process according to claim 1, characterized in that
2-methyl-3-ethyl-6-(2-ethoxyethyl)-4,4a,5,6,7,8,-8a,9-octahydro-4a,8a-trans-1H-pyrrolo[2,3-g]-isoquinolin-4-one
is manufactured.

11. A process according to claim 1, characterized in that
2-methyl-3-ethyl-6-[4-(4-fluorophenyl)-4-oxobutyl]-4,4a,5,6,7,8,8a,9-octahydro-4a,8a-trans-1H-pyrrolo(2,3-g]isoquinolin-4-one
is manufactured.

12. A process according to claim 1, characterized in that
2-methyl-3-ethyl-6-(2-hydroxy-3,3-dimethylbutyl)-4,4a,5,6,7,8,8a,9-octahydro-4a,8a-trans-1H-pyrrolo[2,3-g]isoquinolin-4-one is manufactured.

**Revendications pour les Etats contractants: BE, CH, DE, FR, GB, IT, LI, LU, NL, SE**

1. Octahydro-1H-pyrrolo[2,3-g]isoquinoléine de formule générale

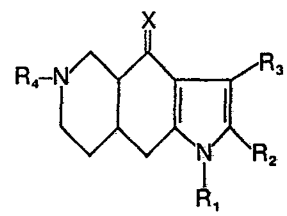

A

dans laquelle $R_1$ représente l'hydrogène, un groupe alkyle en $C_1–C_7$, alcanoyle en $C_1–C_7$ ou un groupe benzoyle ou phényl-(alkyle en $C_1–C_7$), ces deux derniers éventuellement substitués sur le noyau phényle par des halogènes, des groupes trifluorométhyle, alkyle en $C_1–C_7$, alcoxy en $C_1–C_7$, nitro, amino, alkylamino en $C_1–C_7$ ou di-(alkyle en $C_1–C_7$)-amino,
$R_2$ et $R_3$ représentent chacun, indépendamment l'un de l'autre, l'hydrogène, un groupe alkyle en $C_1–C_7$, cycloalkyle en $C_3–C_6$, alcényle en $C_2–C_7$, alcanoyle en $C_1–C_7$ ou un groupe benzoyle, phényle ou phényl-(alkyle en $C_1–C_7$), ces trois derniers éventuellement substitués sur le noyau phényle par des halogènes, des groupes trifluorométhyle, alkyle en $C_1–C_7$, alcoxy en $C_1–C_7$, nitro, amino, alkylamino en $C_1–C_7$ ou di-(alkyle en $C_1–C_7$)-amino et
$R_4$ représente l'hydrogène, un groupe alkyle en $C_1–C_7$, hydroxyalkyle en $C_1–C_7$, phényl-hydroxyalkyl en $C_1–C_7$, halogénophényl-hydroxyalkyle en $C_1–C_7$, (alkyle en $C_1–C_7$)-phényl-hydroxyalkyle en $C_1–C_7$, (alcoxy en $C_1–C_7$)-phényl-hydroxyalkyle en $C_1–C_7$, (alcoxy en $C_1–C_7$)-alkyle en $C_1–C_7$, (alcoxy en $C_1–C_7$)-hydroxyalkyle en $C_1–C_7$, (alcanoyloxy en $C_1–C_7$)-alkyle en $C_1–C_7$, (alcoxy en $C_1–C_7$)-carbonyl-(alkyle en $C_1–C_7$),alcényle en $C_2–C_7$, (cycloalkyle en $C_3–C_6$)-alkyle en $C_1–C_7$, alcynyle en $C_2–C_7$, thiényl-alkyle en $C_1–C_7$, furyl-alkyle en $C_1–C_7$, (alcanoyle en $C_1–C_7$)-alkyle en $C_1–C_7$, (cycloalcoxy en $C_3–C_6$)-alkyle en $C_1–C_7$, (cycloalkyle en $C_3–C_6$)-hydroxy-alkyle en $C_1–C_7$, (alcényloxy en

$C_2$–$C_7$)-alkyle en $C_1$–$C_7$, N-(alkyle en $C_1$–$C_7$)-pyrrolidinyl-alkyle en $C_1$–$C_7$, trifluoralkyle contenant 2 à 6 atomes de carbone ou un groupe phénoxy-hydroxy-alkyle en $C_1$–$C_7$, benzoyloxy-alkyle en $C_1$–$C_7$, benzoyl-alkyle en $C_1$–$C_7$, phényl-alkyle en $C_1$–$C_7$, phénylcarboxamido-alkyle en $C_1$–$C_7$, phényl-alcényle en $C_2$–$C_7$, phénoxy-alkyle en $C_1$–$C_7$ ou phényl-N-imidazolonyl-alkyle en $C_1$–$C_7$, ces derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, ou un groupe 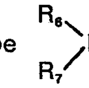N-Alkyle en $C_1$–$C_7$ dans lequel $R_6$ et $R_7$ représentent, indépendamment l'un de l'autre, l'hydrogène ou un groupe alkyle en $C_1$–$C_7$ ou bien forment ensemble et avec l'atome d'azote un hétérocycle saturé à 5 ou 6 chaînons qui peut encore contenir en chaînon un atome d'oxygène ou un atome d'azote éventuellement substitué par un groupe alkyle en $C_1$–$C_7$ ou hydroxyalkyle en $C_1$–$C_7$, et X représente O ou S, son isomère optique ou géométrique ou son sel formé par addition avec un acide acceptable pour l'usage pharmaceutique.

2. Composé selon la rev. 1, dans lequel $R_1$ représente l'hydrogène, un groupe alkyle en $C_1$–$C_7$, alcanoyle en $C_1$–$C_7$ ou un groupe benzoyle ou phényl-(alkyle en $C_1$–$C_7$), ces deux derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, $R_2$ et $R_3$ représentent, indépendamment l'un de l'autre, l'hydrogène, un groupe alkyle en $C_1$–$C_7$, cycloalkyle en $C_3$–$C_6$, alcényle en $C_2$–$C_7$ ou un groupe phényl-(alkyle en $C_1$–$C_7$) éventuellement substitué sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino et $R_4$ représente l'hydrogène, un groupe alkyle en $C_1$–$C_7$, hydroxyalkyle en $C_1$–$C_7$, phényl-hydroxyalkyle en $C_1$–$C_7$, halogénophényl-hydroxy-alkyle en $C_1$–$C_7$, (alkyle en $C_1$–$C_7$)-phényl-hydroxyalkyle en $C_1$–$C_7$, (alcoxy en $C_1$–$C_7$)-phényl-hydroxyalkyle en $C_1$–$C_7$, (alcoxy en $C_1$–$C_7$)-alkyle en $C_1$–$C_7$, (alcoxy en $C_1$–$C_7$)-hydroxyalkyle en $C_1$–$C_7$, (alcanoyloxy en $C_1$–$C_7$)-alkyle en $C_1$–$C_7$, (alcoxy en $C_1$–$C_7$)-carbonyl-(alkyle en $C_1$–$C_7$), alcényle en $C_2$–$C_7$, (cycloalkyle en $C_3$–$C_6$)-alkyle en $C_1$–$C_7$, alcynyle en $C_2$–$C_7$, thiényl-alkyle en $C_1$–$C_7$, furyl-alkyle en $C_1$–$C_7$ ou un groupe phénoxy-hydroxyalkyle en $C_1$–$C_7$, benzoyloxy-alkyle en $C_1$–$C_7$, phényl-alkyle en $C_1$–$C_7$ ou benzoyl-alkyle en $C_1$–$C_7$, ces derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, ou le groupe 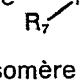N-alkyle en $C_1$–$C_7$ et X représente O, son isomère optique ou son sel formé par addition avec un acide acceptable pour l'usage pharmaceutique.

3. Composé selon la revendication 1 ou 2, dans lequel $R_1$ représente l'hydrogène.

4. Composé selon l'une des rev. 1 à 3, dans lequel $R_2$ et $R_3$ sont des groupes alkyle en $C_1$–$C_7$.

5. Composé selon l'une des rev. 1 à 4, dans lequel $R_4$ représente un groupe alkyle en $C_1$–$C_7$, hydroxyalkyle en $C_1$–$C_7$, phényl-hydroxyalkyle en $C_1$–$C_7$, halogénophényl-hydroxyalkyle en $C_1$–$C_7$, (alcoxy en $C_1$–$C_7$)-alkyle en $C_1$–$C_7$ ou un groupe phénoxy-(alkyle en $C_1$–$C_7$), benzoylalkyle en $C_1$–$C_7$ ou phényl-(alkyle en $C_1$–$C_7$), ces derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino.

6. Composé selon l'une des rev. 1 et 3 à 5, dans lequel X représente O.

7. Composé selon l'une des rev. 1 à 6, consistant en l'isomère trans.

8. La 3-éthyl-2,6-diméthyl-4,4a,5,6,7,8,8a,9-octahydro-4a,8a-trans-1H-pyrrolo[2,3-g]-isoquinoléine-4-one.

9. La 2-méthyl-3-éthyl-6-(2-phényléthyl)-4,4a,5,6,7,8,8a,9-octahydro-4a,8a-trans-1H-pyrrolo[2,3-g]isoquinoléine-4-one.

10. La 2-méthyl-3-éthyl-6-(2-éthoxy-éthyl)-4,4a,5,6,7,8,8a,9-octahydro-4a,8a-trans-1H-pyrrolo[2,3-g]isoquinoléine-4-one.

11. La 2-méthyl-3-éthyl-6-[4-(4-fluorophényl)-4-oxobutyl]-4,4a,5,6,7,8,8a,9-octahydro-4a,8a-trans-1H-pyrrolo-[2,3-g]isoquinoléine-4-one.

12. La 3-éthyl-2-méthyl-6,8a,9-octahydro-4a,8a,-trans-1H-pyrrolo[2,3-g]isoquinoléine-4-one.

11. La 2-méthyl-3-éthyl-6-[4-(4-fluorophényl)-4-oxobutyl]-4,4a,5,6,7,8,8a,9-octahydro-4a,8a-trans-1H-pyrrolo-[2,3-g]isoquinoléine-4-one.

12. La 3-éthyl-2-méthyl-6-(2-hydroxy-3,3-diméthylbutyl)-4,4a,5,6,7,8,8a,9-octahydro-1H-pyrrolo-[2,3-g]isoquinoléine-4-one.

13. Composé selon la rev. 1, dans lequel $R_1$ représente l'hydrogène, $R_2$ représente un groupe méthyle, $R_3$ un groupe éthyle, X représente O et $R_4$ représente un groupe 2-hydroxy-2-phényléthyle, benzyle, 3-(4-fluorophényl)-3-oxopropyle, 3-phénoxypropyle, 2-hydroxy-3-méthylbutyle, 2-hydroxyéthyle, 2-propényle, cyclopropylméthyle, éthyle, 2-oxo-2-(4-fluorophényl)-éthyle.

14. Composé selon la rev. 1, dans lequel $R_1$ représente l'hydrogène, $R_2$ et $R_3$ des groupes méthyle, X représente O et $R_4$ représente un groupe méthyle, 2-phényl-éthyle, 4-(4-fluorophényl)-4-oxobutyle.

15. Une octahydro-1H-pyrrolo[2,3-g]isoquinoléine selon l'une des rev. 1 à 14, en tant que substance active pharmaceutique.

16. Une octahydro-1H-pyrrolo[2,3-g]isoquinoléine selon l'une des rev. 1 à 14, en tant que substance active neuroleptique/antipsychotique.

17. La 3-éthyl-2,6-diméthyl-4,4a,5,6,7,8,8a,9-octahydro-4a,8a-trans-1H-pyrrolo-[2,3-g]isoquinoléine-4-one, en tant que substance active pharmaceutique.

18. La 3-éthyl-2,6-diméthyl-4,4a,5,6,7,8,8a,9-octahydro-4a,8a-trans-1H-pyrrolo-[2,3-g]isoquinoléine-4-one, en tant que substance active neuroleptique/antipsychotique.

19. Procédé de préparation des composés de formule A de la rev. 1, de leurs isomères optiques et géométriques et de leurs sels formés par addition avec des acides acceptables pour l'usage pharmaceutique, caractérisé en ce que:

a) pour la préparation d'un composé de formule générale

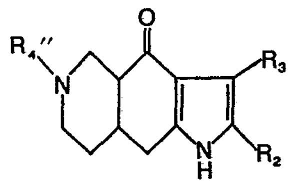

Ia,

dans laquelle R''$_4$ représente un groupe alkyle en $C_1$–$C_7$, (alcoxy en $C_1$–$C_7$)-alkyle en $C_1$–$C_7$ ou (cycloalkyle en $C_3$–$C_6$)-alkyle en $C_1$–$C_7$ et $R_2$ et $R_3$ ont les significations indiquées ci-dessus, on traite un composé de formule générale

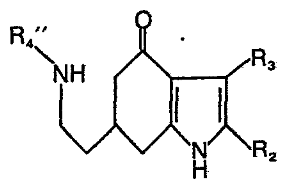

IX,

dans laquelle $R_2$, $R_3$ et R''$_4$ ont les significations indiquées ci-dessus, par le formaldéhyde, ou bien

b) pour la préparation d'un composé de formule Ia ci-dessus, on traite un composé de formule générale

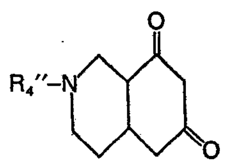

XII,

dans laquelle R''$_4$ a la signification indiquée ci-dessus, par un composé de formule générale

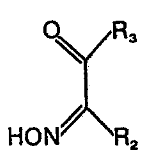

VII

en présence d'un agent réducteur, ou par un composé de formule générale

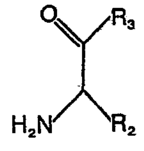

VIII,

dans laquelle $R_2$ et $R_3$ ont les significations indiquées ci-dessus, ou un précurseur de ce composé, ou bien

c) pour la préparation d'un composé de formule générale

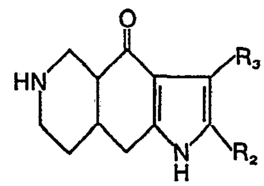

Ib

dans laquelle $R_2$ et $R_3$ ont les significations indiquées ci-dessus, on soumet à N-déméthylation un composé de formule Ia ci-dessus dans laquelle R''$_4$ représente le groupe méthyle, ou bien

d) pour la préparation d'un composé de formule générale

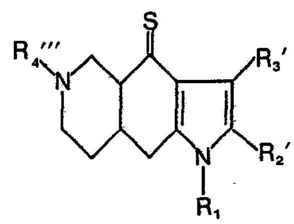

IIc'',

dans laquelle R'$_2$ et R'$_3$ représentent, indépendamment l'un de l'autre, l'hydrogène, un groupe alkyle en $C_1$–$C_7$, cycloalkyle en $C_3$–$C_6$, alcényle en $C_2$–$C_7$ ou un groupe phényle ou phényl-alkyle en $C_1$–$C_7$, ces deux derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, R'''$_4$ représente l'hydrogène, un groupe alkyle en $C_1$–$C_7$, (alcoxy en $C_1$–$C_7$)-alkyle en $C_1$–$C_7$, alcényle en $C_2$–$C_7$, (cycloalkyle en $C_3$–$C_6$)-alkyle en $C_1$–$C_7$, alcynyle en $C_2$–$C_7$, thiényl-alkyle en $C_1$–$C_7$, furyl-alkyle en $C_1$–$C_7$, (alcényloxy en $C_2$–$C_7$)-alkyle en $C_1$–$C_7$ ou un groupe phényl-alkyle en $C_1$–$C_7$, phényl-alcényle en $C_2$–$C_7$, ou phénoxy-alkyle en $C_1$–$C_7$, ces derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, ou un groupe trifluoralkyle en $C_2$–$C_6$, et $R_1$ a la signification indiquée ci-dessus, on traite un composé de formule générale

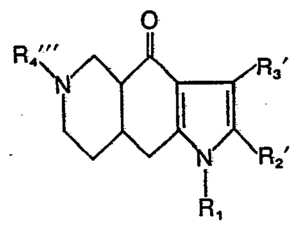

Ic'',

dans laquelle $R_1$, R'$_2$, R'$_3$ et R'''$_4$ ont les significations indiquées ci-dessus, par le pentasulfure de phosphore, ou bien

e) pour la préparation d'un composé de formule générale

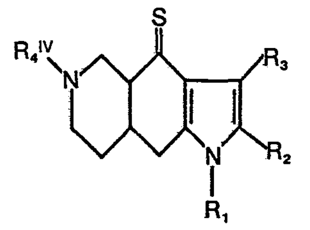

IIc''',

dans laquelle $R_4^{IV}$ représente un groupe hydroxyalkyle en $C_1$–$C_7$, phényl-hydroxyalkyle en $C_1$–$C_7$, halogénophényl-hydroxyalkyle en $C_1$–$C_7$, (alkyle en $C_1$–$C_7$)-phényl-hydroxyalkyle en $C_1$–$C_7$, (alcoxy en $C_1$–$C_7$)-phényl-hydroxyalkyle en $C_1$–$C_7$, (alcoxy en $C_1$–$C_7$)-hydroxyalkyle en $C_1$–$C_7$, (alcanoyloxy en $C_1$–$C_7$)-alkyle en $C_1$–$C_7$, (alcoxy en $C_1$–$C_7$)-carbonyl-(alkyle en $C_1$–$C_7$), (alcanoyle en $C_1$–$C_7$)-alkyle en $C_1$–$C_7$, (cycloalcoxy en $C_3$–$C_6$)-alkyle en $C_1$–$C_7$, (cycloalkyle en $C_3$–$C_6$)-hydroxyalkyle en $C_1$–$C_7$, N-(alkyle en $C_1$–$C_7$)-pyrrolidinyl-(alkyle en $C_1$–$C_7$) ou un groupe phényl-N-imidazolonyl-alkyle en $C_1$–$C_7$, phénoxy-hydroxyalkyle en $C_1$–$C_7$, benzoyloxy-alkyle en $C_1$–$C_7$, benzoyl-alkyle en $C_1$–$C_7$ ou phénylcarboxamido-alkyle en $C_1$–$C_7$, ces derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, ou bien un groupe 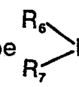N-alkyle en $C_1$–$C_7$, et $R_1$, $R_2$, $R_3$, $R_6$ et $R_7$ ont les significations indiquées précédemment, on élimine le ou les groupes protecteurs d'un composé de formule générale

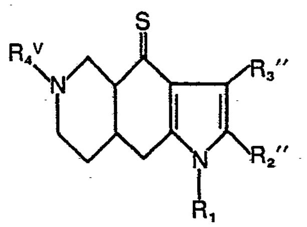

IIf,

dans laquelle $R''_2$ et $R''_3$ ont les mêmes significations que $R_2$ et $R_3$, sauf que lorsque $R_2$ et $R_3$ représentent des groupes alcanoyle en $C_1$–$C_7$ ou benzoyle, ce dernier éventuellement substitué sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, $R''_2$ et $R''_3$ sont à l'état protégé, $R_4^V$ a la même signification que $R_4^{IV}$ mais est à l'état protégé, et $R_1$ a la signification indiquée précédemment, ou bien

f) pour la préparation d'un composé de formule générale

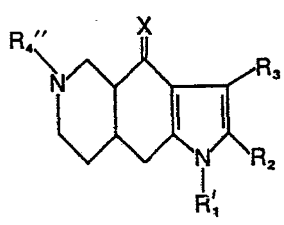

Ac,

dans laquelle $R'_1$ représente un groupe alkyle en $C_1$–$C_7$, alcanoyle en $C_1$–$C_7$ ou un groupe benzoyle ou phényl-alkyle en $C_1$–$C_7$, ces deux derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, et $R_2$, $R_3$, $R''_4$ et X ont les significations indiquées précédemment, on soumet un composé de formule générale

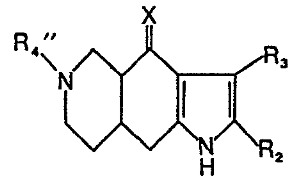

Aa,

dans laquelle $R_2$, $R_3$, $R''_4$ et X ont les significations indiquées précédemment, à substitution sur l'atome d'azote pyrrolique, ou bien

g) pour la préparation d'un composé de formule générale

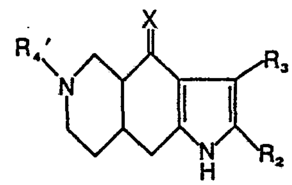

Ad,

dans laquelle $R'_4$ représente un groupe alkyle en $C_1$–$C_7$, hydroxyalkyle en $C_1$–$C_7$, phényl-hydroxyalkyle en $C_1$–$C_7$, halogénophényl-hydroxyalkyle en $C_1$–$C_7$, (alkyle en $C_1$–$C_7$)-phényl-hydroxyalkyle en $C_1$–$C_7$, (alcoxy en $C_1$–$C_7$)-phényl-hydroxyalkyle en $C_1$–$C_7$, (alcoxy en $C_1$–$C_7$)-alkyle en $C_1$–$C_7$, (alcoxy en $C_1$–$C_7$)-hydroxyalkyle en $C_1$–$C_7$, (alcanoyloxy en $C_1$–$C_7$)-alkyle en $C_1$–$C_7$, (alcoxy en $C_1$–$C_7$)-carbonyl-(alkyle en $C_1$–$C_7$), alcényle en $C_2$–$C_7$, (cycloalkyle en $C_3$–$C_6$)-alkyle en $C_1$–$C_7$, alcynyle en $C_2$–$C_7$, thiényl-alkyle en $C_1$–$C_7$, furyl-alkyle en $C_1$–$C_7$, (alcanoyle en $C_1$–$C_7$)-alkyle en $C_1$–$C_7$, (cycloalcoxy en $C_3$–$C_6$)-alkyle en $C_1$–$C_7$, (cycloalkyle en $C_3$–$C_6$)-hydroxyalkyle en $C_1$–$C_7$, (alcényloxy en $C_2$–$C_7$)-alkyle en $C_1$–$C_7$, N-(alkyle en $C_1$–$C_7$)-pyrrolidinyl-alkyle en $C_1$–$C_7$, trifluoralkyle contenant 2 à 6 atomes de carbone, ou un groupe phénoxy-hydroxyalkyle en $C_1$–$C_7$, benzoyloxy-alkyle en $C_1$–$C_7$, benzoyl-alkyle en $C_1$–$C_7$, phényl-alkyle en $C_1$–$C_7$, phénylcarboxamido-alkyle en $C_1$–$C_7$, phényl-alcényle en $C_2$–$C_7$, phénoxy-alkyle en $C_1$–$C_7$ ou phényl-N-imidazolonyl-alkyle en $C_1$–$C_7$, ces derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, ou un groupe 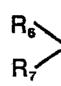N-alkyle en

$C_1$–$C_7$, et $R_2$, $R_3$, $R_6$, $R_7$ et X ont les significations indiquées précédemment, on soumet un composé de formule générale

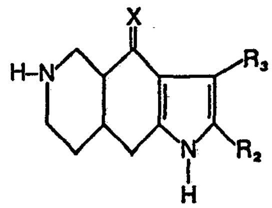

Ab,

dans laquelle $R_2$, $R_3$ et X ont les significations indiquées précédemment, à substitution sur l'atome d'azote d'isoquinoléine, ou bien

h) pour la préparation d'un composé de formule générale

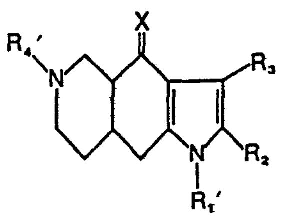

Af,

dans laquelle $R'_1$, $R_2$, $R_3$, $R'_4$ et X ont les significations indiquées précédemment, on soumet un composé de formule Ae ci-dessous à substitution sur l'atome d'azote d'isoquinoléine, ou bien

i) pour la préparation d'un composé de formule générale

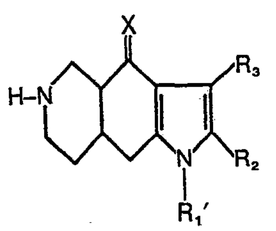

Ae,

dans laquelle $R'_1$, $R_2$, $R_3$ et X ont les significations indiquées précédemment, on élimine le groupe protecteur d'un composé de formule générale

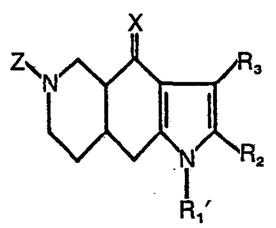

Ah,

dans laquelle Z représente un groupe protecteur et $R'_1$, $R_2$, $R_3$ et X ont les significations indiquées précédemment, ou bien

j) pour la préparation d'un composé de formule générale

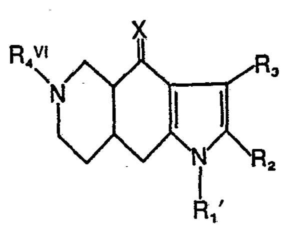

Af',

dans laquelle $R_4^{VI}$ représente l'hydrogène, un groupe alkyle en $C_1$–$C_7$, (alcoxy en $C_1$–$C_7$)-alkyle en $C_1$–$C_7$, (alcanoyloxy en $C_1$–$C_7$)-alkyle en $C_1$–$C_7$, (alcoxy en $C_1$–$C_7$)-carbonyl-(alkyle en $C_1$–$C_7$), alcényle en $C_2$–$C_7$, (cycloalkyle en $C_3$–$C_6$)-alkyle en $C_1$–$C_7$, alcynyle en $C_2$–$C_7$, thiényl-alkyle en $C_1$–$C_7$, furyl-alkyle en $C_1$–$C_7$, (alcanoyle en $C_1$–$C_7$)-alkyle en $C_1$–$C_7$, (cycloalcoxy en $C_3$–$C_6$)-alkyle en $C_1$–$C_7$, (alcényloxy en $C_2$–$C_7$)-alkyle en $C_1$–$C_7$, N-(alkyle en $C_1$–$C_7$)-pyrrolidinyl-alkyle en $C_1$–$C_7$, trifluoralkyle contenant 2 à 6 atomes de carbone ou un groupe phényl-N-imidazolonyl-alkyle en $C_1$–$C_7$, benzoyloxy-alkyle en $C_1$–$C_7$, phényl-alkyle en $C_1$–$C_7$, phénylcarboxamido-alkyle en $C_1$–$C_7$, benzoyl-alkyle en $C_1$–$C_7$, phényl-alcényle en $C_2$–$C_7$ ou phénoxy-alkyle en $C_1$–$C_7$, ces derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, et $R'_1$, $R_2$, $R_3$ et X ont les significations indiquées précédemment, on soumet un composé de formule générale

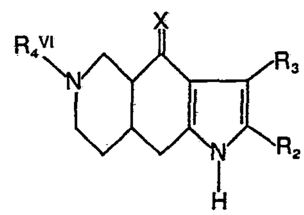

Ad',

dans laquelle $R_2$, $R_3$, $R_4^{VI}$ et X ont les significations indiquées précédemment, à substitution sur l'atome d'azote de pyrrole, ou bien

k) pour la préparation d'un composé de formule générale

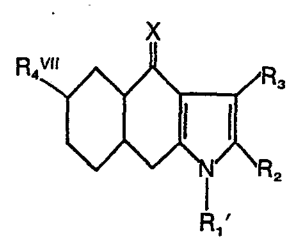

Af''

dans laquelle $R_4^{VII}$ représente un groupe hydroxyalkyle en $C_1$–$C_7$, phényl-hydroxyalkyle en $C_1$–$C_7$, halogénophényl-hydroxyalkyle en $C_1$–$C_7$, (alkyle en $C_1$–$C_7$)-phényl-hydroxyalkyle en $C_1$–$C_7$, (alcoxy en $C_1$–$C_7$)-phényl-hydroxyalkyle en $C_1$–$C_7$, (alcoxy en $C_1$–$C_7$)-hydroxyalkyle en $C_1$–$C_7$, (cycloalkyle en $C_3$–$C_6$)-hydroxyalkyle en $C_1$–$C_7$,

$\begin{matrix}R_6\\R_7\end{matrix}\!>$N-alkyle en $C_1$–$C_7$, ou un groupe phénoxy-

hydroxyalkyle en $C_1$–$C_7$, éventuellement substitué sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, et $R'_1$, $R_2$, $R_3$, $R_6$, $R_7$ et X ont les significations indiquées précédemment,

on élimine le groupe protecteur dans un composé de formule générale

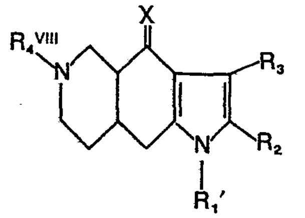

Af'''

dans laquelle $R_4^{VIII}$ a la même signification que $R_4^{VII}$, mais est à l'état protégé, et $R'_1$, $R_2$, $R_3$ et X ont les significations indiquées précédemment, ou bien

l) on porte le mélange d'isomères cis et trans obtenu, par isomérisation, à un rapport final dans lequel l'isomère trans est prépondérant, ou bien

m) on sépare l'isomère trans du mélange obtenu, ou bien

n) on résout un mélange racémique obtenu en les antipodes optiques, et

o) si on le désire, on convertit un composé obtenu ou un sel d'acide non acceptable pour l'usage pharmaceutique en un sel d'acide acceptable pour l'usage pharmaceutique.

20. Médicament contenant une octahydro-1H-pyrrolo[2,3-g]isoquinoléine de formule A de la rev. 1, un isomère optique ou géométrique de ce composé, ou un sel de ce composé formé par addition avec un acide acceptable pour l'usage pharmaceutique.

21. Médicament neuroleptique/antipsychotique contenant une octahydro-1H-pyrrolo[2,3-g]isoquinoléine de formule A de la rev. 1, un isomère optique ou géométrique de ce composé ou un sel de ce composé formé par addition avec un acide acceptable pour l'usage pharmaceutique.

22. Médicament contenant de la 3-éthyl-2,6-diméthyl-4,4a,5,6,7,8,8a,9-octahydro-4a,8a-trans-1H-pyrrolo[2,3-g]isoquinoléine-4-one.

23. Médicament neuroleptique/antipsychotique contenant de la 3-éthyl-2,6-diméthyl-4,4a,5,6,7,8,8a,9-octahydro-4a,8a-trans-1H-pyrrolo[2,3-g]isoquinoléine-4-one.

**Revendications pour l'Etat contractant: AT**

1. Procédé de préparation d'octahydro-1H-pyrrolo[2,3-g]isoquinoléines de formule générale

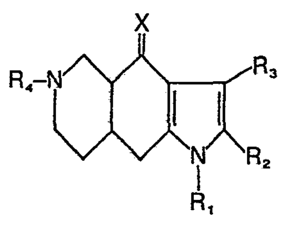

A

dans laquelle $R_1$ représente l'hydrogène, un groupe alkyle en $C_1$–$C_7$, alcanoyle en $C_1$–$C_7$ ou un groupe benzoyle ou phényl-(alkyle en $C_1$–$C_7$), ces deux derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino,

$R_2$ et $R_3$ représentent chacun, indépendamment l'un de l'autre, l'hydrogène, un groupe alkyle en $C_1$–$C_7$, cycloalkyle en $C_3$–$C_6$, alcényle en $C_2$–$C_7$, alcanoyle en $C_1$–$C_7$ ou un groupe benzoyle, phényle ou phényl-(alkyle en $C_1$–$C_7$), ces trois derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino et

$R_4$ représente l'hydrogène, un groupe alkyle en $C_1$–$C_7$, hydroxyalkyle en $C_1$–$C_7$, phényl-hydroxyalkyle en $C_1$–$C_7$, halogénophényl-hydroxyalkyle en $C_1$–$C_7$, (alkyle en $C_1$–$C_7$)-phényl-hydroxyalkyle en $C_1$–$C_7$, (alcoxy en $C_1$–$C_7$)-phényl-hydroxyalkyle en $C_1$–$C_7$, (alcoxy en $C_1$–$C_7$)-alkyle en $C_1$–$C_7$, (alcoxy en $C_1$–$C_7$)-hydroxyalkyle en $C_1$–$C_7$, (alcanoyloxy en $C_1$–$C_7$)-alkyle en $C_1$–$C_7$, (alcoxy en $C_1$–$C_7$)-carbonyl-(alkyle en $C_1$–$C_7$), alcényle en $C_2$–$C_7$, (cycloalkyle en $C_3$–$C_6$)-alkyle en $C_1$–$C_7$, alcynyle en $C_2$–$C_7$, thiényl-alkyle en $C_1$–$C_7$, furyl-alkyle en $C_1$–$C_7$, (alcanoyle en $C_1$–$C_7$)-alkyle en $C_1$–$C_7$, (cycloalcoxy en $C_3$–$C_6$)-alkyle en $C_1$–$C_7$, (cycloalkyle en $C_3$–$C_6$)-hydroxy-alkyle en $C_1$–$C_7$, (alcényloxy en $C_2$–$C_7$)-alkyle en $C_1$–$C_7$, N-(alkyle en $C_1$–$C_7$)-pyrrolidinyl-alkyle en $C_1$–$C_7$, trifluoralkyle contenant 2 à 6 atomes de carbone ou un groupe phénoxy-hydroxy-alkyle en $C_1$–$C_7$, benzoyloxy-alkyle en $C_1$–$C_7$, benzoyl-alkyle en $C_1$–$C_7$, phényl-alkyle en $C_1$–$C_7$, phénylcarboxamido-alkyle en $C_1$–$C_7$, phénylalcényle en $C_2$–$C_7$, phénoxy-alkyle en $C_1$–$C_7$ ou phényl-N-imidazolonyl-alkyle en $C_1$–$C_7$, ces derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, ou un groupe $R_6R_7$N-alkyle en $C_1$–$C_7$ dans lequel $R_6$ et $R_7$ représentent indépendamment l'un de l'autre, l'hydrogène ou un groupe alkyle en $C_1$–$C_7$, ou bien forment ensemble et avec l'atome d'azote un hétérocycle saturé à 5 ou 6 chaînons qui peut encore contenir en chaînon un atome d'oxygène ou un atome d'azote éventuellement substitué par un groupe alkyle en $C_1$–$C_7$ ou hydroxyalkyle en $C_1$–$C_7$, et X représente O ou S, de leurs isomères optiques et géométriques et de leurs sels formés par addition avec des acides acceptables pour l'usage pharmaceutique, caractérisé en ce que:

a) pour la préparation d'un composé de formule générale

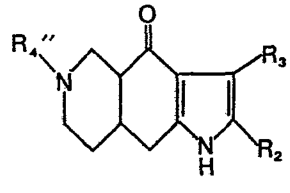

Ia,

dans laquelle $R''_4$ représente un groupe alkyle en $C_1$–$C_7$, (alcoxy en $C_1$–$C_7$)-alkyle en $C_1$–$C_7$ ou (cy-

cloalkyle en $C_3$–$C_6$)-alkyle en $C_1$–$C_7$ et $R_2$ et $R_3$ ont les significations indiquées ci-dessus, on traite un composé de formule générale

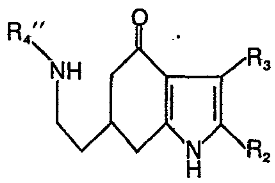

IX,

dans laquelle $R_2$, $R_3$ et $R''_4$ ont les significations indiquées ci-dessus, par le formaldéhyde, ou bien

b) pour la préparation d'un composé de formule Ia ci-dessus, on traite un composé de formule générale

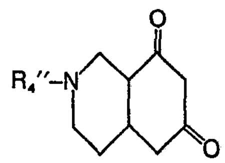

XII,

dans laquelle $R''_4$ a la signification indiquée ci-dessus, par un composé de formule générale

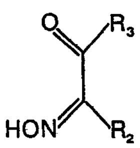

VII

en présence d'un agent réducteur, ou par un composé de formule générale

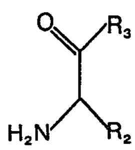

VIII,

dans laquelle $R_2$ et $R_3$ ont les significations indiquées ci-dessus, ou un précurseur de ce composé, ou bien

c) pour la préparation d'un composé de formule générale

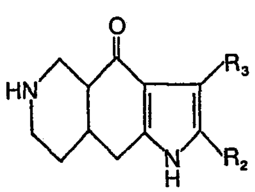

Ib

dans laquelle $R_2$ et $R_3$ ont les significations indiquées ci-dessus, on soumet à N-déméthylation un composé de formule Ia ci-dessus dans laquelle $R''_4$ représente le groupe méthyle, ou bien

d) pour la préparation d'un composé de formule générale

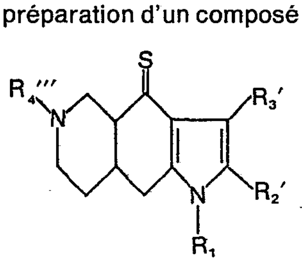

IIc'',

dans laquelle $R'_2$ et $R'_3$ représentent, indépendamment l'un de l'autre, l'hydrogène, un groupe alkyle en $C_1$–$C_7$, cycloalkyle en $C_3$–$C_6$, alcényle en $C_2$–$C_7$ ou un groupe phényle ou phényl-alkyle en $C_1$–$C_7$, ces deux derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, $R'''_4$ représente l'hydrogène, un groupe alkyle en $C_1$–$C_7$, (alcoxy en $C_1$–$C_7$)-alkyle en $C_1$–$C_7$, alcényle en $C_2$–$C_7$, (cycloalkyle en $C_3$–$C_6$)-alkyle en $C_1$–$C_7$, alcynyle en $C_2$–$C_7$, thiényl-alkyle en $C_1$–$C_7$, furyl-alkyle en $C_1$–$C_7$, (alcényloxy en $C_2$–$C_7$)-alkyle en $C_1$–$C_7$ ou un groupe phényl-alkyle en $C_1$–$C_7$, phényl-alcényle en $C_2$–$C_7$, ou phénoxy-alkyle en $C_1$–$C_7$, ces derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, ou un groupe trifluoralkyle en $C_2$–$C_6$, et $R_1$ a la signification indiquée ci-dessus, on traite un composé de formule générale

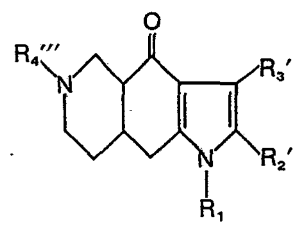

Ic'',

dans laquelle $R_1$, $R'_2$, $R'_3$ et $R'''_4$ ont les significations indiquées ci-dessus, par le pentasulfure de phosphore, ou bien

e) pour la préparation d'un composé de formule générale

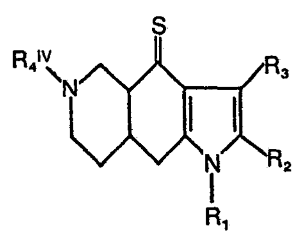

IIc''',

dans laquelle $R_4^{IV}$ représente un groupe hydroxyalkyle en $C_1$–$C_7$, phényl-hydroxyalkyle en $C_1$–$C_7$, halogénophényl-hydroxyalkyle en $C_1$–$C_7$, (alkyle en $C_1$–$C_7$)-phényl-hydroxyalkyle en $C_1$–$C_7$, (alcoxy en $C_1$–$C_7$)-phényl-hydroxyalkyle en $C_1$–$C_7$, (alcoxy en $C_1$–$C_7$)-hydroxyalkyle en $C_1$–$C_7$, (alcanoyloxy en $C_1$–$C_7$)-alkyle en $C_1$–$C_7$, (alcoxy en $C_1$–$C_7$)-carbonyl-(alkyle en $C_1$–$C_7$), (alcanoyle en $C_1$–$C_7$)-alkyle en $C_1$–$C_7$, (cycloalcoxy en $C_3$–$C_6$)-alkyle en $C_1$–$C_7$, (cycloalkyle en $C_3$–$C_6$)-hydroxyalkyle en $C_1$–$C_7$, N-(alkyle en $C_1$–$C_7$)-pyrrolidinyl-(alkyle en $C_1$–$C_7$) ou un groupe phényl-N-imidazolonyl-alkyle en $C_1$–$C_7$, phénoxy-hydroxy-alkyle en $C_1$–$C_7$, benzoyloxy-alkyle en $C_1$–$C_7$, benzoyl-alkyle en $C_1$–$C_7$ ou phénylcarboxamido-alkyle en $C_1$–$C_7$, ces derniers éventuellement substitués sur le noyau phényle par des halogènes, des groupes

trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, ou bien un groupe 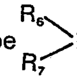 N-alkyle en $C_1$–$C_7$, et $R_1$, $R_2$, $R_3$, $R_6$ et $R_7$ ont les significations indiquées précédemment, on élimine le ou les groupes protecteurs d'un composé de formule générale

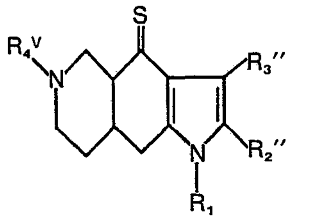

IIf,

dans laquelle $R''_2$ et $R''_3$ ont les mêmes significations que $R_2$ et $R_3$, sauf que lorsque $R_2$ et $R_3$ représentent des groupes alcanoyle en $C_1$–$C_7$ ou benzoyle, ce cernier éventuellement substitué sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, $R''_2$ et $R''_3$ sont à l'état protégé, $R_4^V$ a la même signification que $R_4^{IV}$ mais est à l'état protégé, et $R_1$ a la signification indiquée précédemment, ou bien

f) pour la préparation d'un composé de formule générale

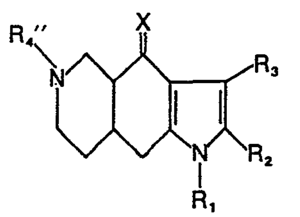

Ac,

dans laquelle $R'_1$ représente un groupe alkyle en $C_1$–$C_7$, alcanoyle en $C_1$–$C_7$ ou un groupe benzoyle ou phényl-alkyle en $C_1$–$C_7$, ces deux derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, et $R_2$, $R_3$, $R''_4$ et X ont les significations indiquées précédemment, on soumet un composé de formule générale

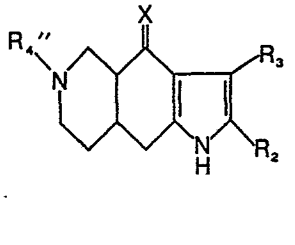

Aa,

dans laquelle $R_2$, $R_3$, $R''_4$ et X ont les significations indiquées précédemment, à substitution sur l'atome d'azote pyrrolique, ou bien

g) pour la préparation d'un composé de formule générale

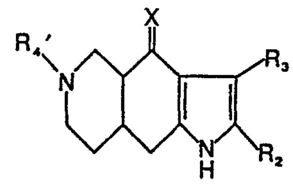

Ad,

dans laquelle $R'_4$ représente un groupe alkyle en $C_1$–$C_7$, hydroxyalkyle en $C_1$–$C_7$, phényl-hydroxyalkyle en $C_1$–$C_7$, halogénophényl-hydroxyalkyle en $C_1$–$C_7$, (alkyle en $C_1$–$C_7$)-phényl-hydroxyalkyle en $C_1$–$C_7$, (alcoxy en $C_1$–$C_7$)-phényl-hydroxyalkyle en $C_1$–$C_7$, (alcoxy en $C_1$–$C_7$)-alkyle en $C_1$–$C_7$, (alcoxy en $C_1$–$C_7$)-hydroxyalkyle en $C_1$–$C_7$, (alcanoyloxy en $C_1$–$C_7$)-alkyle en $C_1$–$C_7$, (alcoxy en $C_1$–$C_7$)-carbonyl-(alkyle en $C_1$–$C_7$), alcényle en $C_2$–$C_7$, (cycloalkyle en $C_3$–$C_6$)-alkyle en $C_1$–$C_7$, alcynyle en $C_2$–$C_7$, thiényl-alkyle en $C_1$–$C_7$, furyl-alkyle en $C_1$–$C_7$, (alcanoyle en $C_1$–$C_7$)-alkyle en $C_1$–$C_7$, (cycloalcoxy en $C_3$–$C_6$)-alkyle en $C_1$–$C_7$, (cycloalkyle en $C_3$–$C_6$)-hydroxyalkyle en $C_1$–$C_7$, (alcényloxy en $C_2$–$C_7$)-alkyle en $C_1$–$C_7$, N-(alkyle en $C_1$–$C_7$)-pyrrolidinyl-alkyle en $C_1$–$C_7$, trifluoralkyle contenant 2 à 6 atomes de carbone, ou un groupe phénoxy-hydroxyalkyle en $C_1$–$C_7$, benzoyloxy-alkyle en $C_1$–$C_7$, benzoyl-alkyle en $C_1$–$C_7$, phényl-alkyle en $C_1$–$C_7$, phénylcarboxamido-alkyle en $C_1$–$C_7$, phényl-alcényle en $C_2$–$C_7$, phénoxy-alkyle en $C_1$–$C_7$ ou phényl-N-imidazolonyl-alkyle en $C_1$–$C_7$, ces derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, ou un groupe 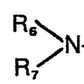 N-alkyle en $C_1$–$C_7$, et $R_2$, $R_3$, $R_6$, $R_7$ et X ont les significations indiquées précédemment, on soumet un composé de formule générale

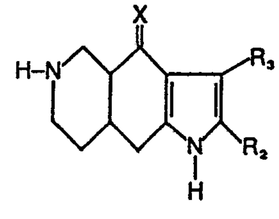

Ab,

dans laquelle $R_2$, $R_3$ et X ont les significations indiquées précédemment, à substitution sur l'atome d'azote d'isoquinoléine, ou bien

h) pour la préparation d'un composé de formule générale

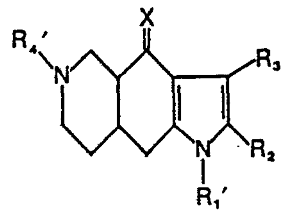

Af,

dans laquelle $R'_1$, $R_2$, $R_3$, $R'_4$ et X ont les significations indiquées précédemment, on soumet un composé de formule Ae ci-dessous à substitution sur l'atome d'azote d'isoquinoléine, ou bien

i) pour la préparation d'un composé de formule générale

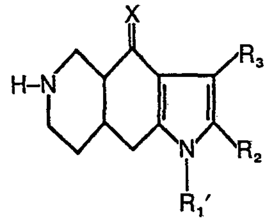

Ae,

dans laquelle $R'_1$, $R_2$, $R_3$ et X ont les significations indiquées précédemment, on élimine le groupe protecteur d'un composé de formule générale

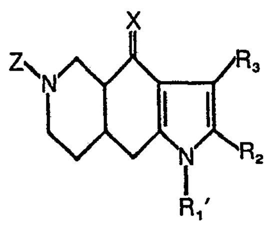

Ah,

dans laquelle Z représente un groupe protecteur et $R'_1$, $R_2$, $R_3$ et X ont les significations indiquées précédemment, ou bien

j) pour la préparation d'un composé de formule générale

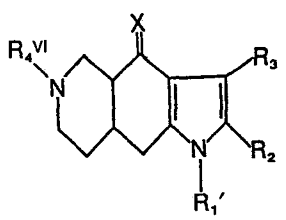

Af',

dans laquelle $R_4^{VI}$ représente l'hydrogène, un groupe alkyle en $C_1$–$C_7$, (alcoxy en $C_1$–$C_7$)-alkyle en $C_1$–$C_7$, (alcanoyloxy en $C_1$–$C_7$)-alkyle en $C_1$–$C_7$, (alcoxy en $C_1$–$C_7$)-carbonyl-(alkyle en $C_1$–$C_7$)-alcényle en $C_2$–$C_7$, (cycloalkyle en $C_3$–$C_6$)-alkyle en $C_1$–$C_7$, alcynyle en $C_2$–$C_7$, thiényl-alkyle en $C_1$–$C_7$, furyl-alkyle en $C_1$–$C_7$, (alcanoyle en $C_1$–$C_7$)-alkyle en $C_1$–$C_7$, (cycloalcoxy en $C_3$–$C_6$)-alkyle en $C_1$–$C_7$, (alcényloxy en $C_2$–$C_7$)-alkyle en $C_1$–$C_7$, N-(alkyle en $C_1$–$C_7$)-pyrrolidinyl-alkyle en $C_1$–$C_7$, trifluoralkyle contenant 2 à 6 atomes de carbone ou un groupe phényl-N-imidazolonyl-alkyle en $C_1$–$C_7$, benzoyloxy-alkyle en $C_1$–$C_7$, phényl-alkyle en $C_1$–$C_7$, phénylcarboxamido-alkyle en $C_1$–$C_7$, benzoyl-alkyle en $C_1$–$C_7$, phényl-alcényle en $C_2$–$C_7$ ou phénoxy-alkyle en $C_1$–$C_7$, ces derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, et $R'_1$, $R_2$, $R_3$ et X ont les significations indiquées précédemment, on soumet un composé de formule générale

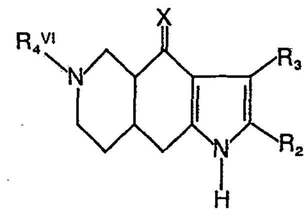

Ad',

dans laquelle $R_2$, $R_3$, $R_4^{VI}$ et X ont les significations indiquées précédemment, à substitution sur l'atome d'azote de pyrrole, ou bien

k) pour la préparation d'un composé de formule générale

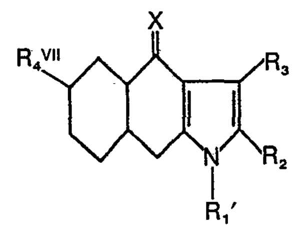

Af''

dans laquelle $R_4^{VII}$ représente un groupe hydroxyalkyle en $C_1$–$C_7$, phényl-hydroxyalkyle en $C_1$–$C_7$, halogénophényl-hydroxyalkyle en $C_1$–$C_7$, (alkyle en $C_1$–$C_7$)-phényl-hydroxyalkyle en $C_1$–$C_7$, (alcoxy en $C_1$–$C_7$)-phényl-hydroxyalkyle en $C_1$–$C_7$, (alcoxy en $C_1$–$C_7$)-hydroxyalkyle en $C_1$–$C_7$, (cycloalkyle en $C_3$–$C_6$)-hydroxyalkyle en $C_1$–$C_7$, $\genfrac{}{}{0pt}{}{R_6}{R_7}\!\!>$N-Alkyle en $C_1$–$C_7$, ou un groupe phénoxy-hydroxyalkyle en $C_1$–$C_7$, éventuellement substitué sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, et $R'_1$, $R_2$, $R_3$, $R_6$, $R_7$ et X ont les significations indiquées précédemment, on élimine le groupe protecteur dans un composé de formule générale

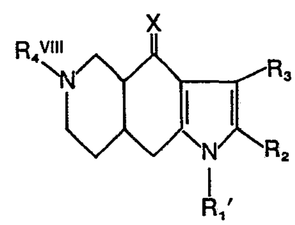

Af'''

dans laquelle $R_4^{VIII}$ a la même signification que $R_4^{VII}$, mais est à l'état protégé, et $R'_1$, $R_2$, $R_3$ et X ont les significations indiquées précédemment, ou bien

l) on porte le mélange d'isomères cis et trans obtenu, par isomérisation, à un rapport final dans lequel l'isomère trans est prépondérant, ou bien

m) on sépare l'isomère trans du mélange obtenu, ou bien

n) on résout un mélange racémique obtenu en les antipodes optiques, et

o) si on le désire, on convertit un composé obtenu ou un sel d'acide non acceptable pour l'usage pharmaceutique en un sel d'acide acceptable pour l'usage pharmaceutique.

2. Procédé selon la rev. 1, dans lequel on prépare les composés de formule A dans laquelle $R_1$ représente l'hydrogène, un groupe alkyle en $C_1$–$C_7$, alcanoyle en $C_1$–$C_7$ ou un groupe benzoyle ou phényl-alkyle en $C_1$–$C_7$, ces deux derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, $R_2$ et $R_3$ représentent, indépendamment l'un de l'autre, l'hydrogène, un groupe alkyle en $C_1$–$C_7$, cycloalkyle en $C_3$–$C_6$, alcényle en $C_2$–$C_7$ ou phényl-alkyle en $C_1$–$C_7$, ce dernier éventuellement substitué sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, et $R_4$ représente l'hydrogène, un groupe alkyle en $C_1$–$C_7$, hydroxyalkyle en $C_1$–$C_7$, phényl-hydroxyalkyle en $C_1$–$C_7$, halogénophényl-hydroxyalkyle en $C_1$–$C_7$, (alkyle en $C_1$–$C_7$)-phényl-hydroxyalkyle en $C_1$–$C_7$, (alcoxy en $C_1$–$C_7$)-phényl-hydroxyalkyle en $C_1$–$C_7$, (alcoxy en $C_1$–$C_7$)-alkyle en $C_1$–$C_7$, (alcoxy en $C_1$–$C_7$)-hydroxyalkyle en $C_1$–$C_7$, (alcanoyloxy en $C_1$–$C_7$)-alkyle en $C_1$–$C_7$, (alcoxy en $C_1$–$C_7$)-carbonyl-(alkyle en $C_1$–$C_7$), alcényle en $C_2$–$C_7$, (cycloalkyle en $C_3$–$C_6$)-alkyle en $C_1$–$C_7$, alcynyle en $C_2$–$C_7$, thiényl-alkyle en $C_1$–$C_7$, furyl-alkyle en $C_1$–$C_7$, ou un groupe phénoxy-hydroxyalkyle en $C_1$–$C_7$, benzoyloxy-alkyle en $C_1$–$C_7$, phényl-alkyle en $C_1$–$C_7$ ou benzoyl-alkyle en $C_1$–$C_7$, ces derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino, ou un groupe $\begin{matrix}R_6\\R_7\end{matrix}\!>$N-alkyle en $C_1$–$C_7$ et X représente O, leurs isomères optiques et géométriques et leurs sels formés par addition avec des acides acceptables pour l'usage pharmaceutique, caractérisé en ce que l'on prépare les composés selon les modes de réalisation c), l), m), n) et o) de la rev. 1 ou selon le mode de réalisation a) ou b) dans lequel on utilise comme produit de départ un composé de formule IX ou XII dans laquelle $R''_4$ représente le groupe méthyle, ou selon le mode de réalisation f) dans lequel on utilise comme produit de départ un composé de formule Aa dans laquelle $R''_4$ représente un groupe méthyle et X représente O, ou selon les modes de réalisation g), h), i), j) ou k) dans lesquels on utilise comme produit de départ un composé de formule Ab, Ae, Ah, Ad' ou Af''' dans laquelle X représente O.

3. Procédé selon la rev. 1 ou 2, caractérisé en ce que $R_1$ représente l'hydrogène.

4. Procédé selon l'une des rev. 1 à 3, caractérisé en ce que $R_2$ et $R_3$ représentent des groupes alkyle en $C_1$–$C_7$.

5. Composé selon l'une des rev. 1 à 4, dans lequel $R_4$ représente un groupe alkyle en $C_1$–$C_7$, hydroxyalkyle en $C_1$–$C_7$, phényl-hydroxyalkyle en $C_1$–$C_7$, halogénophényl-hydroxyalkyle en $C_1$–$C_7$, (alcoxy en $C_1$–$C_7$)-alkyle en $C_1$–$C_7$ ou un groupe phénoxy-alkyle en $C_1$–$C_7$, benzoyl-alkyle en $C_1$–$C_7$ ou phényl-alkyle en $C_1$–$C_7$, ces derniers éventuellement substitués sur le noyau phényle par des halogènes, des groupes trifluorométhyle, alkyle en $C_1$–$C_7$, alcoxy en $C_1$–$C_7$, nitro, amino, alkylamino en $C_1$–$C_7$ ou di-(alkyle en $C_1$–$C_7$)-amino.

6. Procédé selon l'une des rev. 1 et 3 à 5, caractérisé en ce que X représente O.

7. Procédé selon l'une des rev. 1 à 6, caractérisé en ce que le composé est préparé à l'état de l'isomère trans.

8. Procédé selon la rev. 2, caractérisé en ce que l'on prépare la
3-éthyl-2,6-diméthyl-4,4a,5,6,7,8,8a,9-octahydro-4a,8a-trans-1H-pyrrolo[2,3-g]isoquinoléine-4-one.

9. Procédé selon la rev. 1, caractérisé en ce que l'on prépare la
2-méthyl-3-éthyl-6-(2-phényléthyl)-4,4a,5,6,7,8,-8a,9-octahydro-4a,8a-trans-1H-pyrrolo[2,3-g]-isoquinoléine-4-one.

10. Procédé selon la rev. 1, caractérisé en ce que l'on prépare la
2-méthyl-3-éthyl-6-(2-éthoxyéthyl)-4,4a,5,6,7,8,-8a,9-octahydro-4a,8a-trans-1H-pyrrolo[2,3-g]-isoquinoléine-4-one.

11. Procédé selon la rev. 1, caractérisé en ce que l'on prépare la
2-méthyl-3-éthyl-6-[3-(4-fluorophényl)-4-oxobutyl]-4,4a,5,6,7,8,8a,9-octahydro-4a,8a-trans-1H-pyrrolo[2,3-g]isoquinoléine-4-one.

12. Procédé selon la rev. 1, caractérisé en ce que l'on prépare la
2-méthyl-3-éthyl-6-(2-hydroxy-3,3-diméthylbutyl)-4,4a,5,6,7,8,8a,9-octahydro-4a,8a-trans-1H-pyrrolo[2,3-g]isoquinoléine-4-one.